# EUROPÄISCHE PATENTANMELDUNG

(11) **EP 2 245 935 A1**
(43) Veröffentlichungstag der Anmeldung: **03.11.2010**
(21) Anmeldenummer: 09006045.0
(22) Anmeldetag: 02.05.2009
(51) Int. Cl.: A01N 43/88, A01N 47/36, C07D 413/04, C07D 413/14, C07D 498/04

(54) **Herbizide Verbindungen auf Basis von N-Azinyl-N-pyridylsulfonyl-harnstoffen**

(71) Anmelder: Bayer CropScience AG, 40789 Monheim (DE)
(72) Erfinder: Müller, Klaus-Helmut, 40593 Düsseldorf (DE); Waldraff, Christian, 61118 Bad Vibel (DE); Gesing, Ernst Rudolf, 40699 Erkrath (DE); Bojack, Guido, 65207 Wiesbaden-Naurod (DE); Kehne, Heinz, 65719 Hofheim (DE)

(57) **Zusammenfassung**

Beschrieben werden Verbindungen der allgemeinen Formel (I) 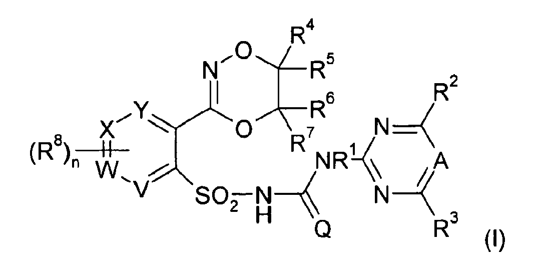 in welcher die jeweiligen Substituenten die in der Beschreibung angegebenen Bedeutungen aufweisen. Die Verbindungen der allgemeinen Formel (I) können als Herbizide und Pflanzenwachstumsregulatoren verwendet werden.

## Beschreibung

Die vorliegende Erfindung betrifft N-Azinyl-N'-pyridylsulfonyl-harnstoffe. Weiterer Gegenstand der vorliegenden Erfindung sind Mischungen der zuvor genannten Harnstoff-Derivate mit anderen Herbiziden und/oder Safenern. Darüber hinaus betrifft die vorliegende Erfindung Verfahren zur Herstellung der zuvor genannten Harnstoff-Derivate sowie die Verwendung dieser Verbindungen als Herbizide und Pflanzenwachstumsregulatoren allein und in Mischung mit Safenern und/oder in Mischung mit anderen Herbiziden, insbesondere deren Verwendung zur Pflanzenbekämpfung in speziellen Pflanzenkulturen oder als Pflanzenschutzregulatoren.

Es ist bereits bekannt, dass bestimmte N-Azinyl-N'-arylsulfonylharnstoffe mit einfachen offenkettigen Hydroxamsäureester-Gruppen im Arylteil, wie z.B. N-(4,6-Dimethylpyrimidin-2-yl)-N'-(2-methoxyaminocarbonyl-phenylsulfonyl)-harnstoff und der entsprechende N'-(2-n-Octyloxyaminocarbonylphenylsulfonyl)-harnstoff herbizide Eigenschaften aufweisen (vgl. DE 3 516 435 A, EP 0 173 958 A, US 4,704,158).

Weiterhin sind auch bestimmte herbizid wirksame N-Azinyl-N'-hetarylsulfonyl-harnstoffe bekannt, welche im Hetarylteil durch O,N-dialkylierte, ebenfalls offenkettige Hydroxamsäure-Gruppen substituiert sind (vgl. EP 0 301 784 A); entsprechende cyclische Hydroxamsäurederivate sind dagegen bisher nicht beschrieben worden.

Aus der US 5,476,936 sind des Weiteren herbizide N-Azinyl-N'-hetarylsulfonyl-harnstoffe der allgemeinen Formel 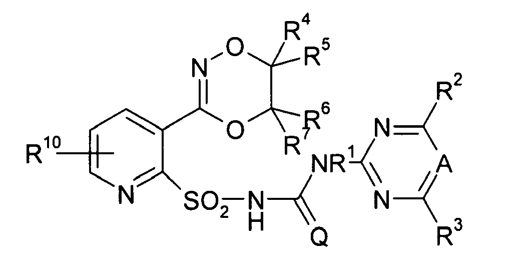 bekannt (wobei die einzelnen Reste die in der US 5,476,936 angegebene Bedeutung aufweisen). Bei diesen Verbindungen befindet sich der Sulfonylharnstoffrest in α-Position zu dem Stickstoffatom des Pyridinringes.

Die gemäß den oben genannten Schriften bereits bekannten Wirkstoffe weisen bei ihrer Anwendung Nachteile auf, sei es,
(a) dass sie keine oder aber eine nur unzureichende herbizide Wirkung gegen Schadpflanzen besitzen,
(b) dass nur ein zu geringes Spektrum an Schadpflanzen, bekämpft werden kann, oder
(c) dass sie eine zu geringe Selektivität in Nutzpflanzenkulturen besitzen.

Es ist deshalb wünschenswert, alternative chemische Wirkstoffe auf Basis von entsprechenden Harnstoff-Derivaten bereitzustellen, die als Herbizide oder Pflanzenwachstumsregulatoren eingesetzt werden können und mit welchen bestimmte Vorteile im Vergleich zu aus dem Stand der Technik bekannten Systemen verbunden sind.

Damit ergibt sich im Allgemeinen als Aufgabe der vorliegenden Erfindung, entsprechende alternative Harnstoff-Derivate bereitzustellen, welche als Herbizide oder Pflanzenwachstumsregulatoren, insbesondere mit einer zufrieden stellenden herbiziden Wirkung gegen Schadpflanzen, mit einem breiten Spektrum gegenüber Schadpflanzen und/oder mit einer hohen Selektivität in Nutzpflanzenkulturen, eingesetzt werden können. Diese Harnstoff-Derivate sollten dabei vorzugsweise ein besseres Eigenschaftsprofil, insbesondere eine bessere herbizide Wirkung gegen Schadpflanzen, ein breiteres Spektrum gegenüber Schadpflanzen und/oder eine höhere Selektivität in Nutzpflanzenkulturen, als die aus dem Stand der Technik bekannten Harnstoff-Derivate zeigen.

Es wurden nun neue N-Azinyl-N'-pyridylsulfonyl-harnstoffe der allgemeinen Formel (I), 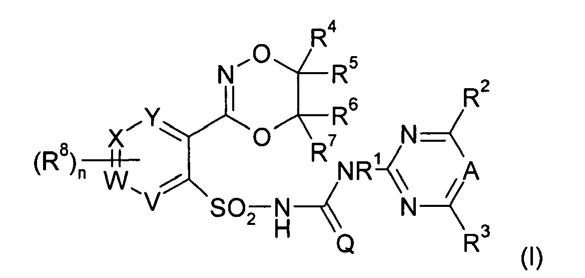 identifiziert, in welchen
- W, X und Y: so gewählt werden, dass ein Index dieser Indizes für ein Stickstoffatom steht und die übrigen Indizes für Kohlenstoffatome stehen, wobei die Kohlenstoffatome unsubstituiert oder unabhängig voneinander mit R⁸ substituiert sind;
- V: für ein Kohlenstoffatom steht, welches unsubstituiert oder mit R⁸ substituiert ist;
- A: ausgewählt ist aus der Gruppe, bestehend aus Stickstoff und CR⁹; wobei
- R⁹: ausgewählt ist aus der Gruppe, bestehend aus Wasserstoff, Alkyl, Halogen und Haloalkyl;
- R¹: ausgewählt ist aus der Gruppe, bestehend aus Wasserstoff und einem gegebenenfalls substituierten Rest aus der Reihe Alkyl, Alkoxy, Alkoxyalkyl, Alkenyl, Alkinyl, Cycloalkyl, Cycloalkylalkyl, Aralkyl und Aryl;
- R²: ausgewählt ist aus der Gruppe, bestehend aus Wasserstoff, Halogen, gegebenenfalls durch Halogen substituiertes Alkyl, gegebenenfalls durch Halogen substituiertes Alkoxy, gegebenenfalls durch Halogen substituiertes Alkylthio, gegebenenfalls durch Halogen substituiertes Alkylamino oder gegebenenfalls durch Halogen substituiertes Dialkylamino;
- R³: ausgewählt ist aus der Gruppe, bestehend aus Wasserstoff, Halogen, gegebenenfalls durch Halogen substituiertes Alkyl, gegebenenfalls durch Halogen substituiertes Alkoxy, gegebenenfalls durch Halogen substituiertes Alkylthio, gegebenenfalls durch Halogen substituiertes Alkylamino oder gegebenenfalls durch Halogen substituiertes Dialkylamino,
- R⁴ bis R⁷,: jeweils unabhängig voneinander, ausgewählt werden aus der Gruppe, bestehend aus Wasserstoff, Halogen, Cyano, Alkyl, Alkoxy, Alkylthio, Alkylsulfinyl, Alkylsulfonyl, Alkylamino, Dialkylamino, Alkylcarbonyl, Alkoxycarbonyl, Alkylaminocarbonyl oder Dialkylaminocarbonyl, wobei die Reste unsubstituiert sein können oder ein oder mehrere Reste, ausgewählt aus der Gruppe, bestehend aus Halogen, Cyano, Alkoxy und Alkylthio, tragen können, oder R⁴ und R⁶ bzw. R⁵ und R⁷ eine gegebenenfalls durch Sauerstoff oder Schwefel unterbrochene Alkyliden-Gruppe darstellen,
- R⁸: ausgewählt ist aus der Gruppe, bestehend aus Halogen, Cyano, Thiocyanato, Nitro, Alkyl, Alkoxy, Alkylthio, Alkylsulfinyl, Alkylsulfonyl, Alkylamino, Dialkylamino, Alkylcarbonyl, Alkoxycarbonyl, Alkylamino- carbonyl, oder Dialkylaminocarbonyl, wobei die Reste unsubstituiert sein können oder ein oder mehrere Reste, ausgewählt aus der Gruppe, bestehend aus Halogen, Cyano, Alkoxy und Alkylthio, tragen können.
- Q: ausgewählt ist aus der Gruppe, bestehend aus Sauerstoff oder Schwefel, insbesondere Sauerstoff ist,
- n: gleich einer ganzzahligen Zahl von 0 bis 3 ist, und
sowie Salze von Verbindungen der Formel (I),
mit der Maßgabe, dass in den Fällen, in welchen n gleich 0, 1 oder 2 ist, die unsubstituierten Kohlenstoffatome V, W, X und/oder Y mit Wasserstoff abgesättigt sind.

Von einer ersten Ausführungsform der vorliegenden Erfindung werden Verbindungen der allgemeinen Formel (I) umfasst, in welchen
- A: bevorzugt ausgewählt ist aus der Gruppe, bestehend aus Stickstoff und CH.

Von einer zweiten Ausführungsform der vorliegenden Erfindungen werden Verbindungen der allgemeinen Formel (I) umfasst, in welchen
- R¹: bevorzugt ausgewählt ist aus der Gruppe, bestehend aus Wasserstoff, Alkyl, Alkoxy, Alkoxyalkyl, Alkenyl und Alkinyl, wobei die Reste unsubstituiert sein können oder ein oder mehrere Halogenatome tragen können,
- R¹: besonders bevorzugt ausgewählt ist aus der Gruppe, bestehend aus Wasserstoff, Methyl, Ethyl, Methoxy, Methoxymethyl und Ethoxy,
- R¹: insbesondere bevorzugt ausgewählt ist aus der Gruppe, bestehend aus Wasserstoff und Methyl,
und
- R¹: speziell bevorzugt Wasserstoff ist.

Von einer dritten Ausführungsform der vorliegenden Erfindung werden Verbindungen der allgemeinen Formel (I) umfasst, in welchen
- R²: bevorzugt ausgewählt ist aus der Gruppe, bestehend aus Wasserstoff, Halogen, Alkyl, Alkoxy, Alkylthio, Alkylamino und Dialkylamino, wobei die Reste unsubstituiert sein können oder ein oder mehrere Halogenatome tragen können,
- R²: besonders bevorzugt ausgewählt ist aus der Gruppe, bestehend aus Wasserstoff, Chlor, Methyl, Ethyl, Trifluoromethyl, Methoxy, Ethoxy, Trifluorethoxy, Difluoromethoxy, Methylthio, Methylamino und Dimethylamino,
und
- R²: speziell bevorzugt ausgewählt ist aus der Gruppe, bestehend aus Wasserstoff, Chlor, Methyl, Methoxy, Methylthio und Dimethylamino.

Von einer vierten Ausführungsform der vorliegenden Erfindung werden Verbindungen der allgemeinen Formel (I) umfasst, in welchen
- R³: bevorzugt ausgewählt ist aus der Gruppe, bestehend aus Wasserstoff, Halogen, Alkyl, Alkoxy, Alkylthio, Alkylamino und Dialkylamino, wobei die Reste unsubstituiert sein können oder ein oder mehrere Halogenatome tragen können,
- R³: besonders bevorzugt ausgewählt ist aus der Gruppe, bestehend aus Wasserstoff, Chlor, Methyl, Ethyl, Trifluoromethyl, Methoxy, Ethoxy, Trifluorethoxy, Difluoromethoxy, Methylthio, Methylamino oder Dimethylamino
und
- R³: speziell bevorzugt ausgewählt ist aus der Gruppe, bestehend aus Methyl, Methoxy und Trifluorethoxy.

Von einer fünften Ausführungsform der vorliegenden Erfindung werden
Verbindungen der allgemeinen Formel (I) umfasst, in welchen
- R⁴ bis R⁷,: jeweils unabhängig voneinander, bevorzugt ausgewählt sind aus der Gruppe, bestehend aus Wasserstoff, Halogen, Cyano, ,Alkyl, Alkoxy, Alkylthio, Alkylsulfinyl, Alkylsulfonyl, Alkylamino, Dialkylamino, Alkylcarbonyl, Alkoxycarbonyl, Alkylaminocarbonyl oder Dialkylamino, wobei die Reste unsubstituiert sein können oder ein oder mehrere Halogenatome tragen können,
- R⁴ bis R⁷,: jeweils unabhängig voneinander, besonders bevorzugt ausgewählt sind aus der Gruppe, bestehend aus Wasserstoff, Fluor, Chlor, Cyano, Methyl, Ethyl, Propyl, Isopropyl, Cyclopropyl, n-, i- s- oder tert. Butyl, Methylthio, Methylsulfinyl, Methylsulfonyl, Methoxycarbonyl und Ethoxycarbonyl, wobei die Reste unsubstituiert sein können oder ein oder mehrere Halogenatome tragen können, und R⁴ und R⁶ bzw. R⁵ und R⁷ eine CH₂-CH₂-CH₂-Gruppe, eine CH₂-CH₂-CH₂-CH₂-Gruppe, eine CH₂-O-CH₂-Gruppe, CH₂-S-CH₂-Gruppe, eine CH₂-O-CH₂-CH₂- Gruppe, eine CH₂-CH₂-O-CH₂-Gruppe oder CH₂-CH₂-O-CH₂-CH₂- Gruppe darstellen,
und
- R⁴ bis R⁷,: jeweils unabhängig voneinander, speziell bevorzugt ausgewählt werden aus der Gruppe, bestehend aus Wasserstoff, Methyl, Ethyl, Propyl und Isopropyl, oder R⁴ und R⁶ bzw. R⁵ und R⁷ eine CH₂-CH₂- CH₂-Gruppe, eine CH₂-CH₂-CH₂-CH₂-Gruppe, eine CH₂-O-CH₂- Gruppe, CH₂-S-CH₂-Gruppe, eine CH₂-O-CH₂-CH₂-Gruppe, eine CH₂- CH₂-O-CH₂-Gruppe oder CH₂-CH₂-O-CH₂-CH₂-Gruppe darstellen.

Von einer sechsten Ausführungsform der vorliegenden Erfindung werden Verbindungen der allgemeinen Formel (I) umfasst, in welchen
- R⁸: bevorzugt ausgewählt ist aus der Gruppe, bestehend aus Halogen, Cyano, Thiocyanato, Nitro, Alkyl, Alkoxy, Alkylthio, Alkylsulfinyl, Alkylsulfonyl, Alkylamino, Dialkylamino, Alkylcarbonyl, Alkoxycarbonyl, Alkylaminocarbonyl und Dialkylaminocarbonyl, wobei die Reste unsubstituiert sein können oder ein oder mehrere Halogenatome tragen können, und
- R⁸: besonders bevorzugt ausgewählt ist aus der Gruppe, bestehend aus Fluor, Chlor, Brom, Iod, Cyano, Methyl, Methoxy, Ethoxy, Methylthio, Ethylthio, Methylsulfinyl, Ethylsulfinyl, Methylsulfonyl, Ethylsulfonyl, Methylamino und Dimethylamino, wobei die Reste unsubstituiert sein können oder ein oder mehrere Halogenatome tragen können.

Von einer siebten Ausführungsform der vorliegenden Erfindung werden Verbindungen der allgemeinen Formel (I) umfasst, in welchen

n gleich 0 oder 1, bevorzugt n gleich 0, ist.

Wenn im Rahmen der vorliegenden Erfindung mehrere Reste R⁸ zugegen sind, also n gleich 2 oder 3 ist, können die Reste R⁸ gleich oder verschieden sein.

Im Rahmen dieser Ausführungsformen der vorliegenden Erfindung ist es möglich, die einzelnen allgemeinen, bevorzugten und besonders bevorzugten Bedeutungen für die Substituenten R¹ bis R⁸, Q und A sowie für den Index n beliebig miteinander zu kombinieren. Das heißt, dass Verbindungen der allgemeinen Formel (I) von der vorliegenden Erfindung umfasst sind, in welchen beispielswiese der Substituent R¹ eine bevorzugte Bedeutung aufweist und die Substituenten R² bis R⁸ die allgemeine Bedeutung aufweisen oder aber beispielsweise der Substituent R² eine bevorzugte Bedeutung aufweist, der Substituent R³ eine besonders bevorzugte Bedeutung, der und die übrigen Substituenten die allgemeine Bedeutung aufweisen. Diese einzelnen Kombinationen werden aus Übersichtsgründen nicht expressis verbis genannt, gelten aber im Rahmen der vorliegenden Erfindung umfasst.

Der mit dem 2-(5,6-Dihydro-[1,4,2]-dioxazin)-3-yl-Rest unmittelbar verbundene Heterocyclus in den Verbindungen der allgemeinen Formel (I) 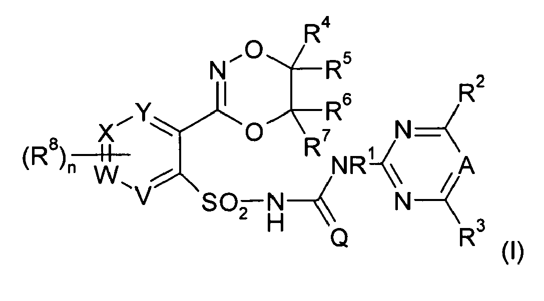 weist ein Stickstoffatom auf, d.h. ein Rest, ausgewählt aus der Gruppe, bestehend aus W, X und Y, entspricht einem Stickstoffatom. Die übrigen zwei Reste weisen die Bedeutung eines Kohlenstoffatoms auf, wobei die Kohlenstoffatome gegebenenfalls mit einem oder zwei Resten R⁸, welche gleich oder verschieden sein können, substituiert sind.

In einer besonderen Ausgestaltung der vorliegenden Erfindung weist die Verbindung der allgemeinen Formel (I) daher die folgende Struktur (la) auf, in welcher W die Bedeutung eines Stickstoffatoms hat:

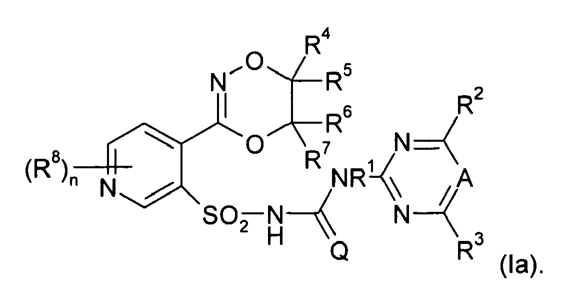

In einer noch weiteren Ausgestaltung der vorliegenden Erfindung weist die Verbindung der allgemeinen Formel (I) daher die folgende Struktur (Ib) auf, in welcher X die Bedeutung eines Stickstoffatoms hat:

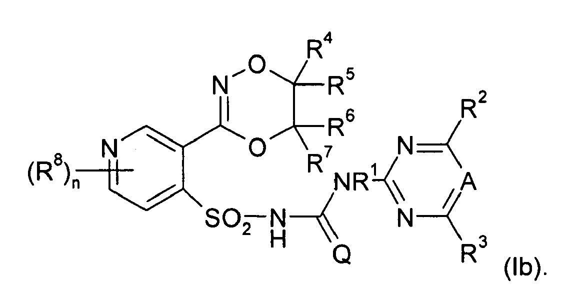

In einer noch weiteren Ausgestaltung der vorliegenden Erfindung weist die Verbindung der allgemeinen Formel (I) daher die folgende Struktur (Ic) auf, in welcher Y die Bedeutung eines Stickstoffatoms hat:

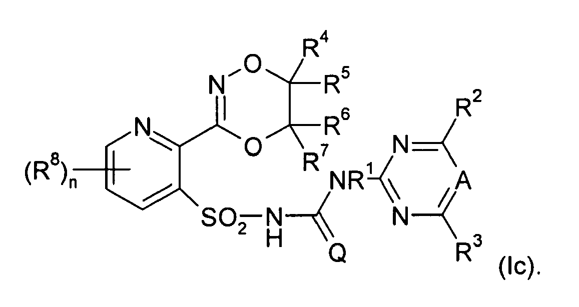

In jeder dieser drei Ausgestaltungen können die einzelnen Reste R¹ bis R⁸, A und Q sowie der Index n die vorstehend definierten allgemeinen, bevorzugten und besonders bevorzugten Bedeutungen aufweisen.

Von diesen drei Ausgestaltungen sind erfindungsgemäß diejenigen Verbindungen besonders bevorzugt, in welchen entweder W oder Y die Bedeutung Stickstoff aufweist (Verbindungen der allgemeinen Formel (Ia) und (Ic)).

Insbesondere bevorzugt sind diejenigen Verbindungen der Formel (I), in welchen W die Bedeutung Stickstoff aufweist (Verbindungen der allgemeinen Formel (Ia)).

In den Verbindungen der allgemeinen Formel (I) weisen die Substituenten und Reste R¹ bis R⁸, Q und A sowie der Index n die vorstehend allgemeinen, bevorzugten, besonders bevorzugten insbesondere bevorzugten und insbesondere ganz bevorzugten Bedeutungen auf.

Gegenstand der vorliegenden Erfindung sind vorzugsweise auch die Lithium-, Natrium-, Kalium-, Magnesium-, Calcium-, Ammonium-, C₁-C₄-Alkyl-ammonium, Di-(C₁-C₄-alkyl)-ammonium-, Tri-(C₁-C₄-alkyl)-ammonium-, Tetra-(C₁-C₄-alkyl)-ammonium-, Tri-(C₁-C₄-alkyl)-sulfonium, C₅- oder C₆-Cycloalkyl-ammonium-, Di-(C₁-C₂-alkyl)-benzyl-ammonium und Tri-(C₁-C₂-alkyl)-benzyl-ammonium-Salze von Verbindungen der Formel (I), in welcher R¹ bis R⁸, A, Q und n die oben allgemeinen, bevorzugten, besonders bevorzugten und insbesondere bevorzugten Bedeutungen aufweisen und die nach allgemein üblichen Verfahren hergestellt werden können.

Die Verbindungen der allgemeinen Formel (I) können darüber hinaus gegebenenfalls durch Anlagerung einer geeigneten anorganischen oder organischen Säure, wie beispielsweise HCl, HBr, H₂SO₄ oder HNO₃, aber auch Oxalsäure oder Sulfonsäuren an eine basische Gruppe, wie z. B. Amino oder Alkylamino, Salze bilden. Geeignete Substituenten, die in deprotonierter Form, wie z.B. Sulfonsäuren oder Carbonsäuren, vorliegen, können innere Salze mit ihrerseits protonierbaren Gruppen, wie Aminogruppen bilden. Salze können ebenfalls dadurch gebildet werden, dass bei geeigneten Substituenten, wie z. B. Sulfonsäuren oder Carbonsäuren, der Wasserstoff durch ein im agrochemischen Bereich geeignetes Kation ersetzt wird. Diese Salze sind beispielsweise Metallsalze, insbesondere Alkalimetallsalze oder Erdalkalimetallsalze, insbesondere Natrium- und Kaliumsalze, oder auch Ammoniumsalze, Salze mit organischen Aminen oder quartäre (quaternäre) Ammoniumsalze mit Kationen der Formel [NRR'R"R"']⁺, worin R bis R"' jeweils unabhängig einen organischen Rest, insbesondere Alkyl, Aryl, Aralkyl oder Alkylaryl darstellen.

In der allgemeinen Formel (I) und allen übrigen Formeln in der vorliegenden Erfindung können die Reste Alkyl, Alkoxy, Haloalkyl, Haloalkoxy, Alkylamino, Alkylthio, Haloalkylthio, Alkylsulfinyl, Alkylsulfonyl, Haloalkylsulfinyl und Haloalkylsulfonyl, sowie die entsprechenden ungesättigten und/oder substituierten Reste im Kohlenstoffgerüst jeweils geradkettig oder verzweigt sein. Wenn nicht speziell angegeben, sind bei diesen Resten die niederen Kohlenstoffgerüste, z.B. mit 1 bis 6 C-Atomen, insbesondere 1 bis 4 C-Atomen, bzw. bei ungesättigten Gruppen mit 2 bis 6 C-Atomen, insbesondere 2 bis 4 C-Atomen, bevorzugt. Alkylreste, auch in den zusammengesetzten Bedeutungen wie Alkoxy, Haloalkyl usw., bedeuten z.B. Methyl, Ethyl, Propyle wie n- oder i-Propyl, Butyle wie n-, iso- oder tert.-Butyl, Pentyle wie n-Pentyl, iso-Pentyl oder neo-Pentyl, Hexyle wie n-Hexyl, i-Hexyl, 3-Methylpentyl, 2,2-Dimethylbutyl oder 2,3-Dimethylbutyl, Heptyle, wie n-Heptyl, 1-Methylhexy oder 1,4-Dimethylpentyl; Alkenyl- und Alkinylreste haben die Bedeutung der den Alkylresten entsprechenden möglichen ungesättigten Reste; wobei mindestens eine Doppelbindung bzw. Dreifachbindung, vorzugsweise eine Doppelbindung bzw. Dreifachbindung enthalten ist. Alkenyl bedeutet z.B. Vinyl, Allyl, 1-Methylprop-2-en-1-yl, 2-Methyl-prop-2-en-1-yl, But-2-en-1-yl, But-3-en-1-yl, 1-Methyl-but-3-en-1-yl und 1-Methyl-but-2-en-1-yl; Alkinyl bedeutet z.B. Ethinyl, Propargyl, But-2-in-1-yl, But-3-in-1-yl und 1-Methyl-but-3-in-1-yl.

Cycloalkyl-Gruppen sind z. B. Cyclopropyl, Cyclobutyl, Cyclopentyl, Cyclohexyl, Cycloheptyl und Cyclooctyl. Die Cycloalkyl Gruppen können in bi- oder tri-cyclischer Form vorkommen.

Wenn Haloalkylgruppen und Haloalkylreste von Haloalkoxy, Haloalkylthio, Haloalkenyl, Haloalkinyl u.a. angegeben sind, sind bei diesen Resten die niederen Kohlenstoffgerüste, z. B. mit 1 bis 6 C-Atomen oder 2 bis 6, insbesondere 1 bis 4 C-Atomen oder bevorzugt 2 bis 4 C-Atomen, sowie die entsprechenden ungesättigten und/oder substituierten Reste im Kohlenstoffgerüst jeweils geradkettig oder verzweigt. Beispiele sind Difluormethyl, 2,2,2-Trifluorethyl, Trifluorallyl, 1-Chlorprop-1-yl-3-yl. Der Begriff "Halo" wird erfindungsgemäß synonym zu "Halogen" verwendet.

Alkylen-Gruppen sind bei diesen Resten die niederen Kohlenstoffgerüste, z. B. mit 1 bis 10 C-Atomen, insbesondere 1 bis 6 C-Atomen oder bevorzugt 2 bis 4 C-Atomen (sofern nicht andersweitig definiert), sowie die entsprechenden ungesättigten und/oder substituierten Reste im Kohlenstoffgerüst, die jeweils geradkettig oder verzweigt sein können. Beispiele sind Methylen, Ethylen, n- und iso-Propylen und n-, sec-, iso- sowie tert.-Butylen.

Hydroxyalkylgruppen sind bei diesen Resten die niederen Kohlenstoffgerüste, z. B. mit 1 bis 6 C-Atomen, insbesondere 1 bis 4 C-Atomen, sowie die entsprechenden ungesättigten und/oder substituierten Resten im Kohlenstoffgerüst, die jeweils geradkettig oder verzweigt sein können. Beispiele hierzu sind 1,2-Dihydroxyethyl und 3-Hydroxypropyl.

Halogen bedeutet Fluor, Chlor, Brom oder Iod. Haloalkyl, -alkenyl und -alkinyl bedeuten durch Halogen, vorzugsweise durch Fluor, Chlor oder Brom, insbesondere durch Fluor und/oder Chlor, teilweise oder vollständig substituiertes Alkyl, Alkenyl bzw. Alkinyl, z.B. Monohaloalkyl (= Monohalogenalkyl), Perhaloalkyl, CF₃, CHF₂, CH₂F, CF₃CF₂, CH₂FCHCl, CCl₃, CHCl₂, CH₂CH₂Cl; Haloalkoxy ist z.B. OCF₃, OCHF₂, OCH₂F, CF₃CF₂O, OCH₂CF₃ und OCH₂CH₂Cl; entsprechendes gilt für Haloalkenyl und andere durch Halogen substituierte Reste. Im Folgenden wird der Rest Halogen mit vorstehender Bedeutung (Fluor, Chlor, Brom oder Iod) auch durch Hal abgekürzt.

Aryl bedeutet ein mono-, bi- oder polycyclisches aromatisches System, beispielsweise Phenyl oder Naphthyl, vorzugsweise Phenyl.

Mit der Definition "mit einem oder mehreren Resten substituiert ist" sind, wenn nicht anders definiert, ein oder mehrere gleiche oder verschiedene Reste gemeint.

Die beispielhaft genannten Substituenten ("erste Substituentenebene") können, sofern sie kohlenwasserstoffhaltige Anteile enthalten, dort gegebenenfalls weiter substituiert sein ("zweite Substitutentenebene"), beispielsweise durch einen der Substituenten, wie er für die erste Substituentenebene definiert ist. Entsprechende weitere Substituentenebenen sind möglich. Vorzugsweise werden vom Begriff "substituierter Rest" nur ein oder zwei Substitutentenebenen umfasst.

Bei Resten mit C-Atomen sind solche mit 1 bis 6 C-Atomen, vorzugsweise 1 bis 4 C-Atomen, insbesondere 1 oder 2 C-Atomen, bevorzugt. Bevorzugt sind in der Regel Substituenten aus der Gruppe Halogen, z.B. Fluor und Chlor, (C₁-C₄)-Alkyl, vorzugsweise Methyl oder Ethyl, (C₁-C₄)-Haloalkyl, vorzugsweise Trifluormethyl, (C₁-C₄)-Alkoxy, vorzugsweise Methoxy oder Ethoxy, (C₁-C₄)-Haloalkoxy, Nitro und Cyano.

Wenn ein Arylrest substituiert ist, so kann es sich vorzugsweise um Phenyl, das ein oder mehrfach, vorzugsweise bis zu dreifach durch gleiche oder verschiedene Reste aus der Gruppe Halogen, (C₁-C₄)-Alkyl, (C₁-C₄)-Alkoxy, (C₁-C₄)- Halogenalkyl, (C₁-C₄)-Halogenalkoxy, Cyano und Nitro substituiert ist, z.B. o-, m- und p-Tolyl, Di-methylphenyle, 2-, 3- und 4-Chlorphenyl, 2-, 3- und 4-Trifluormethyl und 2-, 3- und 4-Trichlormethyl-phenyl, 2,4-, 3,5-, 2,5- und 2,3-Dichlorphenyl, o-, m- und p-Methoxyphenyl.

Die Verbindungen der allgemeinen Formel (I) können je nach Art und Verknüpfung der Substituenten als Stereoisomere vorliegen. Die durch ihre spezifische Raumform definierten möglichen Stereoisomere, wie Enantiomere, Diastereomere, Z- und E-Isomere sind alle von der Formel (I) umfasst.

Sind beispielsweise eine oder mehrere Alkenylgruppen vorhanden, so können Diastereomere (Z- und E-Isomere) auftreten. Sind beispielsweise ein oder mehrere asymmetrische Kohlenstoffatome vorhanden, so können Enantiomere und Diastereomere auftreten. Stereoisomere lassen sich aus den bei der Herstellung anfallenden Gemischen nach üblichen Trennmethoden erhalten. Ebenso können Stereoisomere durch Einsatz stereoselektiver Reaktionen unter Verwendung optisch aktiver Ausgangs- und/oder Hilfsstoffe selektiv hergestellt werden. Die Erfindung betrifft somit auch alle Stereoisomeren, die von der allgemeinen Formel (II) umfasst, jedoch nicht mit ihrer spezifischen Stereoform angegeben sind, sowie deren Gemische.

Herstellung der erfindungsgemäßen Verbindungen der allgemeinen Formel (I)

Weiterer Gegenstand der vorliegenden Erfindung sind Verfahren zur Herstellung entsprechender Verbindungen der allgemeinen Formel (I) und/oder deren Salze.

In einer ersten Ausführungsform der vorliegenden Erfindung erfolgt die Herstellung der Verbindungen der allgemeinen Formel (I) dadurch, dass man (5,6-Dihydro-1,4,2-dioxazin-3-yl)pyridyl-sulfonamide der allgemeinen Formel (II) 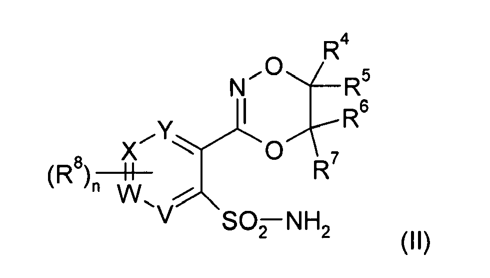 mit einem heterocyclischen (Thio)-Carbamat der allgemeinen Formel (III) 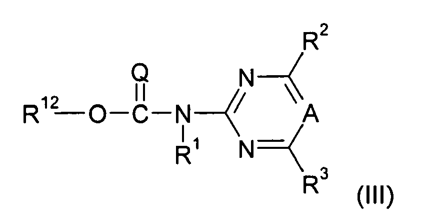 umsetzt, worin R¹² ein substituierter oder unsubstituierter (C₁-C₂₀)-Kohlenwasserstoffrest wie Aryl oder Alkyl, vorzugsweise gegebenenfalls substituiertes Phenyl oder gegebenenfalls substituiertes (C₁-C₄)-Alkyl bedeutet und worin V, W, X, Y, R¹ bis R⁸, Q, A und n die vorstehende Bedeutung aufweisen.

Die Verbindungen der allgemeinen Formel (II) können dabei durch Umsetzung der Verbindungen der allgemeinen Formel (X) mit einem Chlorierungsmittel wie Chlorgas und Ammoniaklösung gemäß nachfolgendem Reaktionsschema erhalten werden (wobei die Reste die vorstehende Bedeutung aufweisen):

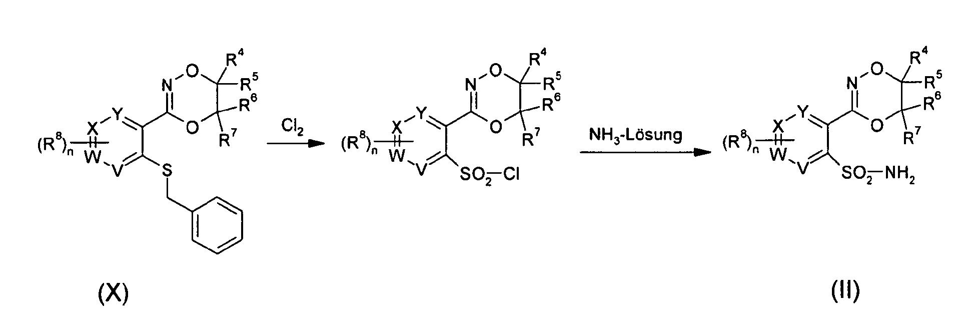

Die Verbindungen der allgemeinen Formel (X) können wiederum durch Umsetzung von Verbindungen der allgemeinen Formel (XI) mit gegebenenfalls substituierten Ethan-Derivaten der allgemeinen Formel (XII) gemäß nachfolgendem Reaktionsschema erhalten werden (wobei die Reste die vorstehende Bedeutung aufweisen):

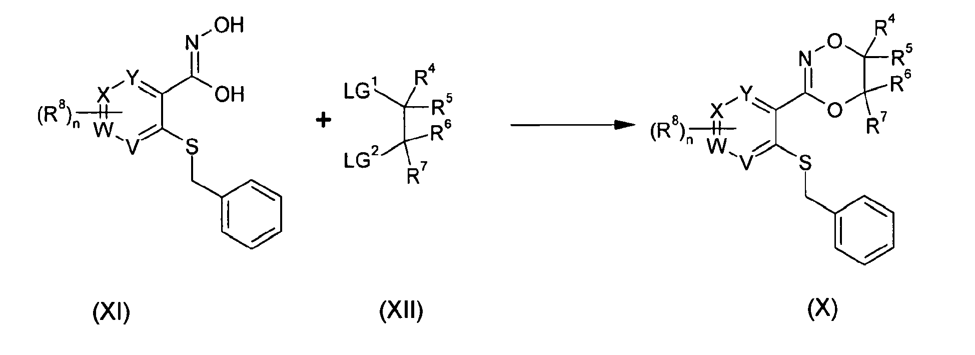

In der allgemeinen Formel (XII) bedeuten LG¹ und LG² eine Abgangsgruppe, beispielsweise Halogenid oder Sulfonat, wobei die Reste LG¹ und LG² gleich oder verschieden sein können.

Die Verbindungen der allgemeinen Formel (XI) können wiederum durch Umsetzung von Carbonsäureestern, z.B. Methylestern, mit Hydroxylamin ausgehend von Verbindungen der allgemeinen Formel (XIII) gemäß nachfolgendem Reaktionsschema erhalten werden (wobei die Reste die vorstehende Bedeutung aufweisen):

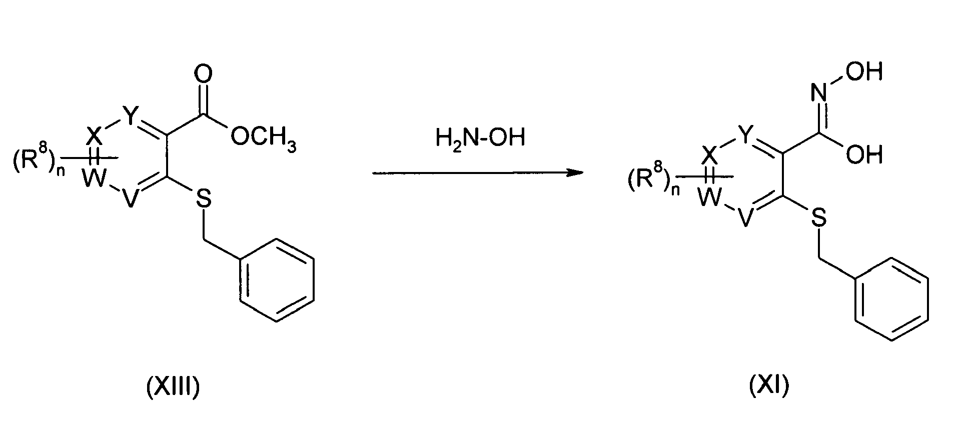

Die Verbindungen der allgemeinen Formel (XIII) können wiederum durch Umsetzung von Carbonsäuren der allgemeinen Formel (XIV) mit Thionylchlorid und Alkoholen, z.B. Methanol, ausgehend von Verbindungen der allgemeinen Formel (XIV) gemäß nachfolgendem Reaktionsschema erhalten werden (wobei die Reste die vorstehende Bedeutung aufweisen): 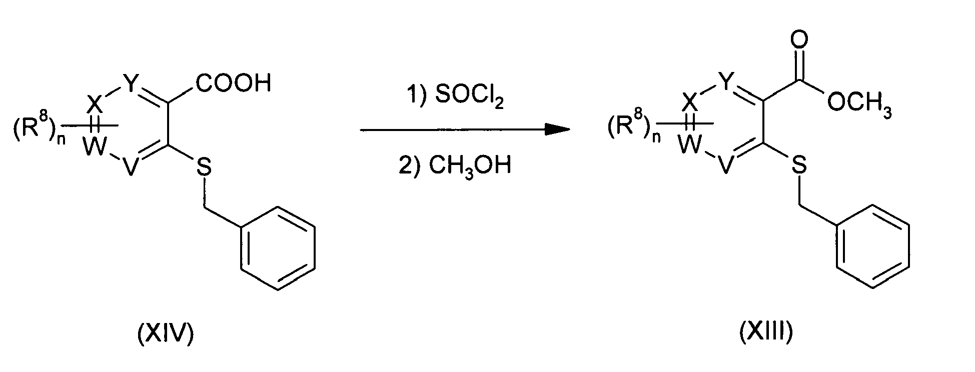 (Benzylsulfanyl)pyridincarbonsäuren der allgemeinen Formel (XIV) sind aus dem Stand der Technik bekannt (vgl. US 4,767,766, J. Med. Chem.1974, 17(10), 1065-1071 und DE 2,216,576) und können nach dem Fachmann bekannten Methoden aus kommerziell erhältlichen Vorstufen hergestellt werden (z.B. 3-Chlorisonicotinonitril, 4-Chlornicotinsäure, 3-Sulfanylpyridin-2-carbonsäure).

In einer zweiten Ausführungsform der vorliegenden Erfindung erfolgt die Herstellung der Verbindungen der allgemeinen Formel (I) dadurch, dass man 5,6-Dihydro-1,4,2-dioxazin-3-yl-pyridin-sulfonsäureiso(thio)cyanate der allgemeinen Formel (IV) 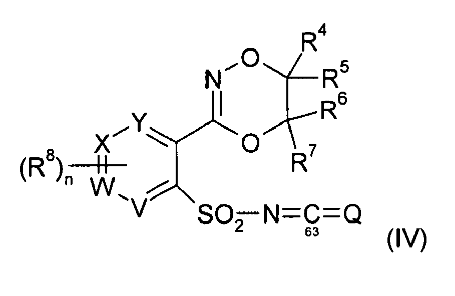 (die Herstellung entsprechender 5,6-Dihydro-1,4,2-dioxazin-3-yl-pyridinsulfonsäureiso(thio)cyanate der allgemeinen Formel (IV) wird weiter unten beschrieben) mit einem Aminoheterocyclus der allgemeinen Formel (V) 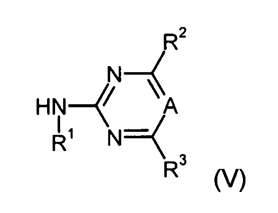 umsetzt, worin V, W, X, Y, R¹ bis R⁸, Q, A und n die vorstehende Bedeutung aufweisen. Die entsprechenden Aminopyrimidine und Aminotriazine sind kommerziell erhältlich.

In einer dritten Ausführungsform der vorliegenden Erfindung erfolgt die Herstellung der Verbindungen der allgemeinen Formel (I) dadurch, dass man Sulfonyl(thio)carbamate der allgemeinen Formel (VI) 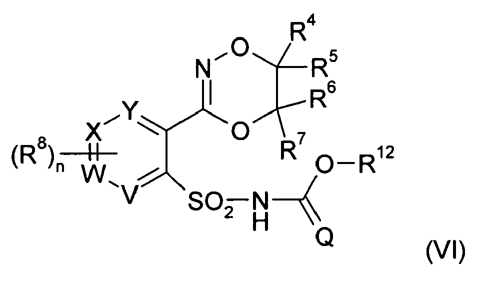 (die Herstellung entsprechender Sulfonyl(thio)carbamate der allgemeinen Formel (VI) wird weiter unten beschrieben), worin R¹² ein substituierter oder unsubstituierter (C₁-C₂₀)-Kohlenwasserstoffrest wie Aryl oder Alkyl, vorzugsweise gebenenfalls substituiertes Phenyl oder gegebenenfalls substituiertes (C₁-C₄)-Alkyl bedeutet, mit einem Aminoheterocyclus der allgemeinen Formel (V) 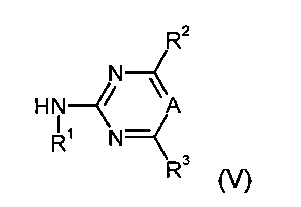 umsetzt, worin V, W, X, Y, R¹ bis R⁸, Q, A und n die vorstehende Bedeutung aufweisen.

In einer vierten Ausführungsform der vorliegenden Erfindung erfolgt die Herstellung der Verbindungen der allgemeinen Formel (I) dadurch, dass man (5,6-Dihydro-1,4,2-dioxazin-3-yl)pyridyl-sulfonamide der allgemeinen Formel (II) 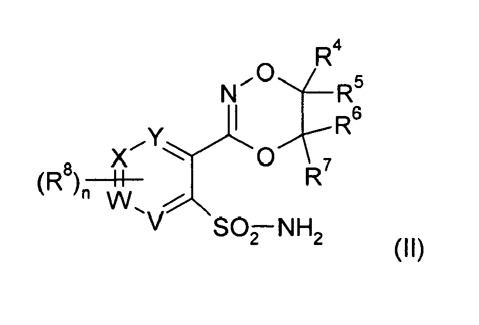 (die Herstellung entsprechender (5,6-Dihydro-1,4,2-dioxazin-3-yl)pyridyl-sulfonamide der allgemeinen Formel (II) ist oben beschrieben) mit einem Iso(thio)cyanat der allgemeinen Formel (VII) 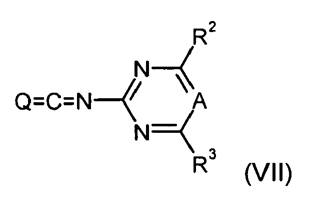 gegebenenfalls in Gegenwart eines Reaktionshilfsmittels umsetzt, worin R¹ Wasserstoff bedeutet und R² bis R⁸, V, W, X, Y, Q, n und A die vorstehende Bedeutung aufweisen. Verbindungen der Formel (VII) werden nach bekannten Methoden aus Verbindungen der allgem. Formel (V) (R¹ = Wasserstoff) in einem inerten Lösungsmittel wie Toluol oder Ethylacetat und Oxalylchlorid oder Phosgen hergestellt (vgl. Herstellungsvorschriften in 0 EP 388 994 A und EP 0 628 031 A).

In einer fünften Ausführungsform der vorliegenden Erfindung erfolgt die Herstellung der Verbindungen der allgemeinen Formel (I) dadurch, dass man einen Aminoheterocyclus der allgemeinen Formel (V) 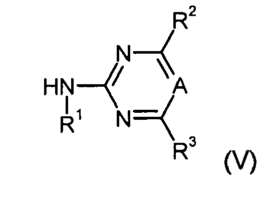 zunächst basenkatalysiert mit einem Kohlensäureester, z.B. Diphenylcarbonat, umsetzt und das gebildete Intermediat der allgemeinen Formel (III) 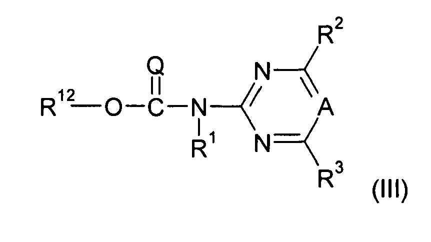 in einer Eintopfreaktion mit einem (5,6-Dihydro-1,4,2-dioxazin-3-yl)pyridyl-sulfonamid der allgemeinen Formel (II) 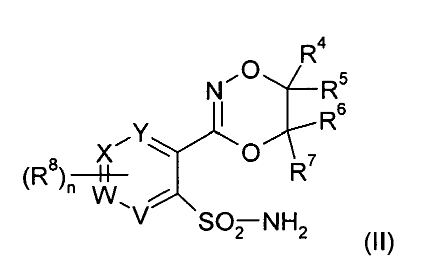 umsetzt (vgl. JP1989221366), worin V, W, X, Y, R¹ bis R⁸, Q, A und n die vorstehende Bedeutung aufweisen.

In einer sechsten Ausführungsform der vorliegenden Erfindung erfolgt die Herstellung der Verbindungen der allgemeinen Formel (I) dadurch, dass man (5,6-Dihydro-1,4,2-dioxazin-3-yl)pyridyl-sulfonsäure-halogenide der allgemeinen Formel (VIII) 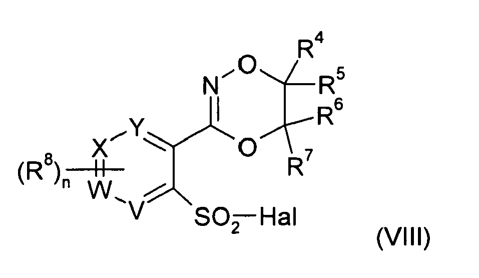 wobei Hal ein Halogenatom, vorzugsweise Chlor ist, mit einem (Thio)-Cyanat, beispielsweise einem Metall(thio)-cyanat, insbesondere einem Alkalimetall(thio)-cyanat, wie Natrium(thio)cyanat, zu einem Iso(thio)cyanat der Formel (IV) 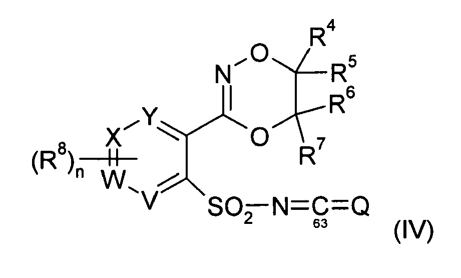 oder einem solvatisierten (stabilisierten) Derivat davon umsetzt, und anschließend mit einem Aminoheterocyclus der allgemeinen Formel (V) 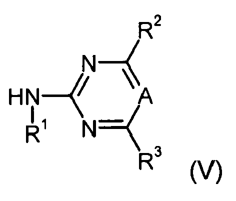 umsetzt, worin V, W, X, Y, R¹ bis R⁸, Q, A und n die vorstehende Bedeutung aufweisen.

In einer siebten Ausführungsform der vorliegenden Erfindung erfolgt die Herstellung der Verbindungen der allgemeinen Formel (I) mit Q = Sauerstoff dadurch, dass man (5,6-Dihydro-1,4,2-dioxazin-3-yl)pyridyl-sulfonamide der allgemeinen Formel (II) 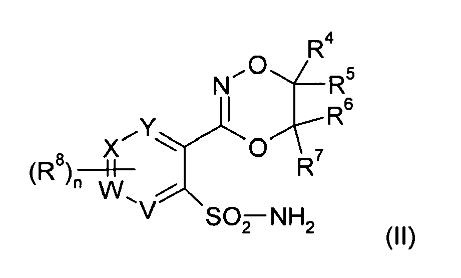 mit einem heterocyclischen Bis-carbamat der allgemeinen Formel (IX), 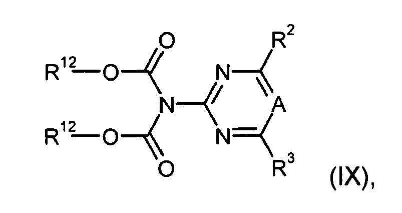 worin R¹² ein substituierter oder unsubstituierter (C₁-C₂₀)-Kohlenwasserstoffrest wie Aryl oder Alkyl, vorzugsweise gegebenenfalls substituiertes Phenyl oder gegebenenfalls substituiertes (C₁-C₄)-Alkyl bedeutet, in Gegenwart eines basischen Reaktionshilfsmittels umsetzt, worin V, W, X, Y, R¹ bis R⁸, A und n die vorstehende Bedeutung aufweisen. Die Verbindungen der Formel (IX) sind bekannt und können nach bekannten Verfahren hergestellt werden, vgl. WO 96/022284 A.

In einer achten Ausführungsform der vorliegenden Erfindung erfolgt die Herstellung der Verbindungen der allgemeinen Formel (I) dadurch, dass man (5,6-Dihydro-1,4,2-dioxazin-3-yl)pyridyl-sulfonamide der allgemeinen Formel (II) 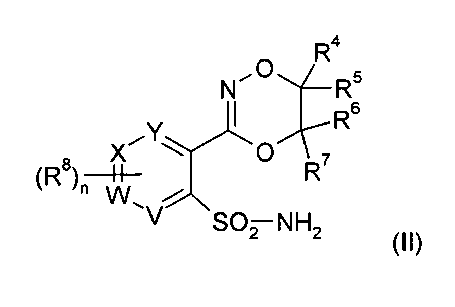 zunächst basenkatalysiert mit einem Kohlensäureester, z.B. Diphenylcarbonat, umsetzt und das gebildete Intermediat der allgemeinen Formel (VI) 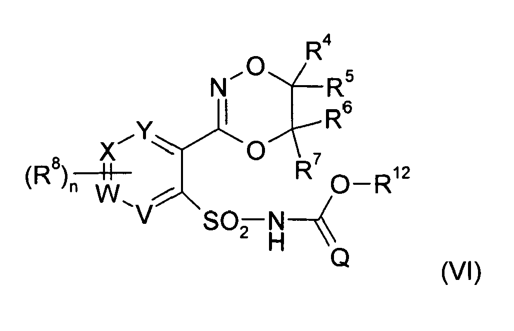 in einer Eintopfreaktion mit einem Aminoheterocyclus der allgemeinen Formel (V) 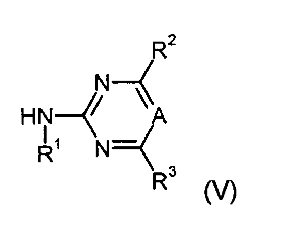 umsetzt, worin V, W, X, Y, R¹ bis R⁸, Q, A und n die vorstehende Bedeutung aufweisen.

Alle dieser Verfahren führen zu erfindungsgemäßen Verbindungen der allgemeinen Formel (I).

In den jeweils vorstehend genannten Verfahrensvarianten werden jeweils inerte Lösemittel verwendet. Unter inerte Lösemittel werden im Sinne der vorliegenden Erfindung Lösemittel verstanden, die unter den jeweiligen Reaktionsbedingungen inert sind, d.h. insbesondere nicht mit den Edukten reagieren, jedoch nicht unter beliebigen Reaktionsbedingungen inert sein müssen.

Beispiele organischer Lösemittel, welche im Rahmen der vorliegenden Erfindung verwendet werden können, sind aromatische oder aliphatische Lösemittel, wie Benzol, Toluol, Xylol, Mesitylen, Hexan, Heptan, Octan, Cyclohexan,; aliphatische und aromatische Halogenkohlenwasserstoffe, wie Methylenchlorid, Dichlorethan, Chloroform, Kohlenstofftetrachlorid, Chlorbenzol, Dichlorbenzol, Ether, wie Diethylether, Dibutylether, Diisobutylether, Methyl-tert-butylether, Isopropylethylether, Diisopropylether, Tetrahydrofuran, und Dioxan; weiter auch Dimethylsulfoxid, und Säureamidderivate, wie N,N-Dimethylformamid, N,N-Dimethylacetamid und N-Methyl-2-pyrrolidon, sowie auch Carbonsäureester, wie Ethylacetat, oder aber auch Diglyme, Dimethylglycol; Nitrile wie Acetonitril, Propionitril oder Butyronitril sowie Ketone wie Aceton, Methylethyl-keton oder Cyclohexanon. Besonders bevorzugt sind Toluol, Xylol, Dichlorbenzol, Chlorbenzol, Acetonitril, Aceton, Butyronitril oder Ethylacetat. Allerdings ist die vorliegende Erfindung nicht auf die zuvor beispielhaft genannten Lösemittel beschränkt.

Die Reaktionstemperatur, bei welcher die Umsetzungen gemäß den vorstehenden Ausführungsformen durchgeführt werden können, kann in weiten Bereichen variieren. Beispielsweise können die Umsetzungen bei einer Temperatur von 0 bis 100 °C, vorzugsweise 20 bis 70 °C, durchgeführt werden.

Der Umsetzungen der vorliegenden Erfindungen werden im Allgemeinen unter Normaldruck durchgeführt. Es ist jedoch auch möglich, unter erhöhtem Druck oder vermindertem Druck - im Allgemeinen zwischen 0,1 bar und 10 bar - zu arbeiten.

Die Verfahren zur Herstellung der erfindungsgemäßen N-Azinyl-N'-pyridylsulfonyl-harnstoffe der allgemeinen Formel (I) werden gegebenenfalls in Gegenwart eines basischen Reaktionshilfsmittels durchgeführt.

Als solche kommen alle üblichen anorganischen oder organischen Basen infrage. Hierzu gehören beispielsweise Alkalimetall- oder Erdalkalimetallhydride, -hydroxide, -amide, -alkoholate, -acetate, -carbonate oder -hydrogencarbonate, wie beispielsweise Lithium-, Natrium-, Kalium- oder Calciumhydrid, Lithium-, Natrium- oder Kaliumamid, Natrium- oder Kaliummethylat, Natrium- oder Kaliumethylat, Natrium- oder Kaliumpropylat, Aluminiumisopropylat, Natrium- oder Kalium-tert.-butylat, Natrium- oder Kaliumhydroxid, Ammoniumhydroxid, Natrium-, Kalium- oder Calciumacetat, Ammoniumacetat, Natrium-, Kalium- oder Calciumcarbonat, Ammoniumcarbonat, Natrium- oder Kaliumhydrogencarbonat, sowie basische organische Stickstoffverbindungen, wie Trimethylamin, Triethylamin, Tripropylamin, Tributylamin, Ethyl-diisopropylamin, N,N-Dimethylcyclohexylamin, Dicyclohexylamin, Ethyldicyclohexylamin, N,N-Dimethylanilin, N,N-Dimethylbenzylamin, Pyridin, 2-Methyl-, 3-Methyl- und 4-Methylpyridin, 2,4-Dimethyl-, 2,6-Dimethyl-, 3,4-Dimethyl- und 3,5-Dimethylpyridin, 5-Ethyl-2-methyl-pyridin, N-Methylpyridin, 4-(N,N-Dimethylamino)-pyridin, Diazabicyclooctan (DABCO), Diazabicyclononen (DBN) oder Diazabicycloundecen (DBU).

### Intermediate

Weiterer Gegenstand der vorliegenden Erfindung sind auch bestimmte Intermediate, welche gemäß den oben dargestellten Synthesewegen bei der Herstellung der erfindungsgemäßen Verbindungen der allgemeinen Formel (I) durchlaufen werden.

Die vorliegenden Intermediate weisen zunächst keine Präferenz für W, X oder Y gleich Stickstoff auf. Grundsätzlich werden daher in den folgenden Intermediaten grundsätzlich alle Verbindungen umfasst, in welchen W gleich Stickstoff, X gleich Stickstoff oder Y gleich Stickstoff ist. Besonders bevorzugt ist es wenn W oder Y gleich Stickstoff ist. Noch weiter bevorzugt ist im Rahmen der vorliegenden Erfindung, wenn in den Intermediaten, die im Folgenden näher beschrieben werden, W gleich Stickstoff ist. Diese Intermediate führen zu der Synthese der bevorzugten Verbindungen der allgemeinen Formel (la).

Demnach betrifft die vorliegende Erfindung in einer ersten Ausführungsform der Intermediate auch Verbindungen der allgemeinen Formel (II) 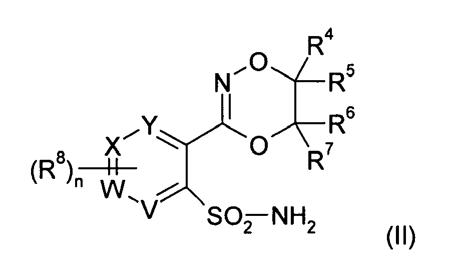 in welchen die Reste V, W, X, Y, R⁴, R⁵, R⁶, R⁷, R⁸ und n die bereits weiter oben angegebenen allgemeinen, bevorzugten und besonders bevorzugten Bedeutungen aufweisen.

Weiterer Gegenstand der vorliegenden Erfindung sind in einer zweiten Ausführungsform der Intermediate auch Verbindungen der allgemeinen Formel (IV) 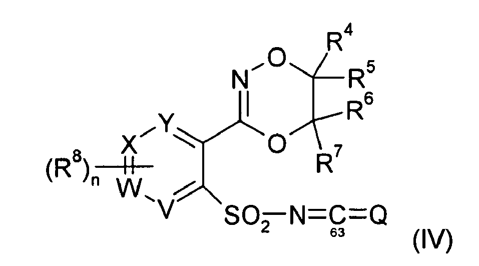 in welchen die Reste V, W, X, Y, R⁴, R⁵, R⁶, R⁷, R⁸, Q und n die bereits weiter oben angegebenen allgemeinen, bevorzugten und besonders bevorzugten Bedeutungen aufweisen.

Weiterer Gegenstand der vorliegenden Erfindung sind in einer dritten Ausführungsform der Intermediate auch Verbindungen der allgemeinen Formel (X) 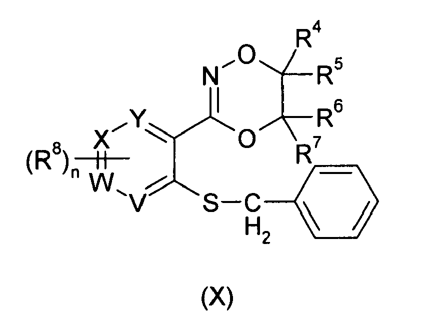 in welchen die Reste V, W, X, Y, R⁴, R⁵, R⁶, R⁷, R⁸ und n die bereits weiter oben angegebenen allgemeinen, bevorzugten und besonders bevorzugten Bedeutungen aufweisen.

Weiterer Gegenstand der vorliegenden Erfindung sind in einer vierten Ausführungsform der Intermediate auch Verbindungen der allgemeinen Formel (VIII) 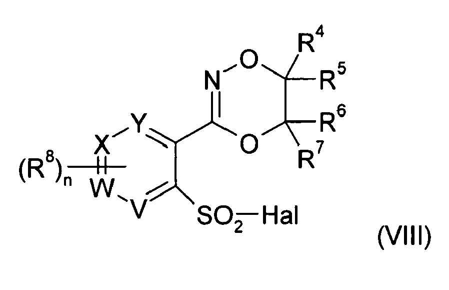 in welchen die Reste V, W, X, Y, R⁴, R⁵, R⁶, R⁷, R⁸, Hal und n die bereits weiter oben angegebenen allgemeinen, bevorzugten und besonders bevorzugten Bedeutungen aufweisen.

Weiterer Gegenstand der vorliegenden Erfindung sind in einer fünften Ausführungsform der Intermediate auch Verbindungen der allgemeinen Formel (VI) 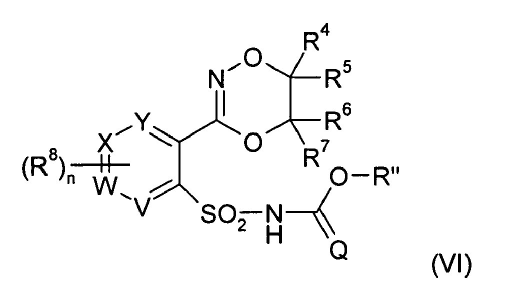 in welchen die Reste V, W, X, Y, R⁴, R⁵, R⁶, R⁷, R⁸, R", Q und n die bereits weiter oben angegebenen allgemeinen, bevorzugten und besonders bevorzugten Bedeutungen aufweisen.

Kollektionen aus Verbindungen der Formel (I) und/oder deren Salzen, die nach den oben genannten Reaktionen synthetisiert werden können, können auch in parallelisierter Weise hergestellt werden, wobei dies in manueller, teilweise automatisierter oder vollständig automatisierter Weise geschehen kann. Dabei ist es beispielsweise möglich, die Reaktionsdurchführung, die Aufarbeitung oder die Reinigung der Produkte bzw. Zwischenstufen zu automatisieren. Insgesamt wird hierunter eine Vorgehensweise verstanden, wie sie beispielsweise durch D. Tiebes in Combinatorial Chemistry - Synthesis, Analysis, Screening (Herausgeber Günther Jung), Verlag Wiley 1999, auf den Seiten 1 bis 34 beschrieben ist.

Zur parallelisierten Reaktionsdurchführung und Aufarbeitung können eine Reihe von im Handel erhältlichen Geräten verwendet werden, beispielsweise Calpyso-Reaktionsblöcke (Caylpso reaction blocks) der Firma Barnstead International, Dubuque, lowa 52004-0797, USA oder Reaktionsstationen (reaction stations) der Firma Radleys, Shirehill, Saffron Walden, Essex, CB 11 3AZ, England oder MultiPROBE Automated Workstations der Firma Perkin Elmer, Waltham, Massachusetts 02451, USA. Für die parallelisierte Aufreinigung von Verbindungen der allgemeinen Formel (I) und deren Salzen beziehungsweise von bei der Herstellung anfallenden Zwischenprodukten stehen unter anderem Chromatographieapparaturen zur Verfügung, beispielsweise der Firma ISCO, Inc., 4700 Superior Street, Lincoln, NE 68504, USA.

Die aufgeführten Apparaturen führen zu einer modularen Vorgehensweise, bei der die einzelnen Arbeitsschritte automatisiert sind, zwischen den Arbeitsschritten jedoch manuelle Operationen durchgeführt werden müssen. Dies kann durch den Einsatz von teilweise oder vollständig integrierten Automationssystemen umgangen werden, bei denen die jeweiligen Automationsmodule beispielsweise durch Roboter bedient werden. Derartige Automationssysteme können zum Beispiel von der Firma Caliper, Hopkinton, MA 01748, USA bezogen werden.

Die Durchführung einzelner oder mehrerer Syntheseschritte kann durch den Einsatz von Polymer-supported reagents/Scavanger-Harze unterstützt werden. In der Fachliteratur sind eine Reihe von Versuchsprotokollen beschrieben, beispielsweise in ChemFiles, Vol. 4, No. 1, Polymer-Supported Scavengers and Reagents for Solution-Phase Synthesis (Sigma-Aldrich).

Neben den hier beschriebenen Methoden kann die Herstellung von Verbindungen der allgemeinen Formel (I) und deren Salzen vollständig oder partiell durch Festphasen unterstützte Methoden erfolgen. Zu diesem Zweck werden einzelne Zwischenstufen oder alle Zwischenstufen der Synthese oder einer für die entsprechende Vorgehensweise angepassten Synthese an ein Syntheseharz gebunden. Festphasen- unterstützte Synthesemethoden sind in der Fachliteratur hinreichend beschrieben, z.B. Barry A. Bunin in "The Combinatorial Index", Verlag Academic Press, 1998 und Combinatorial Chemistry - Synthesis, Analysis, Screening (Herausgeber Günther Jung), Verlag Wiley, 1999. Die Verwendung von Festphasen- unterstützten Synthesemethoden erlaubt eine Reihe von literaturbekannten Protokollen, die wiederum manuell oder automatisiert ausgeführt werden können. Die Reaktionen können beispielsweise mittels IRORI-Technologie in Mikroreaktoren (microreactors) der Firma Nexus Biosystems, 12140 Community Road, Poway, CA92064, USA durchgeführt werden.

Sowohl an fester als auch in flüssiger Phase kann die Durchführung einzelner oder mehrerer Syntheseschritte durch den Einsatz der Mikrowellen-Technologie unterstützt werden. In der Fachliteratur sind eine Reihe von Versuchsprotokollen beschrieben, beispielsweise in Microwaves in Organic and Medicinal Chemistry (Herausgeber C. O. Kappe und a. Stadler), Verlag Wiley, 2005.

Die Herstellung gemäß den hier beschriebenen Verfahren liefert Verbindungen der Formel (I) und deren Salze in Form von Substanzkollektionen, die Bibliotheken genannt werden. Gegenstand der vorliegenden Erfindung sind auch Bibliotheken, die mindestens zwei Verbindungen der Formel (I) und deren Salzen enthalten.

Weiterer Gegenstand der Erfindung ist aufgrund der herbiziden Eigenschaft der Verbindungen der allgemeinen Formel (I) auch die Verwendung der erfindungsgemäßen Verbindungen der allgemeinen Formel (I) als Herbizide zur Bekämpfung von Schadpflanzen.

Weiterer Gegenstand der Erfindung ist aufgrund der herbiziden Eigenschaft der Verbindungen der allgemeinen Formel (I) auch die Verwendung der erfindungsgemäßen Verbindungen der allgemeinen Formel (I) als Herbizide zur Bekämpfung von Schadpflanzen.

Mit den äußeren Bedingungen wie Temperatur, Feuchtigkeit, der Art des verwendeten Herbizids, variiert u.a. die erforderliche Aufwandmenge der Verbindungen der Formel (I). Sie kann innerhalb weiter Grenzen schwanken, z.B. zwischen 0,001 und 10000 g/ha oder mehr Aktivsubstanz, vorzugsweise liegt sie jedoch zwischen 0,5 und 5000 g/ha, bevorzugt zwischen 0.5 und 1000 g/ha und ganz besonders bevorzugt zwischen 0.5 und 500 g/ha.

Die erfindungsgemäßen Verbindungen der Formel (I) (und/oder deren Salze), im folgenden zusammen als "erfindungsgemäße Verbindungen" bezeichnet, weisen eine ausgezeichnete herbizide Wirksamkeit gegen ein breites Spektrum wirtschaftlich wichtiger mono- und dikotyler annueller Schadpflanzen auf. Auch schwer bekämpfbare perennierende Schadpflanzen, die aus Rhizomen, Wurzelstöcken oder anderen Dauerorganen austreiben, werden durch die Wirkstoffe gut erfasst.

Gegenstand der vorliegenden Erfindung ist daher auch ein Verfahren zur Bekämpfung von unerwünschten Pflanzen oder zur Wachstumsregulierung von Pflanzen, vorzugsweise in Pflanzenkulturen, worin eine oder mehrere erfindungsgemäße Verbindung(en) auf die Pflanzen (z.B. Schadpflanzen wie mono- oder dikotyle Unkräuter oder unerwünschte Kulturpflanzen), das Saatgut (z.B. Körner, Samen oder vegetative Vermehrungsorgane wie Knollen oder Sprossteile mit Knospen) oder die Fläche, auf der die Pflanzen wachsen (z.B. die Anbaufläche), ausgebracht werden. Dabei können die erfindungsgemäßen Verbindungen z.B. im Vorsaat- (ggf. auch durch Einarbeitung in den Boden), Vorauflauf- oder Nachauflaufverfahren ausgebracht werden. Im einzelnen seien beispielhaft einige Vertreter der mono- und dikotylen Unkrautflora genannt, die durch die erfindungsgemäßen Verbindungen kontrolliert werden können, ohne dass durch die Nennung eine Beschränkung auf bestimmte Arten erfolgen soll.

Monokotyle Schadpflanzen der Gattungen: Aegilops, Agropyron, Agrostis, Alopecurus, Apera, Avena, Brachiaria, Bromus, Cenchrus, Commelina, Cynodon, Cyperus, Dactyloctenium, Digitaria, Echinochloa, Eleocharis, Eleusine, Eragrostis, Eriochloa, Festuca, Fimbristylis, Heteranthera, Imperata, Ischaemum, Leptochloa, Lolium, Monochoria, Panicum, Paspalum, Phalaris, Phleum, Poa, Rottboellia, Sagittaria, Scirpus, Setaria, Sorghum.

Dikotyle Unkräuter der Gattungen: Abutilon, Amaranthus, Ambrosia, Anoda, Anthemis, Aphanes, Artemisia, Atriplex, Bellis, Bidens, Capsella, Carduus, Cassia, Centaurea, Chenopodium, Cirsium, Convolvulus, Datura, Desmodium, Emex, Erysimum, Euphorbia, Galeopsis, Galinsoga, Galium, Hibiscus, Ipomoea, Kochia, Lamium, Lepidium, Lindernia, Matricaria, Mentha, Mercurialis, Mullugo, Myosotis, Papaver, Pharbitis, Plantago, Polygonum, Portulaca, Ranunculus, Raphanus, Rorippa, Rotala, Rumex, Salsola, Senecio, Sesbania, Sida, Sinapis, Solanum, Sonchus, Sphenoclea, Stellaria, Taraxacum, Thlaspi, Trifolium, Urtica, Veronica, Viola, Xanthium.

Werden die erfindungsgemäßen Verbindungen vor dem Keimen auf die Erdoberfläche appliziert, so wird entweder das Auflaufen der Unkrautkeimlinge vollständig verhindert oder die Unkräuter wachsen bis zum Keimblattstadium heran, stellen jedoch dann ihr Wachstum ein und sterben schließlich nach Ablauf von drei bis vier Wochen vollkommen ab.

Bei Applikation der Wirkstoffe auf die grünen Pflanzenteile im Nachauflaufverfahren tritt nach der Behandlung Wachstumsstop ein und die Schadpflanzen bleiben in dem zum Applikationszeitpunkt vorhandenen Wachstumsstadium stehen oder sterben nach einer gewissen Zeit ganz ab, so dass auf diese Weise eine für die Kulturpflanzen schädliche Unkrautkonkurrenz sehr früh und nachhaltig beseitigt wird.

Obgleich die erfindungsgemäßen Verbindungen eine ausgezeichnete herbizide Aktivität gegenüber mono- und dikotylen Unkräutern aufweisen, werden Kulturpflanzen wirtschaftlich bedeutender Kulturen z.B. dikotyler Kulturen der Gattungen Arachis, Beta, Brassica, Cucumis, Cucurbita, Helianthus, Daucus, Glycine, Gossypium, Ipomoea, Lactuca, Linum, Lycopersicon, Nicotiana, Phaseolus, Pisum, Solanum, Vicia, oder monokotyler Kulturen der Gattungen Allium, Ananas, Asparagus, Avena, Hordeum, Oryza, Panicum, Saccharum, Secale, Sorghum, Triticale, Triticum, Zea, insbesondere Zea und Triticum, abhängig von der Struktur der jeweiligen erfindungsgemäßen Verbindung und deren Aufwandmenge nur unwesentlich oder gar nicht geschädigt. Die vorliegenden Verbindungen eignen sich aus diesen Gründen sehr gut zur selektiven Bekämpfung von unerwünschtem Pflanzenwuchs in Pflanzenkulturen wie landwirtschaftlichen Nutzpflanzungen oder Zierpflanzungen.

Darüber hinaus weisen die erfindungsgemäßen Verbindungen (abhängig von ihrer jeweiligen Struktur und der ausgebrachten Aufwandmenge) hervorragende wachstumsregulatorische Eigenschaften bei Kulturpflanzen auf. Sie greifen regulierend in den pflanzeneigenen Stoffwechsel ein und können damit zur gezielten Beeinflussung von Pflanzeninhaltsstoffen und zur Ernteerleichterung wie z.B. durch Auslösen von Desikkation und Wuchsstauchung eingesetzt werden. Des Weiteren eignen sie sich auch zur generellen Steuerung und Hemmung von unerwünschtem vegetativen Wachstum, ohne dabei die Pflanzen abzutöten. Eine Hemmung des vegetativen Wachstums spielt bei vielen mono- und dikotylen Kulturen eine große Rolle, da beispielsweise die Lagerbildung hierdurch verringert oder völlig verhindert werden kann.

Aufgrund ihrer herbiziden und pflanzenwachstumsregulatorischen Eigenschaften können die Wirkstoffe auch zur Bekämpfung von Schadpflanzen in Kulturen von gentechnisch oder durch konventionelle Mutagenese veränderten Pflanzen eingesetzt werden. Die transgenen Pflanzen zeichnen sich in der Regel durch besondere vorteilhafte Eigenschaften aus, beispielsweise durch Resistenzen gegenüber bestimmten Pestiziden, vor allem bestimmten Herbiziden, Resistenzen gegenüber Pflanzenkrankheiten oder Erregern von Pflanzenkrankheiten wie bestimmten Insekten oder Mikroorganismen wie Pilzen, Bakterien oder Viren. Andere besondere Eigenschaften betreffen z. B. das Erntegut hinsichtlich Menge, Qualität, Lagerfähigkeit, Zusammensetzung und spezieller Inhaltsstoffe. So sind transgene Pflanzen mit erhöhtem Stärkegehalt oder veränderter Qualität der Stärke oder solche mit anderer Fettsäurezusammensetzung des Ernteguts bekannt.

Bevorzugt bezüglich transgener Kulturen ist die Anwendung der erfindungsgemäßen Verbindungen in wirtschaftlich bedeutenden transgenen Kulturen von Nutz- und Zierpflanzen, z. B. von Getreide wie Weizen, Gerste, Roggen, Hafer, Hirse, Reis und Mais oder auch Kulturen von Zuckerrübe, Baumwolle, Soja, Raps, Kartoffel, Tomate, Erbse und anderen Gemüsesorten.

Vorzugsweise können die erfindungsgemäßen Verbindungen als Herbizide in Nutzpflanzenkulturen eingesetzt werden, welche gegenüber den phytotoxischen Wirkungen der Herbizide resistent sind bzw. gentechnisch resistent gemacht worden sind.

Herkömmliche Wege zur Herstellung neuer Pflanzen, die im Vergleich zu bisher vorkommenden Pflanzen modifizierte Eigenschaften aufweisen, bestehen beispielsweise in klassischen Züchtungsverfahren und der Erzeugung von Mutanten. Alternativ können neue Pflanzen mit veränderten Eigenschaften mit Hilfe gentechnischer Verfahren erzeugt werden (siehe z. B. EP-A-0221044, EP-A-0131624). Beschrieben wurden beispielsweise in mehreren Fällen
- gentechnische Veränderungen von Kulturpflanzen zwecks Modifikation der in den Pflanzen synthetisierten Stärke (z. B. WO 92/11376, WO 92/14827, WO 91/19806),
- transgene Kulturpflanzen, welche gegen bestimmte Herbizide vom Typ Glufosinate (vgl. z. B. EP-A-0242236, EP-A-242246) oder Glyphosate (WO 92/00377) oder der Sulfonylharnstoffe (EP-A-0257993, US-A-5013659) resistent sind,
- transgene Kulturpflanzen, beispielsweise Baumwolle, mit der Fähigkeit Bacillus thuringiensis-Toxine (Bt-Toxine) zu produzieren, welche die Pflanzen gegen bestimmte Schädlinge resistent machen (EP-A-0142924, EP-A-0193259).
- transgene Kulturpflanzen mit modifizierter Fettsäurezusammensetzung (WO 91/13972).
- gentechnisch veränderte Kulturpflanzen mit neuen Inhalts- oder Sekundärstoffen z. B. neuen Phytoalexinen, die eine erhöhte Krankheitsresistenz verursachen (EPA 309862, EPA0464461)
- gentechnisch veränderte Pflanzen mit reduzierter Photorespiration, die höhere Erträge und höhere Stresstoleranz aufweisen (EPA 0305398).
- Transgene Kulturpflanzen, die pharmazeutisch oder diagnostisch wichtige Proteine produzieren ("molecular pharming")
- transgene Kulturpflanzen, die sich durch höhere Erträge oder bessere Qualitat auszeichnen
- transgene Kulturpflanzen die sich durch eine Kombinationen z. B. der o. g. neuen Eigenschaften auszeichnen ("gene stacking")

Zahlreiche molekularbiologische Techniken, mit denen neue transgene Pflanzen mit veränderten Eigenschaften hergestellt werden können, sind im Prinzip bekannt; siehe z. B. I. Potrykus und G. Spangenberg (eds.) Gene Transfer to Plants, Springer Lab Manual (1995), Springer Verlag Berlin, Heidelberg. oder Christou, "Trends in Plant Science" 1 (1996) 423-431).

Für derartige gentechnische Manipulationen können Nucleinsäuremoleküle in Plasmide eingebracht werden, die eine Mutagenese oder eine Sequenzveränderung durch Rekombination von DNA-Sequenzen erlauben. Mit Hilfe von Standardverfahren können z. B. Basenaustausche vorgenommen, Teilsequenzen entfernt oder natürliche oder synthetische Sequenzen hinzugefügt werden. Für die Verbindung der DNA-Fragmente untereinander können an die Fragmente Adaptoren oder Linker angesetzt werden, siehe z. B. Sambrook et al., 1989, Molecular Cloning, A Laboratory Manual, 2. Aufl. Cold Spring Harbor Laboratory Press, Cold Spring Harbor, NY; oder Winnacker "Gene und Klone", VCH Weinheim 2. Auflage 1996

Die Herstellung von Pflanzenzellen mit einer verringerten Aktivität eines Genprodukts kann beispielsweise erzielt werden durch die Expression mindestens einer entsprechenden antisense-RNA, einer sense-RNA zur Erzielung eines Cosuppressionseffektes oder die Expression mindestens eines entsprechend konstruierten Ribozyms, das spezifisch Transkripte des obengenannten Genprodukts spaltet.

Hierzu können zum einen DNA-Moleküle verwendet werden, die die gesamte codierende Sequenz eines Genprodukts einschließlich eventuell vorhandener flankierender Sequenzen umfassen, als auch DNA-Moleküle, die nur Teile der codierenden Sequenz umfassen, wobei diese Teile lang genug sein müssen, um in den Zellen einen antisense-Effekt zu bewirken. Möglich ist auch die Verwendung von DNA-Sequenzen, die einen hohen Grad an Homologie zu den codiereden Sequenzen eines Genprodukts aufweisen, aber nicht vollkommen identisch sind.

Bei der Expression von Nucleinsäuremolekülen in Pflanzen kann das synthetisierte Protein in jedem beliebigen Kompartiment der pflanzlichen Zelle lokalisiert sein. Um aber die Lokalisation in einem bestimmten Kompartiment zu erreichen, kann z. B. die codierende Region mit DNA-Sequenzen verknüpft werden, die die Lokalisierung in einem bestimmten Kompartiment gewährleisten. Derartige Sequenzen sind dem Fachmann bekannt (siehe beispielsweise Braun et al., EMBO J. 11 (1992), 3219-3227; Wolter et al., Proc. Natl. Acad. Sci. USA 85 (1988), 846-850; Sonnewald et al., Plant J. 1 (1991), 95-106). Die Expression der Nukleinsäuremoleküle kann auch in den Organellen der Pflanzenzellen stattfinden.

Die transgenen Pflanzenzellen können nach bekannten Techniken zu ganzen Pflanzen regeneriert werden. Bei den transgenen Pflanzen kann es sich prinzipiell um Pflanzen jeder beliebigen Pflanzenspezies handeln, d.h., sowohl monokotyle als auch dikotyle Pflanzen.

So sind transgene Pflanzen erhältlich, die veränderte Eigenschaften durch Überexpression, Suppression oder Inhibierung homologer (= natürlicher) Gene oder Gensequenzen oder Expression heterologer (= fremder) Gene oder Gensequenzen aufweisen.

Vorzugsweise können die erfindungsgemäßen Verbindungen (I) in transgenen Kulturen eingesetzt werden, welche gegen Wuchsstoffe, wie z. B. Dicamba oder gegen Herbizide, die essentielle Pflanzenenzyme, z. B. Acetolactatsynthasen (ALS), EPSP Synthasen, Glutaminsynthasen (GS) oder Hydroxyphenylpyruvat Dioxygenasen (HPPD) hemmen, respektive gegen Herbizide aus der Gruppe der Sulfonylharnstoffe, der Glyphosate, Glufosinate oder Benzoylisoxazole und analogen Wirkstoffe, resistent sind.

Bei der Anwendung der erfindungsgemäßen Wirkstoffe in transgenen Kulturen treten neben den in anderen Kulturen zu beobachtenden Wirkungen gegenüber Schadpflanzen oftmals Wirkungen auf, die für die Applikation in der jeweiligen transgenen Kultur spezifisch sind, beispielsweise ein verändertes oder speziell erweitertes Unkrautspektrum, das bekämpft werden kann, veränderte Aufwandmengen, die für die Applikation eingesetzt werden können, vorzugsweise gute Kombinierbarkeit mit den Herbiziden, gegenüber denen die transgene Kultur resistent ist, sowie Beeinflussung von Wuchs und Ertrag der transgenen Kulturpflanzen.

Gegenstand der Erfindung ist deshalb auch die Verwendung der erfindungsgemäßen Verbindungen der Formel (I) als Herbizide zur Bekämpfung von Schadpflanzen in transgenen Kulturpflanzen.

Die erfindungsgemäßen Verbindungen können in Form von Spritzpulvern, emulgierbaren Konzentraten, versprühbaren Lösungen, Stäubemitteln oder Granulaten in den üblichen Zubereitungen angewendet werden. Gegenstand der Erfindung sind deshalb auch herbizide und pflanzenwachstumsregulierende Mittel, welche die erfindungsgemäßen Verbindungen enthalten.

Die erfindungsgemäßen Verbindungen können auf verschiedene Art formuliert werden, je nachdem welche biologischen und/oder chemisch-physikalischen Parameter vorgegeben sind. Als Formulierungsmöglichkeiten kommen beispielsweise in Frage: Spritzpulver (WP), wasserlösliche Pulver (SP), wasserlösliche Konzentrate, emulgierbare Konzentrate (EC), Emulsionen (EW), wie Öl-in-Wasser- und Wasser-in-Öl-Emulsionen, versprühbare Lösungen, Suspensionskonzentrate (SC), Dispersionen auf Öl- oder Wasserbasis, ölmischbare Lösungen, Kapselsuspensionen (CS), Stäubemittel (DP), Beizmittel, Granulate für die Streu- und Bodenapplikation, Granulate (GR) in Form von Mikro-, Sprüh-, Aufzugs- und Adsorptionsgranulaten, wasserdispergierbare Granulate (WG), wasserlösliche Granulate (SG), ULV-Formulierungen, Mikrokapseln und Wachse. Diese einzelnen Formulierungstypen sind im Prinzip bekannt und werden beispielsweise beschrieben in: Winnacker-Küchler, "Chemische Technologie", Band 7, C. Hanser Verlag München, 4. Aufl. 1986; Wade van Valkenburg, "Pesticide Formulations", Marcel Dekker, N.Y., 1973; K. Martens, "Spray Drying" Handbook, 3rd Ed. 1979, G. Goodwin Ltd. London.

Die notwendigen Formulierungshilfsmittel wie Inertmaterialien, Tenside, Lösungsmittel und weitere Zusatzstoffe sind ebenfalls bekannt und werden beispielsweise beschrieben in: Watkins, "Handbook of Insecticide Dust Diluents and Carriers", 2nd Ed., Darland Books, Caldwell N.J., H.v. Olphen, "Introduction to Clay Colloid Chemistry"; 2nd Ed., J. Wiley & Sons, N.Y.; C. Marsden, "Solvents Guide"; 2nd Ed., Interscience, N.Y. 1963; McCutcheon's "Detergents and Emulsifiers Annual", MC Publ. Corp., Ridgewood N.J.; Sisley and Wood, "Encyclopedia of Surface Active Agents", Chem. Publ. Co. Inc., N.Y. 1964; Schönfeldt, "Grenzflächenaktive Äthylenoxidaddukte", Wiss. Verlagsgesell., Stuttgart 1976; Winnacker-Küchler, "Chemische Technologie", Band 7, C. Hanser Verlag München, 4. Aufl. 1986.

Auf der Basis dieser Formulierungen lassen sich auch Kombinationen mit anderen pestizid wirksamen Stoffen, wie z.B. Insektiziden, Akariziden, Herbiziden, Fungiziden, sowie mit Safenern, Düngemitteln und/oder Wachstumsregulatoren herstellen, z.B. in Form einer Fertigformulierung oder als Tankmix.

Spritzpulver sind in Wasser gleichmäßig dispergierbare Präparate, die neben dem Wirkstoff außer einem Verdünnungs- oder Inertstoff noch Tenside ionischer und/oder nichtionischer Art (Netzmittel, Dispergiermittel), z.B. polyoxyethylierte Alkylphenole, polyoxethylierte Fettalkohole, polyoxethylierte Fettamine, Fettalkoholpolyglykolethersulfate, Alkansulfonate, Alkylbenzolsulfonate, ligninsulfonsaures Natrium, 2,2'-dinaphthylmethan-6,6'-disulfonsaures Natrium, dibutylnaphthalin-sulfonsaures Natrium oder auch oleoylmethyltaurinsaures Natrium enthalten. Zur Herstellung der Spritzpulver werden die herbiziden Wirkstoffe beispielsweise in üblichen Apparaturen wie Hammermühlen, Gebläsemühlen und Luftstrahlmühlen feingemahlen und gleichzeitig oder anschließend mit den Formulierungshilfsmitteln vermischt.

Emulgierbare Konzentrate werden durch Auflösen des Wirkstoffes in einem organischen Lösungsmittel z.B. Butanol, Cyclohexanon, Dimethylformamid, Xylol oder auch höhersiedenden Aromaten oder Kohlenwasserstoffen oder Mischungen der organischen Lösungsmittel unter Zusatz von einem oder mehreren Tensiden ionischer und/oder nichtionischer Art (Emulgatoren) hergestellt. Als Emulgatoren können beispielsweise verwendet werden: Alkylarylsulfonsaure Calzium-Salze wie Ca-Dodecylbenzolsulfonat oder nichtionische Emulgatoren wie Fettsäurepolyglykolester, Alkylarylpolyglykolether, Fettalkoholpolyglykolether, Propylenoxid-Ethylenoxid-Kondensationsprodukte, Alkylpolyether, Sorbitanester wie z.B. Sorbitanfettsäureester oder Polyoxethylensorbitanester wie z.B. Polyoxyethylensorbitanfettsäureester.

Stäubemittel erhält man durch Vermahlen des Wirkstoffes mit fein verteilten festen Stoffen, z.B. Talkum, natürlichen Tonen, wie Kaolin, Bentonit und Pyrophyllit, oder Diatomeenerde.

Suspensionskonzentrate können auf Wasser- oder Ölbasis sein. Sie können beispielsweise durch Nass-Vermahlung mittels handelsüblicher Perlmühlen und gegebenenfalls Zusatz von Tensiden, wie sie z.B. oben bei den anderen Formulierungstypen bereits aufgeführt sind, hergestellt werden.

Emulsionen, z.B. Öl-in-Wasser-Emulsionen (EW), lassen sich beispielsweise mittels Rührern, Kolloidmühlen und/oder statischen Mischern unter Verwendung von wässrigen organischen Lösungsmitteln und gegebenenfalls Tensiden, wie sie z.B. oben bei den anderen Formulierungstypen bereits aufgeführt sind, herstellen.

Granulate können entweder durch Verdüsen des Wirkstoffes auf adsorptionsfähiges, granuliertes Inertmaterial hergestellt werden oder durch Aufbringen von Wirkstoffkonzentraten mittels Klebemitteln, z.B. Polyvinylalkohol, polyacrylsaurem Natrium oder auch Mineralölen, auf die Oberfläche von Trägerstoffen wie Sand, Kaolinite oder von granuliertem Inertmaterial. Auch können geeignete Wirkstoffe in der für die Herstellung von Düngemittelgranulaten üblichen Weise - gewünschtenfalls in Mischung mit Düngemitteln - granuliert werden.

Wasserdispergierbare Granulate werden in der Regel nach den üblichen Verfahren wie Sprühtrocknung, Wirbelbett-Granulierung, Teller-Granulierung, Mischung mit Hochgeschwindigkeitsmischern und Extrusion ohne festes Inertmaterial hergestellt.

Zur Herstellung von Teller-, Fließbett-, Extruder- und Sprühgranulate siehe z.B. Verfahren in "Spray-Drying Handbook" 3rd ed. 1979, G. Goodwin Ltd., London; J.E. Browning, "Agglomeration", Chemical and Engineering 1967, Seiten 147 ff; "Perry's Chemical Engineer's Handbook", 5th Ed., McGraw-Hill, New York 1973, S. 8-57.

Für weitere Einzelheiten zur Formulierung von Pflanzenschutzmitteln siehe z.B. G.C. Klingman, "Weed Control as a Science", John Wiley and Sons, Inc., New York, 1961, Seiten 81-96 und J.D. Freyer, S.A. Evans, "Weed Control Handbook", 5th Ed., Blackwell Scientific Publications, Oxford, 1968, Seiten 101-103.

Die agrochemischen Zubereitungen enthalten in der Regel 0.1 bis 99 Gew.-%, insbesondere 0.1 bis 95 Gew.-%, erfindungsgemäße Verbindungen.

In Spritzpulvern beträgt die Wirkstoffkonzentration z.B. etwa 10 bis 90 Gew.-%, der Rest zu 100 Gew.-% besteht aus üblichen Formulierungsbestandteilen. Bei emulgierbaren Konzentraten kann die Wirkstoffkonzentration etwa 1 bis 90, vorzugsweise 5 bis 80 Gew.-% betragen. Staubförmige Formulierungen enthalten 1 bis 30 Gew.-% Wirkstoff, vorzugsweise meistens 5 bis 20 Gew.-% an Wirkstoff, versprühbare Lösungen enthalten etwa 0.05 bis 80, vorzugsweise 2 bis 50 Gew.-% Wirkstoff. Bei wasserdispergierbaren Granulaten hängt der Wirkstoffgehalt zum Teil davon ab, ob die wirksame Verbindung flüssig oder fest vorliegt und welche Granulierhilfsmittel, Füllstoffe usw. verwendet werden. Bei den in Wasser dispergierbaren Granulaten liegt der Gehalt an Wirkstoff beispielsweise zwischen 1 und 95 Gew.-%, vorzugsweise zwischen 10 und 80 Gew.-%.

Daneben enthalten die genannten Wirkstoff-Formulierungen gegebenenfalls die jeweils üblichen Haft-, Netz-, Dispergier-, Emulgier-, Penetrations-, Konservierungs-, Frostschutz- und Lösungsmittel, Füll-, Träger- und Farbstoffe, Entschäumer, Verdunstungshemmer und den pH-Wert und die Viskosität beeinflussende Mittel.

Die Verbindungen der allgemeinen Formel (I) oder deren Salze können als solche oder in Form ihrer Zubereitungen (Formulierungen) mit anderen pestizid wirksamen Stoffen, wie z. B. Insektiziden, Akariziden, Nematiziden, Herbiziden, Fungiziden, Safenern, Düngemitteln und/oder Wachstumsregulatoren kombiniert eingesetzt werden, z. B. als Fertigformulierung oder als Tankmischungen.

Als Kombinationspartner für die erfindungsgemäßen Verbindungen in Mischungsformulierungen oder im Tank-Mix sind beispielsweise bekannte Wirkstoffe, die auf einer Inhibition von beispielsweise Acetolactat-Synthase, Acetyl-CoA-Carboxylase, Cellulose-Synthase, Enolpyruvylshikimat-3-phosphat-Synthase, Glutamin-Synthetase, p-Hydroxyphenylpyruvat-Dioxygenase, Phytoendesaturase, Photosystem I, Photosystem II, Protoporphyrinogen-Oxidase beruhen, einsetzbar, wie sie z.B. aus Weed Research 26 (1986) 441-445 oder "The Pesticide Manual", 13th edition, The British Crop Protection Council and the Royal Soc. of Chemistry, 2003 und dort zitierter Literatur beschrieben sind. Als bekannte Herbizide oder Pflanzenwachstumsregulatoren, die mit den erfindungsgemäßen Verbindungen kombiniert werden können, sind z.B. folgende Wirkstoffe zu nennen (die Verbindungen sind entweder mit dem "common name" nach der International Organization for Standardization (ISO) oder mit dem chemischen Namen oder mit der Codenummer bezeichnet) und umfassen stets sämtliche Anwendungsformen wie Säuren, Salze, Ester und Isomere wie Stereoisomere und optische Isomere. Dabei sind beispielhaft eine und zum Teil auch mehrere Anwendungsformen genannt:
Acetochlor, Acibenzolar, Acibenzolar-S-methyl, Acifluorfen, Acifluorfen-sodium, Aclonifen, Alachlor, Allidochlor, Alloxydim, Alloxydim-sodium, Ametryn, Amicarbazone, Amidochlor, Amidosulfuron, Aminocyclopyrachlor, Aminopyralid, Amitrole, Ammoniumsulfamat, Ancymidol, Anilofos, Asulam, Atrazine, Azafenidin, Azimsulfuron, Aziprotryn, BAH-043, BAS-140H, BAS-693H, BAS-714H, BAS-762H, BAS-776H, BAS-800H, Beflubutamid, Benazolin, Benazolin-ethyl, bencarbazone, Benfluralin, Benfuresate, Bensulide, Bensulfuron-methyl, Bentazone, Benzfendizone, Benzobicyclon, Benzofenap, Benzofluor, Benzoylprop, Bifenox, Bilanafos, Bilanafosnatrium, Bispyribac, Bispyribac-natrium, Bromacil, Bromobutide, Bromofenoxim, Bromoxynil, Bromuron, Buminafos, Busoxinone, Butachlor, Butafenacil, Butamifos, Butenachlor, Butralin, Butroxydim, Butylate, Cafenstrole, Carbetamide, Carfentrazone, Carfentrazone-ethyl, Chlomethoxyfen, Chloramben, Chlorazifop, Chlorazifop-butyl, Chlorbromuron, Chlorbufam, Chlorfenac, Chlorfenac-natrium, Chlorfenprop, Chlorflurenol, Chlorflurenol-methyl, Chloridazon, Chlorimuron, Chlorimuron-ethyl, Chlormequat-chlorid, Chlornitrofen, Chlorophthalim, Chlorthaldimethyl, Chlorotoluron, Chlorsulfuron, Cinidon, Cinidon-ethyl, Cinmethylin, Cinosulfuron, Clethodim, Clodinafop Clodinafop-propargyl, Clofencet, Clomazone, Clomeprop, Cloprop, Clopyralid, Cloransulam, Cloransulam-methyl, Cumyluron, Cyanamide, Cyanazine, Cyclanilide, Cycloate, Cyclosulfamuron, Cycloxydim, Cycluron, Cyhalofop, Cyhalofop-butyl, Cyperquat, Cyprazine, Cyprazole, 2,4-D, 2,4-DB, Daimuron/Dymron, Dalapon, Daminozide, Dazomet, n-Decanol, Desmedipham, Desmetryn, Detosyl-Pyrazolate (DTP), Diallate, Dicamba, Dichlobenil, Dichlorprop, Dichlorprop-P, Diclofop, Diclofop-methyl, Diclofop-P-methyl, Diclosulam, Diethatyl, Diethatyl-ethyl, Difenoxuron, Difenzoquat, Diflufenican, Diflufenzopyr, Diflufenzopyrnatrium, Dimefuron, Dikegulac-sodium, Dimefuron, Dimepiperate, Dimethachlor, Dimethametryn, Dimethenamid, Dimethenamid-P, Dimethipin, Dimetrasulfuron, Dinitramine, Dinoseb, Dinoterb, Diphenamid, Dipropetryn, Diquat, Diquat-dibromide, Dithiopyr, Diuron, DNOC, Eglinazine-ethyl, Endothal, EPTC, Esprocarb, Ethalfluralin, Ethametsulfuron-methyl, Ethephon, Ethidimuron, Ethiozin, Ethofumesate, Ethoxyfen, Ethoxyfen-ethyl, Ethoxysulfuron, Etobenzanid, F-5331, d.h. N-[2-Chlor-4-fluor-5-[4-(3fluorpropyl)-4,5-dihydro-5-oxo-1H-tetrazol-1-yl]-phenyl]-ethansulfonamid, Fenoprop, Fenoxaprop, Fenoxaprop-P, Fenoxaprop-ethyl, Fenoxaprop-P-ethyl, Fenoxasulfone, Fentrazamide, Fenuron, Flamprop, Flamprop-M-isopropyl, Flamprop-M-methyl, Flazasulfuron, Florasulam, Fluazifop, Fluazifop-P, Fluazifopbutyl, Fluazifop-P-butyl, Fluazolate, Flucarbazone, Flucarbazone-sodium, Flucetosulfuron, Fluchloralin, Flufenacet (Thiafluamide), Flufenpyr, Flufenpyr-ethyl, Flumetralin, Flumetsulam, Flumiclorac, Flumiclorac-pentyl, Flumioxazin, Flumipropyn, Fluometuron, Fluorodifen, Fluoroglycofen, Fluoroglycofen-ethyl, Flupoxam, Flupropacil, Flupropanate, Flupyrsulfuron, Flupyrsulfuron-methyl-sodium, Flurenol, Flurenol-butyl, Fluridone, Flurochloridone, Fluroxypyr, Fluroxypyr-meptyl, Flurprimidol, Flurtamone, Fluthiacet, Fluthiacet-methyl, Fluthiamide, Fomesafen, Foramsulfuron, Forchlorfenuron, Fosamine, Furyloxyfen, Gibberellinsäure, Glufosinate, L-Glufosinate, L-Glufosinate-ammonium, Glufosinate-ammonium, Glyphosate, Glyphosate-isopropylammonium, H-9201, Halosafen, Halosulfuron, Halosulfuron-methyl, Haloxyfop, Haloxyfop-P, Haloxyfop-ethoxyethyl, Haloxyfop-P-ethoxyethyl, Haloxyfop-methyl, Haloxyfop-P-methyl, Hexazinone, HNPC-9908, HOK-201, HW-02, Imazamethabenz, Imazamethabenz-methyl, Imazamox, Imazapic, Imazapyr, Imazaquin, Imazethapyr, Imazosulfuron, Inabenfide, Indanofan, Indaziflam, Indolessigsäure (IAA), 4-Indol-3-ylbuttersäure (IBA), lodosulfuron, Iodosulfuron-methyl-natrium, Ioxynil, Ipfencarbazone, Isocarbamid, Isopropalin, Isoproturon, Isouron, Isoxaben, Isoxachlortole, Isoxaflutole, Isoxapyrifop, IDH-100, KUH-043, KUH-071, Karbutilate, Ketospiradox, Lactofen, Lenacil, Linuron, Maleinsäurehydrazid, MCPA, MCPB, MCPB-methyl, -ethyl und -natrium, Mecoprop, Mecoprop-natrium, Mecoprop-butotyl, Mecoprop-P-butotyl, Mecoprop-P-dimethylammonium, Mecoprop-P-2-ethylhexyl, Mecoprop-P-kalium, Mefenacet, Mefluidide, Mepiquat-chlorid, Mesosulfuron, Mesosulfuron-methyl, Mesotrione, Methabenzthiazuron, Metam, Metamifop, Metamitron, Metazachlor, Methazole, Methiozolin, Methoxyphenone, Methyldymron, 1-Methylcyclopropen, Methylisothiocyanat, Metobenzuron, Metobenzuron, Metobromuron, Metolachlor, S-Metolachlor, Metosulam, Metoxuron, Metribuzin, Metsulfuron, Metsulfuron-methyl, Molinate, Monalide, Monocarbamide, Monocarbamide-dihydrogensulfat, Monolinuron, Monosulfuron, Monuron, MT 128, MT-5950, d. h. N-[3-Chlor-4-(1-methylethyl)-phenyl]-2-methylpentanamid, NGGC-011, Naproanilide, Napropamide, Naptalam, NC-310, d.h. 4-(2,4-dichlorobenzoyl)-1-methyl-5-benzyloxypyrazole, Neburon, Nicosulfuron, Nipyraclofen, Nitralin, Nitrofen, Nitrophenolat-natrium (Isomerengemisch), Nitrofluorfen, Nonansäure, Norflurazon, Orbencarb, Orthosulfamuron, Oryzalin, Oxadiargyl, Oxadiazon, Oxasulfuron, Oxaziclomefone, Oxyfluorfen, Paclobutrazol, Paraquat, Paraquat-dichlorid, Pelargonsäure (Nonansäure), Pendimethalin, Pendralin, Penoxsulam, Pentanochlor, Pentoxazone, Perfluidone, Pethoxamid, Phenisopham, Phenmedipham, Phenmedipham-ethyl, Picloram, Picolinafen, Pinoxaden, Piperophos, Pirifenop, Pirifenop-butyl, Pretilachlor, Primisulfuron, Primisulfuron-methyl, Probenazole, Profluazol, Procyazine, Prodiamine, Prifluraline, Profoxydim, Prohexadione, Prohexadione-calcium, Prohydrojasmone, Prometon, Prometryn, Propachlor, Propanil, Propaquizafop, Propazine, Propham, Propisochlor, Propoxycarbazone, Propoxycarbazone-natrium, Propyrisulfuron, Propyzamide, Prosulfalin, Prosulfocarb, Prosulfuron, Prynachlor, Pyraclonil, Pyraflufen, Pyraflufen-ethyl, Pyrasulfotole, Pyrazolynate (Pyrazolate), Pyrazosulfuron-ethyl, Pyrazoxyfen, Pyribambenz, Pyribambenz-isopropyl, Pyribenzoxim, Pyributicarb, Pyridafol, Pyridate, Pyriftalid, Pyriminobac, Pyriminobacmethyl, Pyrimisulfan, Pyrithiobac, Pyrithiobac-natrium, Pyroxasulfone, Pyroxsulam, Quinclorac, Quinmerac, Quinoclamine, Quizalofop, Quizalofop-ethyl, Quizalofop-P, Quizalofop-P-ethyl, Quizalofop-P-tefuryl, Rimsulfuron, Secbumeton, Sethoxydim, Siduron, Simazine, Simetryn, SN-106279, Sulcotrione, Sulfallate (CDEC), Sulfentrazone, Sulfometuron, Sulfometuron-methyl, Sulfosate (Glyphosatetrimesium), Sulfosulfuron, SYN-523, SYP-249, SYP-298, SYP-300, Tebutam, Tebuthiuron, Tecnazene, Tefuryltrione, Tembotrione, Tepraloxydim, Terbacil, Terbucarb, Terbuchlor, Terbumeton, Terbuthylazine, Terbutryn, TH-547, Thenylchlor, Thiafluamide, Thiazafluron, Thiazopyr, Thidiazimin, Thidiazuron, Thiencarbazone, Thiencarbazone-methyl, Thifensulfuron, Thifensulfuron-methyl, Thiobencarb, Tiocarbazil, Topramezone, Tralkoxydim, Triallate, Triasulfuron, Triaziflam, Triazofenamide, Tribenuron, Tribenuron-methyl, Trichloressigsäure (TCA), Triclopyr, Tridiphane, Trietazine, Trifloxysulfuron, Trifloxysulfuron-natrium, Trifluralin, Triflusulfuron, Triflusulfuron-methyl, Trimeturon, Trinexapac, Trinexapacethyl, Tritosulfuron, Tsitodef, Uniconazole, Uniconazole-P, Vernolate, ZJ-0166, ZJ-0270, ZJ-0543, ZJ-0862 sowie die folgenden Verbindungen

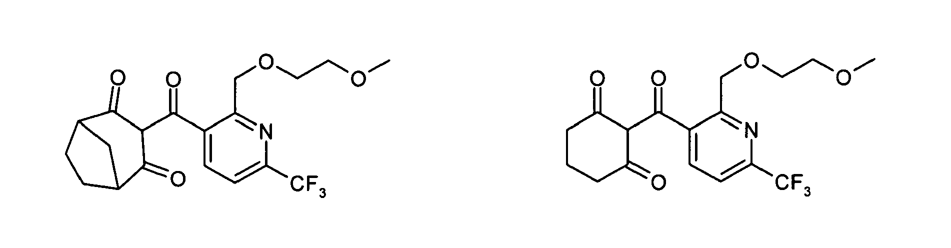

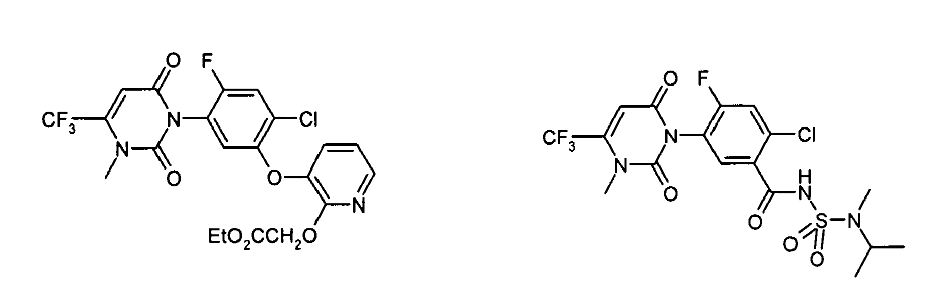

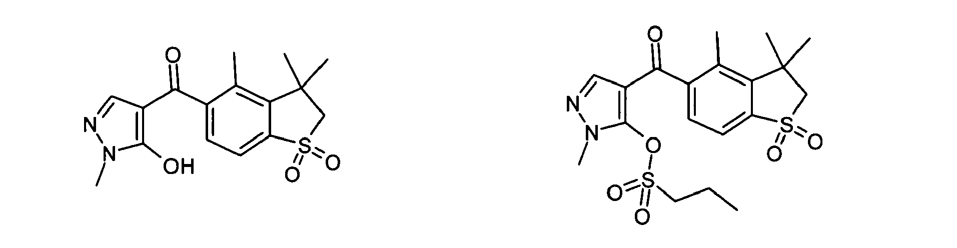

Von besonderem Interesse ist die selektive Bekämpfung von Schadpflanzen in Kulturen von Nutz- und Zierpflanzen. Obgleich die erfindungsgemäßen Verbindungen der allgemeinen Formel (I) bereits in vielen Kulturen sehr gute bis ausreichende Selektivität aufweisen, können prinzipiell in einigen Kulturen und vor allem auch im Falle von Mischungen mit anderen Herbiziden, die weniger selektiv sind, Phytotoxizitäten an den Kulturpflanzen auftreten. Diesbezüglich sind Kombinationen erfindungsgemäßer Verbindungen der allgemeinen Formel (I) von besonderem Interesse, welche die Verbindungen der allgemeinen Formel (I) bzw. deren Kombinationen mit anderen Herbiziden oder Pestiziden und Safenern enthalten. Die Safener, welche in einem antidotisch wirksamen Gehalt eingesetzt werden, reduzieren die phytotoxischen Nebenwirkungen der eingesetzten Herbizide/Pestizide, z. B. in wirtschaftlich bedeutenden Kulturen wie Getreide (Weizen, Gerste, Roggen, Mais, Reis, Hirse), Zuckerrübe, Zuckerrohr, Raps, Baumwolle und Soja, vorzugswiese Getreide. Folgende Gruppen von Verbindungen kommen beispielsweise als Safener für die Verbindungen (I) alleinig oder aber in deren Kombinationen mit wieteren Pestiziden in Frage:
Die Safener sind vorzugsweise ausgewählt aus der Gruppe bestehend aus:
   S1) Verbindungen der Formel (S1), 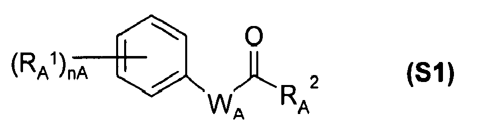 wobei die Symbole und Indizes folgende Bedeutungen haben:
      - n_{A}: ist eine natürliche Zahl von 0 bis 5, vorzugsweise 0 bis 3;
      - R_{A}¹: ist Halogen, (C₁-C₄)Alkyl, (C₁-C₄)Alkoxy, Nitro oder (C₁-C₄)Haloalkyl;
      - W_{A}: ist ein unsubstituierter oder substituierter divalenter heterocyclischer Rest aus der Gruppe der teilungesättigten oder aromatischen Fünfring-Heterocyclen mit 1 bis 3 Heteroringatomen aus der Gruppe N und O, wobei mindestens ein N- Atom und höchstens ein O-Atom im Ring enthalten ist, vorzugsweise ein Rest aus der Gruppe (W_{A}¹) bis (W_{A}⁴),

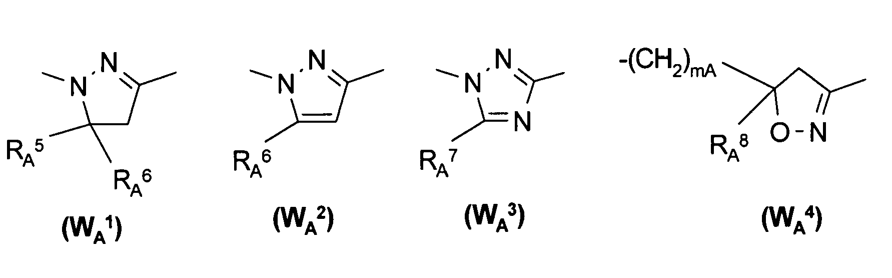

      - m_{A}: ist 0 oder 1;
      - R_{A}²: ist OR_{A}³, SR_{A}³ oder NR_{A}³R_{A}⁴ oder ein gesättigter oder ungesättigter 3- bis 7- gliedriger Heterocyclus mit mindestens einem N-Atom und bis zu 3 Hetero- atomen, vorzugsweise aus der Gruppe O und S, der über das N-Atom mit der Carbonylgruppe in (S1) verbunden ist und unsubstituiert oder durch Reste aus der Gruppe (C₁-C₄)Alkyl, (C₁-C₄)Alkoxy oder gegebenenfalls substituiertes
      - R_{A}³: Phenyl substituiert ist, vorzugsweise ein Rest der Formel OR_{A}³, NHR_{A}⁴ oder N(CH₃)₂, insbesondere der Formel OR_{A}³; ist Wasserstoff oder ein unsubstituierter oder substituierter aliphatischer Kohlenwasserstoffrest, vorzugsweise mit insgesamt 1 bis 18 C-Atomen;
      - R_{A}⁴: ist Wasserstoff, (C₁-C₆)Alkyl, (C₁-C₆)Alkoxy oder substituiertes oder unsubstituiertes Phenyl;
      - R_{A}⁵: ist H, (C₁-C₈)Alkyl, (C₁-C₈)Haloalkyl, (C₁-C₄)Alkoxy(C₁-C₈)Alkyl, Cyano oder COOR_{A}⁹, worin R_{A}⁹ Wasserstoff, (C₁-C₈)Alkyl, (C₁-C₈)Haloalkyl, (C₁-C₄)Alkoxy-(C₁-C₄)alkyl, (C₁-C₆)Hydroxyalkyl, (C₃-C₁₂)Cycloalkyl oder Tri- (C₁-C₄)-alkyl-silyl ist;
      - R_{A}⁶, R_{A}⁷, R_{A}⁸: sind gleich oder verschieden Wasserstoff, (C₁-C₈)Alkyl, (C₁-C₈)Halo- alkyl, (C₃-C₁₂)Cycloalkyl oder substituiertes oder unsubstituiertes Phenyl;
   vorzugsweise:
   a) Verbindungen vom Typ der Dichlorphenylpyrazolin-3-carbonsäure (S1^{a}), vorzugsweise Verbindungen wie 1-(2,4-Dichlorphenyl)-5-(ethoxycarbonyl)-5-methyl-2-pyrazolin-3-carbonsäure, 1-(2,4-Dichlorphenyl)-5-(ethoxycarbonyl)-5-methyl-2-pyrazolin-3-carbonsäure-ethylester (S1-1) ("Mefenpyr-diethyl"), und verwandte Verbindungen, wie sie in der WO-A-91/07874 beschrieben sind;
   b) Derivate der Dichlorphenylpyrazolcarbonsäure (S1^{b}), vorzugsweise Verbindungen wie 1-(2,4-Dichlorphenyl)-5-methyl-pyrazol-3-carbonsäureethyl-ester (S1-2), 1-(2,4-Dichlorphenyl)-5-isopropyl-pyrazol-3-carbonsäureethyl-ester (S1-3), 1-(2,4-Dichlorphenyl)-5-(1,1-dimethyl-ethyl)pyrazol-3-carbonsäureethyl-ester (S1-4) und verwandte Verbindungen, wie sie in EP-A-333 131 und EP-A-269 806 beschrieben sind;
   c) Derivate der 1,5-Diphenylpyrazol-3-carbonsäure (S1^{c}), vorzugsweise Verbindungen wie 1-(2,4-Dichlorphenyl)-5-phenylpyrazol-3-carbonsäureethyl-ester (S1-5), 1-(2-Chlorphenyl)-5-phenylpyrazol-3-carbonsäuremethylester (S1-6) und verwandte Verbindungen wie sie beispielsweise in der EP-A-268554 beschrieben sind;
   d) Verbindungen vom Typ der Triazolcarbonsäuren (S1^{d}), vorzugsweise Verbindungen wie Fenchlorazol(-ethylester), d.h. 1-(2,4-Dichlorphenyl)-5-trichlormethyl-(1H)-1,2,4-triazol-3-carbonsäureethylester (S1-7), und verwandte Verbindungen wie sie in EP-A-174 562 und EP-A-346 620 beschrieben sind;
   e) Verbindungen vom Typ der 5-Benzyl- oder 5-Phenyl-2-isoxazolin-3-carbonsäure oder der 5,5-Diphenyl-2-isoxazolin-3-carbonsäure (S1^{e}), vorzugsweise Verbindungen wie 5-(2,4-Dichlorbenzyl)-2-isoxazolin-3-carbonsäureethylester (S1-8) oder 5-Phenyl-2-isoxazolin-3-carbonsäureethylester (S1-9) und verwandte Verbindungen, wie sie in WO-A-91/08202 beschrieben sind, bzw. 5,5-Diphenyl-2-isoxazolin-carbonsäure (S1-10) oder 5,5-Diphenyl-2-isoxazolin-carbonsäureethylester (S1-11) ("Isoxadifen-ethyl") oder -n-propylester (S1-12) oder der 5-(4-Fluorphenyl)-5-phenyl-2-isoxazolin-3-carbonsäureethylester (S1-13), wie sie in der Patentanmeldung WO-A-95/07897 beschrieben sind.
S2) Chinolinderivate der Formel (S2), 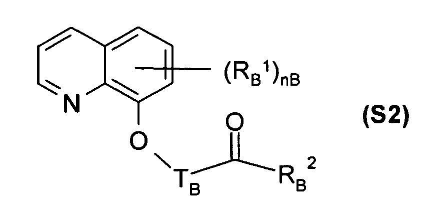 wobei die Symbole und Indizes folgende Bedeutungen haben:
   - R_{B}¹: ist Halogen, (C₁-C₄)Alkyl, (C₁-C₄)Alkoxy, Nitro oder (C₁-C₄)Haloalkyl;
   - n_{B}: ist eine natürliche Zahl von 0 bis 5, vorzugsweise 0 bis 3;
   - R_{B}²: ist OR_{B}³, SR_{B}³ oder NR_{B}³R_{B}⁴ oder ein gesättigter oder ungesättigter 3- bis 7- gliedriger Heterocyclus mit mindestens einem N-Atom und bis zu 3 Heteroatomen, vorzugsweise aus der Gruppe O und S, der über das N-Atom mit der Carbonylgruppe in (S2) verbunden ist und unsubstituiert oder durch Reste aus der Gruppe (C₁-C₄)Alkyl, (C₁-C₄)Alkoxy oder gegebenenfalls substituiertes Phenyl substituiert ist, vorzugsweise ein Rest der Formel OR_{B}³, NHR_{B}⁴ oder N(CH₃)₂, insbesondere der Formel OR_{B}³;
   - R_{B}³: ist Wasserstoff oder ein unsubstituierter oder substituierter aliphatischer Kohlenwasserstoffrest, vorzugsweise mit insgesamt 1 bis 18 C-Atomen;
   - R_{B}⁴: ist Wasserstoff, (C₁-C₆)Alkyl, (C₁-C₆)Alkoxy oder substituiertes oder unsubstituiertes Phenyl;
   - T_{B}: ist eine (C₁ oder C₂)-Alkandiylkette, die unsubstituiert oder mit einem oder zwei (C₁-C₄)Alkylresten oder mit [(C₁-C₃)-Alkoxy]-carbonyl substituiert ist;
   vorzugsweise:
   a) Verbindungen vom Typ der 8-Chinolinoxyessigsäure (S2^{a}), vorzugsweise (5-Chlor-8-chinolinoxy)essigsäure-(1-methylhexyl)ester ("Cloquintocet-mexyl") (S2-1),
      (5-Chlor-8-chinolinoxy)essigsäure-(1,3-dimethyl-but-1-yl)ester (S2-2),
      (5-Chlor-8-chinolinoxy)essigsäure-4-allyloxy-butylester (S2-3),
      (5-Chlor-8-chinolinoxy)essigsäure-1-allyloxy-prop-2-ylester (S2-4),
      (5-Chlor-8-chinolinoxy)essigsäureethylester (S2-5),
      (5-Chlor-8-chinolinoxy)essigsäuremethylester (S2-6),
      (5-Chlor-8-chinolinoxy)essigsäureallylester (S2-7),
      (5-Chlor-8-chinolinoxy)essigsäure-2-(2-propyliden-iminoxy)-1-ethylester (S2-8), (5-Chlor-8-chinolinoxy)essigsäure-2-oxo-prop-1-ylester (S2-9) und
      verwandte Verbindungen, wie sie in EP-A-86 750, EP-A-94 349 und EP-A-191 736 oder EP-A-0 492 366 beschrieben sind, sowie (5-Chlor-8-chinolinoxy)essigsäure (S2-10), deren Hydrate und Salze, beispielsweise deren Lithium-, Natrium- Kalium-, Kalzium-, Magnesium-, Aluminium-, Eisen-, Ammonium-, quartäre Ammonium-, Sulfonium-, oder Phosphoniumsalze wie sie in der WO-A-2002/34048 beschrieben sind;
   b) Verbindungen vom Typ der (5-Chlor-8-chinolinoxy)malonsäure (S2^{b}), vorzugsweise Verbindungen wie (5-Chlor-8-chinolinoxy)malonsäurediethylester, (5-Chlor-8-chinolinoxy)malonsäurediallylester, (5-Chlor-8-chinolinoxy)malonsäure-methyl-ethylester und verwandte Verbindungen, wie sie in EP-A-0 582 198 beschrieben sind.
S3) Verbindungen der Formel (S3) 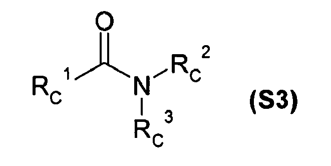 wobei die Symbole und Indizes folgende Bedeutungen haben:
   R_{C}¹ ist (C₁-C₄)Alkyl, (C₁-C₄)Haloalkyl, (C₂-C₄)Alkenyl, (C₂-C₄)Haloalkenyl,
      (C₃-C₇)Cycloalkyl, vorzugsweise Dichlormethyl;
   R_{C}², R_{C}³ sind gleich oder verschieden Wasserstoff, (C₁-C₄)Alkyl, (C₂-C₄)Alkenyl, (C₂-C₄)Alkinyl, (C₁-C₄)Haloalkyl, (C₂-C₄)Haloalkenyl, (C₁-C₄)Alkylcarbamoyl-(C₁-C₄)alkyl, (C₂-C₄)Alkenylcarbamoyl-(C₁-C₄)alkyl, (C₁-C₄)Alkoxy-(C₁-C₄)alkyl, Dioxolanyl-(C₁-C₄)alkyl, Thiazolyl, Furyl, Furylalkyl, Thienyl, Piperidyl, substituiertes oder unsubstituiertes Phenyl, oder R_{C}² und R_{C}³ bilden zusammen einen substituierten oder unsubstituierten heterocyclischen Ring, vorzugsweise einen Oxazolidin-, Thiazolidin-, Piperidin-, Morpholin-, Hexahydropyrimidin- oder Benzoxazinring;
   vorzugsweise:
      Wirkstoffe vom Typ der Dichloracetamide, die häufig als Vorauflaufsafener (bodenwirksame Safener) angewendet werden, wie z. B.
         "Dichlormid" (N,N-Diallyl-2,2-dichloracetamid) (S3-1),
         "R-29148" (3-Dichloracetyl-2,2,5-trimethyl-1,3-oxazolidin) der Firma Stauffer (S3-2), "R-28725" (3-Dichloracetyl-2,2,-dimethyl-1,3-oxazolidin) der Firma Stauffer (S3-3), "Benoxacor" (4-Dichloracetyl-3,4-dihydro-3-methyl-2H-1,4-benzoxazin) (S3-4), "PPG-1292" (N-Allyl-N-[(1,3-dioxolan-2-yl)-methyl]-dichloracetamid) der Firma PPG Industries (S3-5),
         "DKA-24" (N-Allyl-N-[(allylaminocarbonyl)methyl]-dichloracetamid) der Firma Sagro-Chem (S3-6),
         "AD-67" oder "MON 4660" (3-Dichloracetyl-1-oxa-3-aza-spiro[4,5]decan) der Firma Nitrokemia bzw. Monsanto (S3-7),
         "TI-35" (1-Dichloracetyl-azepan) der Firma TRI-Chemical RT (S3-8),
         "Diclonon" (Dicyclonon) oder "BAS145138" oder "LAB145138" (S3-9) ((RS)-1-Dichloracetyl-3,3,8a-trimethylperhydropyrrolo[1,2-a]pyrimidin-6-on) der Firma BASF, "Furilazol" oder "MON 13900" ((RS)-3-Dichloracetyl-5-(2-furyl)-2,2-dimethyloxazolidin) (S3-10); sowie dessen (R)-Isomer (S3-11).
S4) N-Acylsulfonamide der Formel (S4) und ihre Salze, 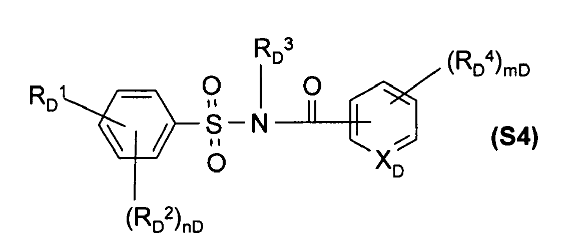 worin die Symbole und Indizes folgende Bedeutungen haben:
   - X_{D}: ist CH oder N;
   - R_{D}¹: ist CO-NR_{D}⁵R_{D}⁶ oder NHCO-R_{D}⁷;
   - R_{D}²: ist Halogen, (C₁-C₄)Haloalkyl, (C₁-C₄)Haloalkoxy, Nitro, (C₁-C₄)Alkyl, (C₁-C₄)Alkoxy, (C₁-C₄)Alkylsulfonyl, (C₁-C₄)Alkoxycarbonyl oder (C₁-C₄)Alkylcarbonyl;
   - R_{D}³: ist Wasserstoff, (C₁-C₄)Alkyl, (C₂-C₄)Alkenyl oder (C₂-C₄)Alkinyl;
   - R_{D}⁴: ist Halogen, Nitro, (C₁-C₄)Alkyl, (C₁-C₄)Haloalkyl, (C₁-C₄)Haloalkoxy, (C₃-C₆)Cycloalkyl, Phenyl, (C₁-C₄)Alkoxy, Cyano, (C₁-C₄)Alkylthio, (C₁-C₄)Alkylsulfinyl, (C₁-C₄)Alkylsulfonyl, (C₁-C₄)Alkoxycarbonyl oder (C₁-C₄)Alkylcarbonyl;
   - R_{D}⁵: ist Wasserstoff, (C₁-C₆)Alkyl, (C₃-C₆)Cycloalkyl, (C₂-C₆)Alkenyl, (C₂-C₆)Alkinyl, (C₅-C₆)Cycloalkenyl, Phenyl oder 3- bis 6-gliedriges Heterocyclyl enthaltend V_{D} Heteroatome aus der Gruppe Stickstoff, Sauerstoff und Schwefel, wobei die sieben letztgenannten Reste durch V_{D} Substituenten aus der Gruppe Halogen, (C₁-C₆)Alkoxy, (C₁-C₆)Haloalkoxy, (C₁-C₂)Alkylsulfinyl, (C₁- C₂)Alkylsulfonyl, (C₃-C₆)Cycloalkyl, (C₁-C₄)Alkoxycarbonyl, (C₁- C₄)Alkylcarbonyl und Phenyl und im Falle cyclischer Reste auch (C₁-C₄) Alkyl und (C₁-C₄)Haloalkyl substituiert sind;
   - R_{D}⁶: ist Wasserstoff, (C₁-C₆)Alkyl, (C₂-C₆)Alkenyl oder (C₂-C₆)Alkinyl, wobei die drei letztgenannten Reste durch V_{D} Reste aus der Gruppe Halogen, Hydroxy, (C₁-C₄)Alkyl, (C₁-C₄)Alkoxy und (C₁-C₄)Alkylthio substituiert sind, oder
   - R_{D}⁵ und R_{D}⁶: gemeinsam mit dem dem sie tragenden Stickstoffatom einen Pyrrolidinyl- oder Piperidinyl-Rest bilden;
   - R_{D}⁷: ist Wasserstoff, (C₁-C₄)Alkylamino, Di-(C₁-C₄)alkylamino, (C₁-C₆)Alkyl, (C₃-C₆)Cycloalkyl, wobei die 2 letztgenannten Reste durch V_{D} Substituenten aus der Gruppe Halogen, (C₁-C₄)Alkoxy, (C₁-C₆)Haloalkoxy und (C₁-C₄)Alkylthio und im Falle cyclischer Reste auch (C₁-C₄)Alkyl und (C₁-C₄)Haloalkyl substituiert sind;
   - n_{D}: ist 0, 1 oder 2;
   - m_{D}: ist 1 oder 2;
   - V_{D}: ist 0, 1, 2 oder 3;
   davon bevorzugt sind Verbindungen von Typ der N-Acylsulfonamide, z.B. der nachfolgenden Formel (S4^{a}), die z. B. bekannt sind aus WO-A-97/45016 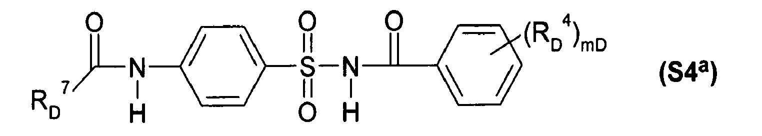 worin
   - R_{D}⁷: (C₁-C₆)Alkyl, (C₃-C₆)Cycloalkyl, wobei die 2 letztgenannten Reste durch V_{D} Substituenten aus der Gruppe Halogen, (C₁-C₄)Alkoxy, (C₁-C₆)Haloalkoxy und (C₁-C₄)Alkylthio und im Falle cyclischer Reste auch (C₁-C₄)Alkyl und (C₁-C₄)Haloalkyl substituiert sind;
   - R_{D}⁴: Halogen, (C₁-C₄)Alkyl, (C₁-C₄)Alkoxy, CF₃;
   - m_{D}: 1 oder 2;
   - V_{D}: ist 0, 1, 2 oder 3 bedeutet;
   sowie
   Acylsulfamoylbenzoesäureamide, z.B. der nachfolgenden Formel (S4^{b}), die z.B. bekannt sind aus WO-A-99/16744, 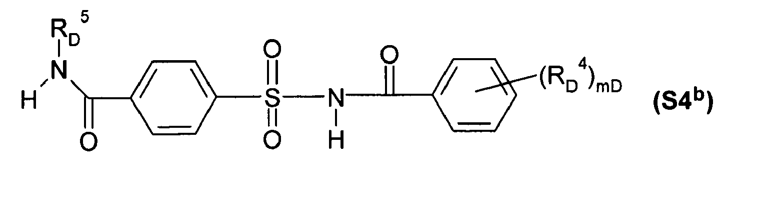 z.B. solche worin
   R_{D}⁵ = Cyclopropyl und (R_{D}⁴) = 2-OMe ist ("Cyprosulfamide", S4-1),
   R_{D}⁵ = Cyclopropyl und (R_{D}⁴) = 5-Cl-2-OMe ist (S4-2),
   R_{D}⁵ = Ethyl und (R_{D}⁴) = 2-OMe ist (S4-3),
   R_{D}⁵ = Isopropyl und (R_{D}⁴) = 5-Cl-2-OMe ist (S4-4) und
   R_{D}⁵ = Isopropyl und (R_{D}⁴) = 2-OMe ist (S4-5).
   sowie
   Verbindungen vom Typ der N-Acylsulfamoylphenylharnstoffe der Formel (S4^{c}), die z.B. bekannt sind aus der EP-A-365484, 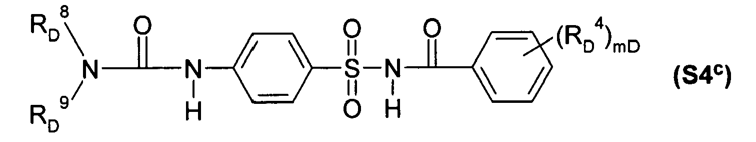 worin
   R_{D}⁸ und R_{D}⁹ unabhängig voneinander Wasserstoff, (C₁-C₈)Alkyl, (C₃-C₈)Cycloalkyl, (C₃-C₆)Alkenyl, (C₃-C₆)Alkinyl,
   R_{D}⁴ Halogen, (C₁-C₄)Alkyl, (C₁-C₄)Alkoxy, CF₃
   m_{D} 1 oder 2 bedeutet;
   beispielsweise
   1-[4-(N-2-Methoxybenzoylsulfamoyl)phenyl]-3-methylharnstoff,
   1-[4-(N-2-Methoxybenzoylsulfamoyl)phenyl]-3,3-dimethylharnstoff,
   1-[4-(N-4,5-Dimethylbenzoylsulfamoyl)phenyl]-3-methylharnstoff.
S5) Wirkstoffe aus der Klasse der Hydroxyaromaten und der aromatischaliphatischen Carbonsäurederivate (S5), z.B.
   3,4,5-Triacetoxybenzoesäureethylester, 3,5-Dimethoxy-4-hydroxybenzoesäure, 3,5-Dihydroxybenzoesäure, 4-Hydroxysalicylsäure, 4-Fluorsalicyclsäure, 2-Hydroxyzimtsäure, 2,4-Dichlorzimtsäure, wie sie in der WO-A-2004/084631, WO-A-2005/015994, WO-A-2005/016001 beschrieben sind.
S6) Wirkstoffe aus der Klasse der 1,2-Dihydrochinoxalin-2-one (S6), z.B.
   1-Methyl-3-(2-thienyl)-1,2-dihydrochinoxalin-2-on, 1-Methyl-3-(2-thienyl)-1,2-dihydrochinoxalin-2-thion, 1-(2-Aminoethyl)-3-(2-thienyl)-1,2-dihydrochinoxalin-2-on-hydrochlorid, 1-(2-Methylsulfonylaminoethyl)-3-(2-thienyl)-1,2-dihydro-chinoxalin-2-on, wie sie in der WO-A-2005/112630 beschrieben sind.
S7) Verbindungen der Formel (S7),wie sie in der WO-A-1998/38856 beschrieben
   sind 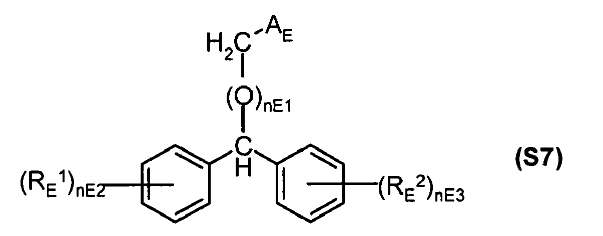 worin die Symbole und Indizes folgende Bedeutungen haben:
   - R_{E}¹, R_{E}²: sind unabhängig voneinander Halogen, (C₁-C₄)Alkyl, (C₁-C₄)Alkoxy, (C₁-C₄)Haloalkyl, (C₁-C₄)Alkylamino, Di-(C₁-C₄)Alkylamino, Nitro;
   - A_{E}: ist COOR_{E}³ oder COSR_{E}⁴
   - R_{E}³, R_{E}⁴: sind unabhängig voneinander Wasserstoff, (C₁-C₄)Alkyl, (C₂- C₆)Alkenyl, (C₂-C₄)Alkinyl, Cyanoalkyl, (C₁-C₄)Haloalkyl, Phenyl, Nitrophenyl, Benzyl, Halobenzyl, Pyridinylalkyl und Alkylammonium,
   - n_{E}¹: ist 0 oder 1
   - n_{E}², n_{E}³: sind unabhängig voneinander 0, 1 oder 2,
   vorzugsweise:
   Diphenylmethoxyessigsäure,
   Diphenylmethoxyessigsäureethylester,
   Diphenylmethoxyessigsäuremethylester (CAS-Reg.Nr. 41858-19-9) (S7-1).
S8) Verbindungen der Formel (S8), wie sie in der WO-A-98/27049 beschrieben sind 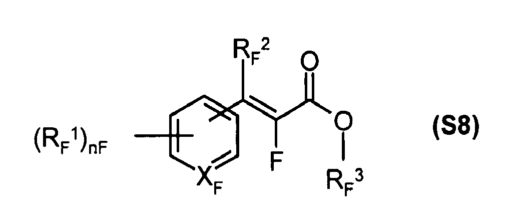 worin
   - X_{F}: CH oder N,
   - n_{F}: für den Fall, dass X_{F}=N ist, eine ganze Zahl von 0 bis 4 und für den Fall, dass X_{F}=CH ist, eine ganze Zahl von 0 bis 5,
   - R_{F}¹: Halogen, (C₁-C₄)Alkyl, (C₁-C₄)Haloalkyl, (C₁-C₄)Alkoxy, (C₁-C₄)Haloalkoxy, Nitro, (C₁-C₄)Alkylthio, (C₁-C₄)-Alkylsulfonyl, (C₁-C₄)Alkoxycarbonyl, ggf. substituiertes. Phenyl, ggf. substituiertes Phenoxy,
   - R_{F}²: Wasserstoff oder (C₁-C₄)Alkyl
   - R_{F}³: Wasserstoff, (C₁-C₈)Alkyl, (C₂-C₄)Alkenyl, (C₂-C₄)Alkinyl, oder Aryl, wobei jeder der vorgenannten C-haltigen Reste unsubstituiert oder durch einen oder mehrere, vorzugsweise bis zu drei gleiche oder verschiedene Reste aus der Gruppe, bestehend aus Halogen und Alkoxy substituiert ist; bedeuten, oder deren Salze,
   vorzugsweise Verbindungen worin
   - X_{F}: CH,
   - n_{F}: eine ganze Zahl von 0 bis 2 ,
   - R_{F}¹: Halogen, (C₁-C₄)Alkyl, (C₁-C₄)Haloalkyl, (C₁-C₄)Alkoxy, (C₁-C₄)Haloalkoxy,
   - R_{F}²: Wasserstoff oder (C₁-C₄)Alkyl,
   - R_{F}³: Wasserstoff, (C₁-C₈)Alkyl, (C₂-C₄)Alkenyl, (C₂-C₄)Alkinyl, oder Aryl, wobei jeder der vorgenannten C-haltigen Reste unsubstituiert oder durch einen oder mehrere, vorzugsweise bis zu drei gleiche oder verschiedene Reste aus der Gruppe, bestehend aus Halogen und Alkoxy substituiert ist, bedeuten, oder deren Salze.
S9) Wirkstoffe aus der Klasse der 3-(5-Tetrazolylcarbonyl)-2-chinolone (S9), z.B.
   1,2-Dihydro-4-hydroxy-1-ethyl-3-(5-tetrazolylcarbonyl)-2-chinolon (CAS-Reg.Nr. 219479-18-2), 1,2-Dihydro-4-hydroxy-1-methyl-3-(5-tetrazolylcarbonyl)-2-chinolon (CAS-Reg.Nr. 95855-00-8), wie sie in der WO-A-1999/000020 beschrieben sind.
S10) Verbindungen der Formeln (S10^{a}) oder (S10^{b})
   wie sie in der WO-A-2007/023719 und WO-A-2007/023764 beschrieben sind 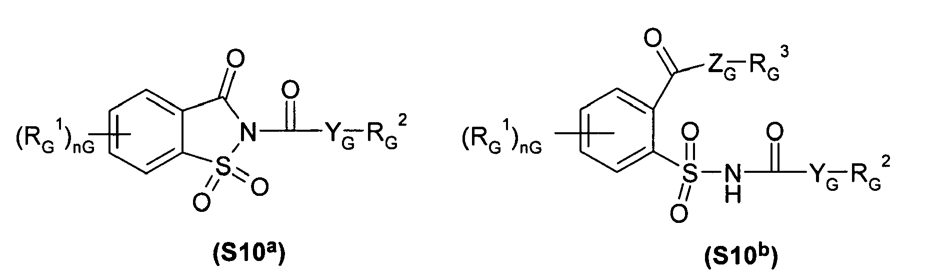 worin
   - R_{G}¹: Halogen, (C₁-C₄)Alkyl, Methoxy, Nitro, Cyano, CF₃, OCF₃
   - Y_{G}, Z_{G}: unabhängig voneinander O oder S,
   - n_{G}: eine ganze Zahl von 0 bis 4,
   - R_{G}²: (C₁-C₁₆)Alkyl, (C₂-C₆)Alkenyl, (C₃-C₆)Cycloalkyl, Aryl; Benzyl, Halogenbenzyl,
   - R_{G}³: Wasserstoff oder (C₁-C₆)Alkyl bedeutet.
S11) Wirkstoffe vom Typ der Oxyimino-Verbindungen (S11), die als Saatbeizmittel
   bekannt sind, wie z. B.
   "Oxabetrinil" ((Z)-1,3-Dioxolan-2-ylmethoxyimino(phenyl)acetonitril) (S11-1), das als Saatbeiz-Safener für Hirse gegen Schäden von Metolachlor bekannt ist,
   "Fluxofenim" (1-(4-Chlorphenyl)-2,2,2-trifluor-1-ethanon-O-(1,3-dioxolan-2-ylmethyl)-oxim) (S11-2), das als Saatbeiz-Safener für Hirse gegen Schäden von Metolachlor bekannt ist, und
   "Cyometrinil" oder "CGA-43089" ((Z)-Cyanomethoxyimino(phenyl)acetonitril)
   (S11-3), das als Saatbeiz-Safener für Hirse gegen Schäden von Metolachlor bekannt ist.
S12) Wirkstoffe aus der Klasse der Isothiochromanone (S12), wie z.B. Methyl-[(3-oxo-1H-2-benzothiopyran-4(3H)-yliden)methoxy]acetate (CAS-Reg.Nr. 205121-04-6) (S12-1) und verwandte Verbindungen aus WO-A-1998/13361.
S13) Eine oder mehrere Verbindungen aus Gruppe (S13):
   "Naphthalic anhydrid" (1,8-Naphthalindicarbonsäureanhydrid) (S13-1), das als Saatbeiz-Safener für Mais gegen Schäden von Thiocarbamatherbiziden bekannt ist,
   "Fenclorim" (4,6-Dichlor-2-phenylpyrimidin) (S13-2), das als Safener für Pretilachlor in gesätem Reis bekannt ist,
   "Flurazole" (Benzyl-2-chlor-4-trifluormethyl-1,3-thiazol-5-carboxylat) (S13-3), das als Saatbeiz-Safener für Hirse gegen Schäden von Alachlor und Metolachlor bekannt ist,
   "CL 304415" (CAS-Reg.Nr. 31541-57-8) (4-Carboxy-3,4-dihydro-2H-1-benzopyran-4-essigsäure) (S13-4) der Firma American Cyanamid, das als Safener für Mais gegen Schäden von Imidazolinonen bekannt ist,
   "MG 191" (CAS-Reg.Nr. 96420-72-3) (2-Dichlormethyl-2-methyl-1,3-dioxolan) (S13-5) der Firma Nitrokemia, das als Safener für Mais bekannt ist, "MG-838" (CAS-Reg.Nr. 133993-74-5)
   (2-propenyl 1-oxa-4-azaspiro[4.5]decane-4-carbodithioate) (S13-6) der Firma Nitrokemia,
   "Disulfoton" (O,O-Diethyl S-2-ethylthioethyl phosphordithioat) (S13-7),
   "Dietholate" (O,O-Diethyl-O-phenylphosphorotioat) (S13-8),
   "Mephenate" (4-Chlorphenyl-methylcarbamat) (S13-9).
S14) Wirkstoffe, die neben einer herbiziden Wirkung gegen Schadpflanzen auch
   Safenerwirkung an Kulturpflanzen wie Reis aufweisen, wie z. B. "Dimepiperate" oder "MY-93" (S-1-Methyl-1-phenylethyl-piperidin-1-carbothioat), das als Safener für Reis gegen Schäden des Herbizids Molinate bekannt ist,
   "Daimuron" oder "SK 23" (1-(1-Methyl-1-phenylethyl)-3-p-tolyl-harnstoff), das als Safener für Reis gegen Schäden des Herbizids Imazosulfuron bekannt ist, "Cumyluron" = "JC-940" (3-(2-Chlorphenylmethyl)-1-(1-methyl-1-phenylethyl)harnstoff, siehe JP-A-60087254), das als Safener für Reis gegen Schäden einiger Herbizide bekannt ist,
   "Methoxyphenon" oder "NK 049" (3,3'-Dimethyl-4-methoxy-benzophenon), das als Safener für Reis gegen Schäden einiger Herbizide bekannt ist, "CSB" (1-Brom-4-(chlormethylsulfonyl)benzol) von Kumiai, (CAS-Reg.Nr. 54091-06-4), das als Safener gegen Schäden einiger Herbizide in Reis bekannt ist.
S15) Wirkstoffe, die vorrangig als Herbizide eingesetzt werden, jedoch auch
   Safenerwirkung auf Kulturpflanzen aufweisen, z.B.
   (2,4-Dichlorphenoxy)essigsäure (2,4-D),
   (4-Chlorphenoxy)essigsäure,
   (R,S)-2-(4-Chlor-o-tolyloxy)propionsäure (Mecoprop),
   4-(2,4-Dichlorphenoxy)buttersäure (2,4-DB),
   (4-Chlor-o-tolyloxy)essigsäure (MCPA),
   4-(4-Chlor-o-tolyloxy)buttersäure,
   4-(4-Chlorphenoxy)buttersäure,
   3,6-Dichlor-2-methoxybenzoesäure (Dicamba),
   1-(Ethoxycarbonyl)ethyl-3,6-dichlor-2-methoxybenzoat (Lactidichlor-ethyl).

Bevorzugt sind Herbizid-Safener-Kombinationen, enthaltend (A) eine herbizid wirksame Menge an einer oder mehrerer Verbindungen der Formel (I) oder deren Salzen, und (B) eine antidotische wirksame Menge an einem oder mehreren Safenern.

Herbizid wirksame Menge bedeutet im Sinne der Erfindung eine Menge an einem oder mehreren Herbiziden, die geeignet ist, den Pflanzenwuchs negativ zu beeinflussen. Antidotisch wirksame Menge bedeutet im Sinne der Erfindung eine Menge an einem oder mehreren Safenern, die geeignet ist, die phytotoxische Wirkung von Pflanzenschutzmittelwirkstoffen (z. B. von Herbiziden) an Kulturpflanzen zu reduzieren.

Einige der Safener sind bereits als Herbizide bekannt und entfalten somit neben der Herbizidwirkung bei Schadpflanzen zugleich auch Schutzwirkung bei den Kulturpflanzen.

Die Gewichtsverhältnisse von Herbizid(mischung) zu Safener hängt im Allgemeinen von der Aufwandmenge an Herbizid und der Wirksamkeit des jeweiligen Safeners ab und kann innerhalb weiter Grenzen variieren, beispielsweise im Bereich von 200:1 bis 1:200, vorzugsweise 100:1 bis 1:100, insbesondere 20:1 bis 1:20. Die Safener können analog den Verbindungen der Formel (I) oder deren Mischungen mit weiteren Herbiziden/Pestiziden formuliert werden und als Fertigformulierung oder Tankmischung mit den Herbiziden bereitgestellt und angewendet werden.

Zur Anwendung werden die in handelsüblicher Form vorliegenden Formulierungen gegebenenfalls in üblicher Weise verdünnt z. B. bei Spritzpulvern, emulgierbaren Konzentraten, Dispersionen und wasserdispergierbaren Granulaten mittels Wasser. Staubförmige Zubereitungen, Boden- bzw. Streugranulate sowie versprühbare Lösungen werden vor der Anwendung üblicherweise nicht mehr mit weiteren inerten Stoffen verdünnt.

Mit den äußeren Bedingungen wie Temperatur, Feuchtigkeit, der Art des verwendeten Herbizids, u.a. variiert die erforderliche Aufwandmenge der Verbindungen der allgemeinen Formel (I). Sie kann innerhalb weiter Grenzen schwanken, z. B. zwischen 0,001 und 10,0 kg/ha oder mehr Aktivsubstanz, vorzugsweise liegt sie jedoch zwischen 0,005 und 5 kg/ha.

Die vorliegende Erfindung wird anhand der nachfolgenden Beispiele näher erläutert, welche die Erfindung jedoch keinesfalls beschränken.

### A. Synthesebeispiele

### A. Synthesebeispiele

1. 4-(5,6-Dihydro-1,4,2-dioxazin-3-yl)-N-[(4,6-dimethoxypyrimidin-2-yl)carbamoyl]pyridin-3-sulfonamid (Ia-2) 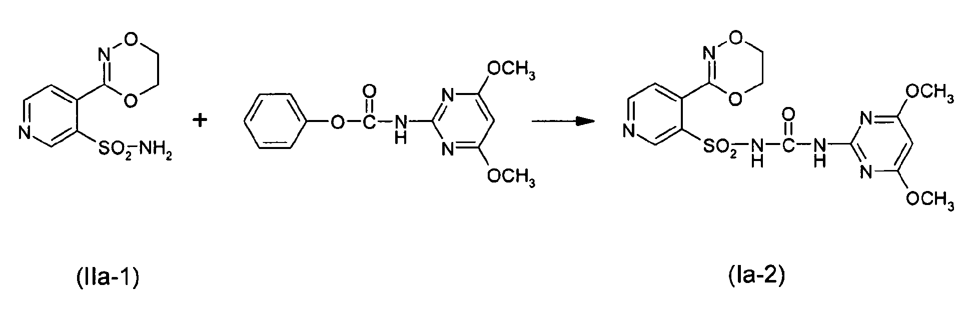 4.9 g (0.02 mol) 4-(5,6-Dihydro-1,4,2-dioxazin-3-yl)pyridin-3-sulfonamid (IIa-1) werden in 40 ml Acetonitril gelöst und mit 3.1 g (0.02 mol) DBU (Diazabicyclo-undecen) versetzt. Die klare Lösung wird mit 5.5 g (0.02 mol) Phenyl-(4,6-dimethoxypyrimidin-2-yl)carbamat versetzt und 30 min bei Raumtemperatur gerührt. Unter Rühren wird die Mischung auf 400 ml wässrige verdünnte Salzsäure gegossen und gründlich durchgerührt. Die ausgefallenen Kristalle werden abgesaugt und mit Wasser bis zur neutralen Reaktion nachgewaschen. Mit Isopropanol wird wasserfrei gewaschen, dann aus Isopropanol umkristallisiert. Man erhält 7.9 g (0.0175 mol) 4-(5,6-Dihydro-1,4,2-dioxazin-3-yl)-N-[(4,6-dimethoxypyrimidin-2-yl)carbamoyl]pyridin-3-sulfonamid vom Reinheitsgehalt (HPLC) 93.9 %.
N-[(4-Chlor-6-methoxypyrimidin-2-yl)carbamoyl]-4-(5,6-dihydro-1,4,2-dioxazin-3-yl)pyridin-3-sulfonamid (Ia-1) 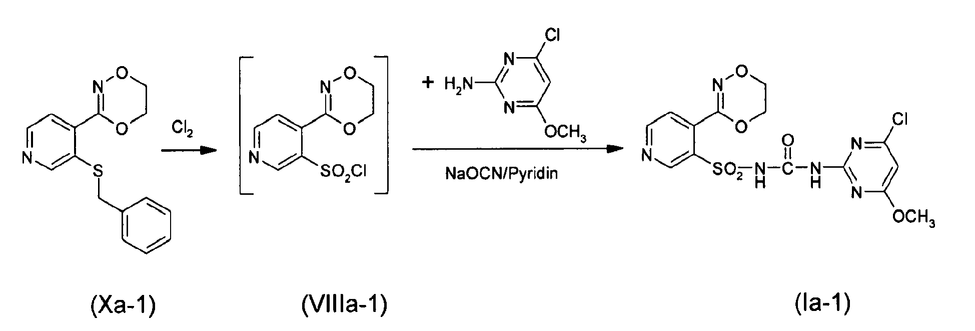 2.0 g (7 mmol) 3-[3-(Benzylsulfanyl)pyridin-4-yl]-5,6-dihydro-1,4,2-dioxazin (Xa-1) werden in 50 ml Dichlormethan gelöst und mit 6.3 g (0.04 mol) Natriumdihydrogenphosphat-monohydrat in 30 ml Wasser gelöst versetzt. Bei 0 °C wird Chlorgas eingeleitet, bis eine deutliche Gelbfärbung zu erkennen ist. Nach 15 min Nachrührzeit wird die organische Phase abgetrennt, zweimal mit Eiswasser gewaschen, getrocknet und eingeengt.
Der Rückstand (rohes Sulfochlorid (VIIIa-1)) wird in 5 ml Acetonitril gelöst und zu einer Lösung aus 560 mg (3.5 mmol) 4-Chlor-6-methoxypyrimidin-2-amin, 560 mg (7 mmol) Pyridin und 460 mg (7 mmol) Natrium-cyanat in 30 ml Acetonitril getropft. Das Reaktionsgemisch wird 16 Stunden bei 20 °C gerührt, dann auf 500 ml Eiswasser gegossen und mit verdünnter Salzsäure schwach sauer gestellt. Es wird gründlich durchgerührt und die ausgefallenen Kristalle werden abgesaugt. Die Kristalle werden erst mit Wasser nachgewaschen, dann mit Isopropanol verrieben, erneut abgesaugt und mit Isopropanol und Diethylether nachgewaschen. Nach dem Trocknen wird über eine Kieselgelsäule chromatographiert (Methylenchlorid/Methanol = 9/1 Volumenverhältnis). Man erhält 66 mg (0.148 mmol) N-[(4-Chlor-6-methoxypyrimidin-2-yl)carbamoyl]-4-(5,6-dihydro-1,4,2-dioxazin-3-yl)pyridin-3-sulfonamid vom Reinheitsgehalt (HPLC) 96.2 %.
4-(5,6-Dihydro-1,4,2-dioxazin-3-yl)-N-[(4,6-dimethoxy-1,3,5-triazin-2-yl)carbamoyl]pyridin-3-sulfonamid (Ia-6) 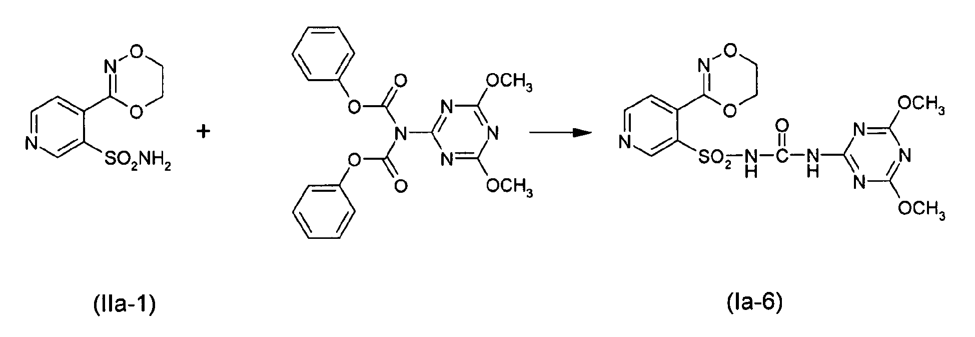 490 mg (2 mmol) 4-(5,6-Dihydro-1,4,2-dioxazin-3-yl)pyridin-3-sulfonamid (IIa-1) werden bei 20 °C in 20 ml Acetonitril gelöst und mit 310 mg (2 mmol) DBU (Diazabicyclo-undecen) versetzt. Man fügt 800 mg (2 mmol) Diphenyl-(4,6-dimethoxy-1,3,5-triazin-2-yl)imidodicarbonat (vgl. WO 1996/022284) zu und rührt 30 min. Dann wird der Ansatz unter Rühren auf ein Gemisch aus
Methylenchlorid/wässrige Salzsäure gegossen und die organische Phase abgetrennt. Sie wird zweimal mit Wasser und mit gesättigter Natriumchlorid-Lösung gewaschen, getrocknet und eingeengt. Der Rückstand wird mit Diethylether zur Kristallisation gebracht, abgesaugt und mit Isopropanol verrührt. Nach dem Absaugen wird mit Diethylether nachgewaschen und getrocknet. Man erhält 530 mg (1.14 mmol) 4-(5,6-Dihydro-1,4,2-dioxazin-3-yl)-N-[(4,6-dimethoxy-1,3,5-triazin-2-yl)carbamoyl]pyridin-3-sulfonamid vom Gehalt (HPLC) 91.3 %.
Die in den nachfolgenden Tabellen beschriebenen Verbindungen der allgemeinen Formel (I) erhält man gemäß oder analog zu den oben beschriebenen Synthesebeispielen:

**Tabelle 1 a: Verbindungen der Formel (Ia)**

| | | | | | | | | | |
|---|---|---|---|---|---|---|---|---|---|
| 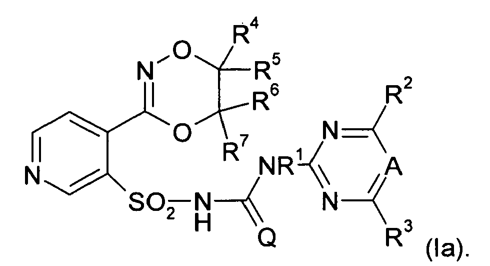 | | | | | | | | | |

| Bsp. Nr. | R¹ | R² | R³ | R⁴ | R⁶ | R⁵ | R⁷ | Q | A |
|---|---|---|---|---|---|---|---|---|---|
| Ia-1 | H | Cl | OCH₃ | H | H | H | H | O | CH |
| Ia-2 | H | OCH₃ | OCH₃ | H | H | H | H | O | CH |
| Ia-3 | H | CH₃ | CH₃ | H | H | H | H | O | CH |
| Ia-4 | H | SCH₃ | OCH₃ | H | H | H | H | O | CH |
| Ia-5 | H | H | OCH₃ | H | H | H | H | O | CH |
| Ia-6 | H | OCH₃ | OCH₃ | H | H | H | H | O | N |
| Ia-7 | H | CH₃ | OCH₃ | H | H | H | H | O | N |
| Ia-8 | H | N(CH₃)₂ | OCH₂CF₃ | H | H | H | H | O | N |
| Ia-9 | H | CH₃ | OCH₃ | H | H | H | H | O | CH |
| Ia-10 | H | OCH₃ | OCH₃ | CH(CH₃)₂ | H | H | H | O | CH |
| Ia-11 | H | OCH₃ | OCH₃ | CH₃ | H | H | H | O | CH |
| Ia-12 | H | CH₃ | OCH₃ | CH₃ | H | CH₃ | H | O | N |
| Ia-13 | H | OCH₃ | OCH₃ | CH₃ | H | CH₃ | H | O | CH |
| Ia-14 | H | Cl | OCH₃ | CH₃ | H | CH₃ | H | O | CH |
| Ia-15 | H | CH₃ | OCH₃ | CH₃ | H | H | H | O | CH |
| Ia-16 | H | Cl | OCH₃ | CH₃ | H | H | H | O | CH |
| Ia-17 | H | CH₃ | OCH₃ | CH₂CH₂CH₃ | H | H | H | O | CH |
| Ia-18 | H | CH₃ | OCH₃ | CH₂CH₃ | H | H | H | O | N |
| Ia-19 | H | CH₃ | OCH₃ | CH₂CH₃ | H | H | H | O | CH |
| Ia-20 | H | Cl | OCH₃ | CH₂CH₃ | H | H | H | O | CH |
| Ia-21 | H | OCH₃ | OCH₃ | CH₂CH₂CH₃ | H | H | H | O | CH |
| Ia-22 | H | CH₃ | OCH₃ | CH(CH₃)₂ | H | H | H | O | CH |
| Ia-23 | H | CH₃ | OCH₃ | CH₂CH₂CH₃ | H | H | H | O | N |
| Ia-24 | H | OCH₃ | OCH₃ | CH₂CH₃ | H | H | H | O | CH |
| Ia-25 | H | OCH₃ | OCH₃ | H | H | H | H | S | CH |
| Ia-26 | H | OCH₃ | OCH₃ | CH₂CH₂CH₂ | | H | H | O | CH |
| Ia-27 | H | OCH₃ | OCH₃ | CH₂CH₂CH₂CH₂ | | H | H | O | CH |
| Ia-28 | H | OCH₃ | OCH₃ | CH₂-O-CH₂ | | H | H | O | CH |
| Ia-29 | H | OCH₃ | OCH₃ | CH₂-S-CH₂ | | H | H | O | CH |
| Ia-30 | H | OCH₃ | OCH₃ | CH₂-O-CH₂CH₂ | | H | H | O | CH |
| Ia-31 | H | OCH₃ | OCH₃ | CH₂-S-CH_{Z}CH₂ | | H | H | O | CH |

| | | | | | | | | | |
|---|---|---|---|---|---|---|---|---|---|
| ¹H-NMR-Daten (400 MHz., Solvens: CD₃CN, interner Standard: Tetramethylsilan δ = 0.00 ppm; s = Singulett, br. s = breites Singulett, d = Dublett, dd = Doppeldublett, m = Multiplett, q = Quartett, t = Triplett) **Ia-1:** δ (CDCl₃) = 4.01 (s, 3H); 4.27 (m, 2H); 4.58 (m, 2H); 6.50 (s, 1H); 7.54 (d, 1H); 8.91 (d, 1H); 9.52 (br. s, 1H); 12.02 (br. s, 1H) ppm **Ia-2:** δ ([D₇]-DMF) = 3.99 (s, 6H); 4.30 (m, 2H); 4.60 (m, 2H); 6.00 (s, 1H); 7.80 (dd, 1H); 9.05 (d, 1H); 9.39 (br. s, 1H); 10.80 (s, 1H); 12.91 (s, 1H) ppm **Ia-3:** δ (CD₃CN) = 2.42 (s, 6H); 4.19 (m, 2H); 4.45 (m, 2H); 6.88 (s, 1H); 7.57 (dd, 1H); 8.09 (br. s, 1H); 8.88 (d, 1H); 9.33 (br. s, 1H); 13.2 (br. s, 1H) ppm **Ia-6:** δ (CD₃CN) = 4.02 (s, 6H); 4.20 (m, 2H); 4.51 (m, 2H); 7.60 (d, 1H); 8.38 (br. s, 1H); 8.90 (d, 1H); 9.32 (br. s, 1H); 12.3 (br. s, 1H) ppm **Ia-7:** δ (CD₃CN) = 2.50 (s, 3H); 4.01 (s, 3H); 4.21 (m, 2H); 4.49 (m, 2H); 7.60 (m, 1H); 8.40 (br. s, 1H); 8.90 (d, 1H); 9.33 (br. s, 1H); 12.58 (br. s, 1H) ppm **Ia-8:** δ (CD₃CN) = 3.17 (s, 6H); 4.18 (m, 2H); 4.47 (m, 2H); 4.86 (q, 2H); 7.57 (d, 1H); 8.87 (d, 1H); 9.31 (br. s, 1H) ppm **Ia-9:** δ (CD₃CN) = 2.41 (s, 3H); 3.94 (s, 3H); 4.19 (m, 2H); 4.46 (m, 2H); 6.39 (br. s, 1H); 7.57 (d, 1H); 8.25 (br. s, 1H); 8.88 (d, 1H); 9.33 (br. s, 1H) ppm | | | | | | | | | |

**Tabelle 1 b: Verbindungen der Formel (Ib)**

| | | | | | | | | | |
|---|---|---|---|---|---|---|---|---|---|
| 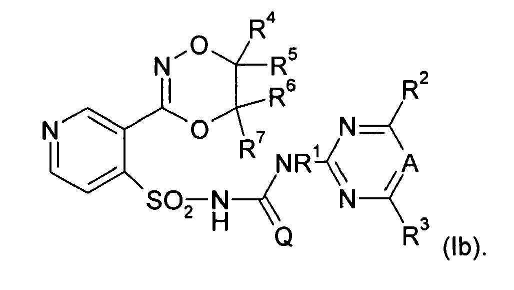 | | | | | | | | | |

| Bsp. Nr. | R¹ | R² | R³ | R⁴ | R⁶ | R⁵ | R⁷ | Q | A |
|---|---|---|---|---|---|---|---|---|---|
| Ib-1 | H | Cl | OCH₃ | H | H | H | H | O | CH |
| Ib-2 | H | OCH₃ | OCH₃ | H | H | H | H | O | CH |
| Ib-3 | H | CH₃ | CH₃ | H | H | H | H | O | CH |
| Ib-4 | H | SCH₃ | OCH₃ | H | H | H | H | O | CH |
| Ib-5 | H | H | OCH₃ | H | H | H | H | O | CH |
| Ib-6 | H | OCH₃ | OCH₃ | H | H | H | H | O | N |
| Ib-7 | H | CH₃ | OCH₃ | H | H | H | H | O | N |
| Ib-8 | H | N(CH₃)₂ | OCH₂CF₃ | H | H | H | H | O | N |
| Ib-9 | H | CH₃ | OCH₃ | H | H | H | H | O | CH |
| Ib-10 | H | OCH₃ | OCH₃ | CH(CH₃)₂ | H | H | H | O | CH |
| Ib-11 | H | OCH₃ | OCH₃ | CH₃ | H | H | H | O | CH |
| Ib-12 | H | CH₃ | OCH₃ | CH₃ | H | CH₃ | H | O | N |
| Ib-13 | H | OCH₃ | OCH₃ | CH₃ | H | CH₃ | H | O | CH |
| Ib-14 | H | Cl | OCH₃ | CH₃ | H | CH₃ | H | O | CH |
| Ib-15 | H | CH₃ | OCH₃ | CH₃ | H | H | H | O | CH |
| Ib-16 | H | Cl | OCH₃ | CH₃ | H | H | H | O | CH |
| Ib-17 | H | CH₃ | OCH₃ | CH₂CH₂CH₃ | H | H | H | O | CH |
| Ib-18 | H | CH₃ | OCH₃ | CH₂CH₃ | H | H | H | O | N |
| Ib-19 | H | CH₃ | OCH₃ | CH₂CH₃ | H | H | H | O | CH |
| Ib-20 | H | Cl | OCH₃ | CH₂CH₃ | H | H | H | O | CH |
| Ib-21 | H | OCH₃ | OCH₃ | CH₂CH₂CH₃ | H | H | H | O | CH |
| Ib-22 | H | CH₃ | OCH₃ | CH(CH₃)₂ | H | H | H | O | CH |
| Ib-23 | H | CH₃ | OCH₃ | CH₂CH₂CH₃ | H | H | H | O | N |
| Ib-24 | H | OCH₃ | OCH₃ | CH₂CH₃ | H | H | H | O | CH |
| Ib-25 | H | OCH₃ | OCH₃ | H | H | H | H | S | CH |
| Ib-26 | H | OCH₃ | OCH₃ | CH₂CH₂CH₂ | | H | H | O | CH |
| Ib-27 | H | OCH₃ | OCH₃ | CH₂CH₂CH₂CH₂ | | H | H | O | CH |
| Ib-28 | H | OCH₃ | OCH₃ | CH₂-O-CH₂ | | H | H | O | CH |
| Ib-29 | H | OCH₃ | OCH₃ | CH₂-S-CH₂ | | H | H | O | CH |
| Ib-30 | H | OCH₃ | OCH₃ | CH₂-O-CH₂CH₂ | | H | H | O | CH |
| Ib-31 | H | OCH₃ | OCH₃ | CH₂-S-CH₂CH₂ | | H | H | O | CH |

**Tabelle 1c: Verbindungen der Formel (Ic)**

| | | | | | | | | | |
|---|---|---|---|---|---|---|---|---|---|
| 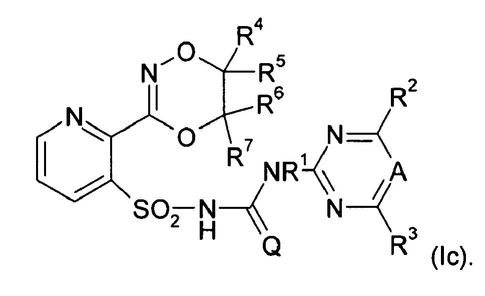 | | | | | | | | | |

| Bsp. Nr. | R¹ | R² | R³ | R⁴ | R⁶ | R⁵ | R⁷ | Q | A |
|---|---|---|---|---|---|---|---|---|---|
| Ic-1 | H | OCH₃ | OCH₃ | H | H | H | H | O | CH |
| Ic-2 | H | Cl | OCH₃ | H | H | H | H | O | CH |
| Ic-3 | H | CH₃ | CH₃ | H | H | H | H | O | CH |
| Ic-4 | H | SCH₃ | OCH₃ | H | H | H | H | O | CH |
| Ic-5 | H | H | OCH₃ | H | H | H | H | O | CH |
| Ic-6 | H | OCH₃ | OCH₃ | H | H | H | H | O | N |
| Ic-7 | H | CH₃ | OCH₃ | H | H | H | H | O | N |
| Ic-8 | H | N(CH₃)₂ | OCH₂CF₃ | H | H | H | H | O | N |
| Ic-9 | H | CH₃ | OCH₃ | H | H | H | H | O | CH |
| Ic-10 | H | OCH₃ | OCH₃ | CH(CH₃)₂ | H | H | H | O | CH |
| Ic-11 | H | OCH₃ | OCH₃ | CH₃ | H | H | H | O | CH |
| Ic-12 | H | CH₃ | OCH₃ | CH₃ | H | CH₃ | H | O | N |
| Ic-13 | H | OCH₃ | OCH₃ | CH₃ | H | CH₃ | H | O | CH |
| Ic-14 | H | Cl | OCH₃ | CH₃ | H | CH₃ | H | O | CH |
| Ic-15 | H | CH₃ | OCH₃ | CH₃ | H | H | H | O | CH |
| Ic-16 | H | Cl | OCH₃ | CH₃ | H | H | H | O | CH |
| Ic-17 | H | CH₃ | OCH₃ | CH₂CH₂CH₃ | H | H | H | O | CH |
| Ic-18 | H | CH₃ | OCH₃ | CH₂CH₃ | H | H | H | O | N |
| Ic-19 | H | CH₃ | OCH₃ | CH₂CH₃ | H | H | H | O | CH |
| Ic-20 | H | CI | OCH₃ | CH₂CH₃ | H | H | H | O | CH |
| Ic-21 | H | OCH₃ | OCH₃ | CH₂CH₂CH₃ | H | H | H | O | CH |
| Ic-22 | H | CH₃ | OCH₃ | CH(CH₃)₂ | H | H | H | O | CH |
| Ic-23 | H | CH₃ | OCH₃ | CH₂CH₂CH₃ | H | H | H | O | N |
| Ic-24 | H | OCH₃ | OCH₃ | CH₂CH₃ | H | H | H | O | CH |
| Ic-25 | H | OCH₃ | OCH₃ | H | H | H | H | S | CH |
| Ic-26 | H | OCH₃ | OCH₃ | CH₂CH₂CH₂ | | H | H | O | CH |
| Ic-27 | H | OCH₃ | OCH₃ | CH₂CH₂CH₂CH₂ | | H | H | O | CH |
| Ic-28 | H | OCH₃ | OCH₃ | CH₂-O-C | | H | H | O | CH |
| Ic-29 | H | OCH₃ | OCH₃ | CH₂-S-CH₂ | | H | H | O | CH |
| Ic-30 | H | OCH₃ | OCH₃ | CH₂-O-CH₂CH₂ | | H | H | O | CH |
| Ic-31 | H | OCH₃ | OCH₃ | CH₂-S-CH₂CH₂ | | H | H | O | CH |

| | | | | | | | | | |
|---|---|---|---|---|---|---|---|---|---|
| ¹H-NMR-Daten (400 MHz., Solvens: CD₃CN, interner Standard: Tetramethylsilan δ = 0.00 ppm; s = Singulett, br. s = breites Singulett, d = Dublett, dd = Doppeldublett, m = Multiplett, q = Quartett, t = Triplett) **Ic-1:** δ = 3.95 (s, 6H); 4.19 (m, 2H); 4.48 (m, 2H); 5.86 (s, 1H); 7.68 (dd, 1H); 8.29 (br. s, 1H); 8.59 (dd, 1H); 8.85 (dd, 1H); 12.80 (s, 1H) ppm **Ic-2:** δ = 4.02 (s, 3H); 4.21 (m, 2H); 4.50 (m, 2H); 6.62 (s, 1H); 7.69 (dd, 1H); 8.51 (br. s, 1H); 8.58 (dd, 1H); 8.86 (dd, 1H); 12.25 (s, 1H) ppm | | | | | | | | | |

2. 4-(5,6-Dihydro-1,4,2-dioxazin-3-yl)pyridin-3-sulfonamid (IIa-1) 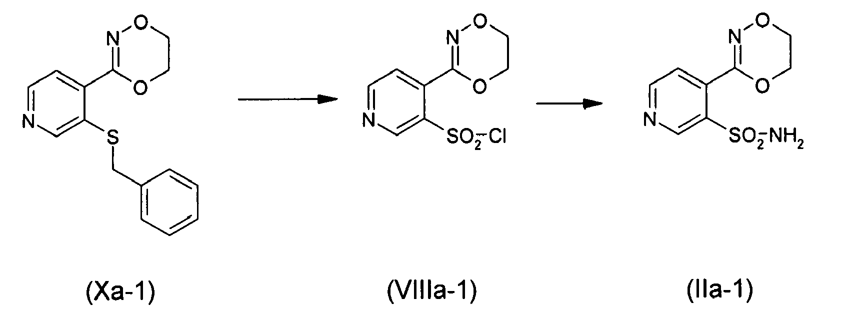 10.6 g (37.1 mmol) 3-[3-(Benzylsulfanyl)pyridin-4-yl]-5,6-dihydro-1,4,2-dioxazin (Xa-1) werden in 85 ml Dichlormethan gelöst und mit einer Lösung aus 33.2 g (0.24 mol) Natriumdihydrogenphosphat-monohydrat in 70 ml Wasser versetzt. Bei <10°C wird Chlorgas zügig eingeleitet bis eine leichte Gelbfärbung zu erkennen ist. Nach 5 min Nachrührzeit wird die organische Phase abgetrennt und zweimal mit je 50 ml Eiswasser gewaschen. Die kalte Lösung aus 4-(5,6-Dihydro-1,4,2-dioxazin-3-yl)pyridin-3-sulfonylchlorid (VIIIa-1) wird bei <10°C unter gutem Rühren zu 10 ml 25 %iger Ammoniak-Lösung getropft. Man lässt auf Raumtemperatur kommen und rührt noch 1 h kräftig durch. Dann kühlt man auf 0 °C ab und saugt den entstandenen Niederschlag ab, der mit Eiswasser und 10 ml kaltem Methyl-tert. butylether nachgewaschen wird. Man verrührt mit Diethylether und saugt ab. Man erhält 7.1 g (28.7 mmol) 4-(5,6-Dihydro-1,4,2-dioxazin-3-yl)pyridin-3-sulfonamid (IIa-1) vom Gehalt (HPLC) 98.4 %, Schmp.: 163-164 °C.
Analog können hergestellt werden:

**Tabelle 2a: Verbindungen der Formel (VIIIa)**

| | | | | | |
|---|---|---|---|---|---|
| 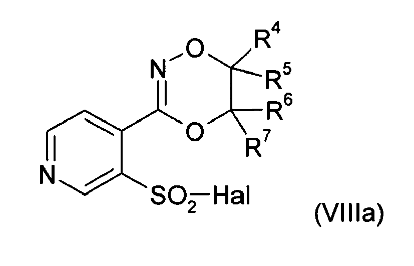 | | | | | |

| Bsp. Nr. | R⁴ | R⁶ | R⁵ | R⁷ | Hal |
|---|---|---|---|---|---|
| VIIIa-1 | H | H | H | H | Cl |
| VIIIa-2 | CH(CH₃)₂ | H | H | H | Cl |
| VIIIa-3 | CH₃ | H | H | H | Cl |
| VIIIa-4 | CH₃ | H | CH₃ | H | Cl |
| VIIIa-5 | CH₂CH₂CH₃ | H | H | H | Cl |
| VIIIa-6 | CH₂CH₃ | H | H | H | Cl |
| VIIIa-7 | CH₃ | CH₃ | H | H | Cl |
| VIIIa-8 | CH₂CH₂CH₂ | | H | H | Cl |
| VIIIa-9 | CH₂CH₂CH₂CH₂ | | H | H | Cl |
| VIIIa-10 | CH₂-O-CH₂ | | H | H | Cl |
| VIIIa-11 | CH₂-S-CH₂ | | H | H | Cl |
| VIIIa-12 | CH₂-O-CH₂CH₂ | | H | H | Cl |
| VIIIa-13 | CH₂-S-CH₂CH₂ | | H | H | Cl |

**Tabelle 2b: Verbindungen der Formel (VIIIb)**

| | | | | | |
|---|---|---|---|---|---|
| 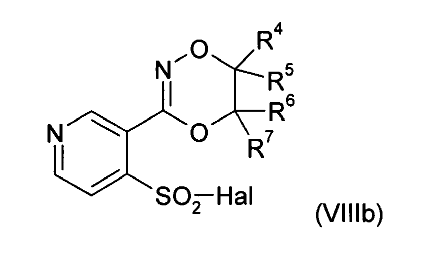 | | | | | |

| Bsp. Nr. | R⁴ | R⁶ | R⁵ | R⁷ | Hal |
|---|---|---|---|---|---|
| VIIIb-1 | H | H | H | H | Cl |
| VIIIb-2 | CH(CH₃)₂ | H | H | H | Cl |
| VIIIb-3 | CH₃ | H | H | H | Cl |
| VIIIb-4 | CH₃ | H | CH₃ | H | Cl |
| VIIIb-5 | CH₂CH₂CH₃ | H | H | H | Cl |
| VIIIb-6 | CH₂CH₃ | H | H | H | Cl |
| VIIIb-7 | CH₃ | CH₃ | H | H | Cl |
| VIIIb-8 | CH₂CH₂CH₂ | | H | H | Cl |
| VIIIb-9 | CH₂CH₂CH₂CH₂ | | H | H | Cl |
| VIIIb-10 | CH₂-O-CH₂ | | H | H | Cl |
| VIIIb-11 | CH₂-S-CH₂ | | H | H | Cl |
| VIIIb-12 | CH₂-O-CH₂CH₂ | | H | H | Cl |
| VIIIb-13 | CH₂-S-CH₂CH₂ | | H | H | Cl |

**Tabelle 2c: Verbindungen der Formel (VIIIc)**

| | | | | | |
|---|---|---|---|---|---|
| 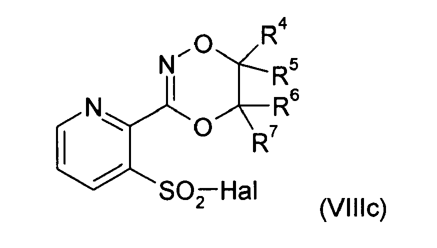 | | | | | |

| Bsp. Nr. | R⁴ | R⁶ | R⁵ | R⁷ | Hal |
|---|---|---|---|---|---|
| VIIIc-1 | H | H | H | H | Cl |
| VIIIc-2 | CH(CH₃)₂ | H | H | H | Cl |
| VIIIc-3 | CH₃ | H | H | H | Cl |
| VIIIc-4 | CH₃ | H | CH₃ | H | Cl |
| VIIIc-5 | CH₂CH₂CH₃ | H | H | H | Cl |
| VIIIc-6 | CH₂CH₃ | H | H | H | Cl |
| VIIIc-7 | CH₃ | CH₃ | H | H | Cl |
| VIIIc-8 | CH₂CH₂CH₂ | | H | H | Cl |
| VIIIc-9 | CH₂CH₂CH₂CH₂ | | H | H | Cl |
| VIIIc-10 | CH₂-O-CH₂ | | H | H | Cl |
| VIIIc-11 | CH₂-S-CH₂ | | H | H | Cl |
| VIIIc-12 | CH₂-O-CH₂CH₂ | | H | H | Cl |
| VIIIc-13 | CH₂-S-CH₂CH₂ | | H | H | Cl |

**Tabelle 3a: Verbindungen der Formel (IIa)**

| | | | | |
|---|---|---|---|---|
| 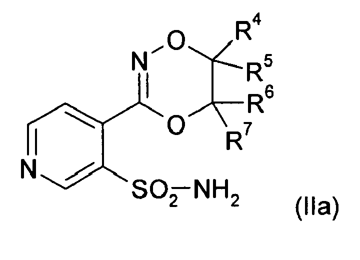 | | | | |

| Bsp. Nr. | R⁴ | R⁶ | R⁵ | R⁷ |
|---|---|---|---|---|
| IIa-1 | H | H | H | H |
| IIa-2 | CH(CH₃)₂ | H | H | H |
| IIa-3 | CH₃ | H | H | H |
| IIa-4 | CH₃ | H | CH₃ | H |
| IIa-5 | CH₂CH₂CH₃ | H | H | H |
| IIa-6 | CH₂CH₃ | H | H | H |
| IIa-7 | CH₃ | CH₃ | H | H |
| IIa-8 | CH₂CH₂CH₂ | | H | H |
| IIa-9 | CH₂CH₂CH₂CH₂ | | H | H |
| IIa-10 | CH₂-O-CH₂ | | H | H |
| IIa-11 | CH₂-S-CH₂ | | H | H |
| IIa-12 | CH₂-O-CH₂CH₂ | | H | H |
| IIa-13 | CH₂-S-CH₂CH₂ | | H | H |

Schmelzpunkt:
- IIa-1:: 163-164 °C

**Tabelle 3b: Verbindungen der Formel (IIb)**

| | | | | |
|---|---|---|---|---|
| 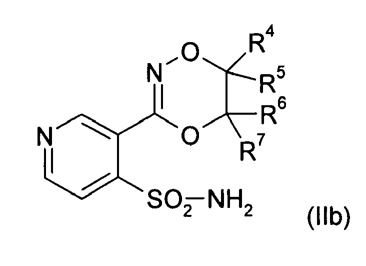 | | | | |

| Bsp. Nr. | R⁴ | R⁶ | R⁵ | R⁷ |
|---|---|---|---|---|
| IIb-1 | H | H | H | H |
| IIb-2 | CH(CH₃)₂ | H | H | H |
| IIb-3 | CH₃ | H | H | H |
| IIb-4 | CH₃ | H | CH₃ | H |
| IIb-5 | CH₂CH₂CH₃ | H | H | H |
| IIb-6 | CH₂CH₃ | H | H | H |
| IIb-7 | CH₃ | CH₃ | H | H |
| IIb-8 | CH₂CH₂CH₂ | | H | H |
| IIb-9 | CH₂CH₂CH₂CH₂ | | H | H |
| IIb-10 | CH₂-O-CH₂ | | H | H |
| IIb-11 | CH₂-S-CH₂ | | H | H |
| IIb-12 | CH₂-O-CH₂CH₂ | | H | H |
| IIb-13 | CH₂-S-CH₂CH₂ | | H | H |

**Tabelle 3c: Verbindungen der Formel (IIc)**

| | | | | |
|---|---|---|---|---|
| 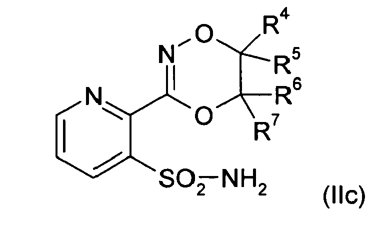 | | | | |

| Bsp. Nr. | R⁴ | R⁶ | R⁵ | R⁷ |
|---|---|---|---|---|
| IIc-1 | H | H | H | H |
| IIc-2 | CH(CH₃)₂ | H | H | H |
| IIc-3 | CH₃ | H | H | H |
| IIc-4 | CH₃ | H | CH₃ | H |
| IIc-5 | CH₂CH₂CH₃ | H | H | H |
| IIc-6 | CH₂CH₃ | H | H | H |
| IIc-7 | CH₃ | CH₃ | H | H |
| IIc-8 | CH₂CH₂CH₂ | | H | H |
| IIc-9 | CH₂CH₂CH₂CH₂ | | H | H |
| IIc-10 | CH₂-O-CH₂ | | H | H |
| IIc-11 | CH₂-S-CH₂ | | H | H |
| IIc-12 | CH₂-O-CH₂CH₂ | | H | H |
| IIc-13 | CH₂-S-CH₂CH₂ | | H | H |

3. 3-[3-(Benzylsulfanyl)pyridin-4-yl]-5,6-dihydro-1,4,2-dioxazin (Xa-1) 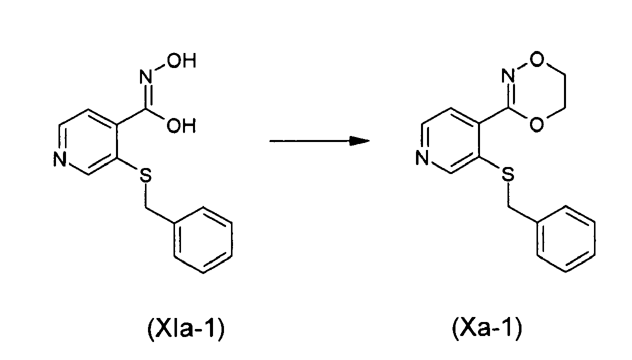 13.0 g (50 mmol) 3-(Benzylsulfanyl)-N-hydroxypyridin-4-carboximidsäure (XIa-1) werden in 60 ml Wasser und 60 ml Ethanol aufgenommen und mit 39 g (0.28 mol) Kaliumcarbonat-Pulver versetzt. Unter Rühren werden 13.5 g (61.5 mmol) Ethan-1,2-diyldimethansulfonat zugefügt. Dann wird der Ansatz 16 h bei 40°C gerührt, wobei sich 2 Phasen bilden. Beim Abkühlen fallen Kristalle aus. Der Reaktionsansatz wird mit 100 ml Wasser verdünnt, auf 5 °C abgekühlt und filtriert. Die abgesaugten Kristalle werden erst mit Wasser und dann mit Isopropanol und Diethylether nachgewaschen. Man erhält 11.0 g (38.4 mmol) 3-[3-(Benzylsulfanyl)pyridin-4-yl]-5,6-dihydro-1,4,2-dioxazin vom Gehalt (HPLC) 100 %, Schmp.: 110-112 °C.
Analog können hergestellt werden:

**Tabelle 4a: Verbindungen der Formel (Xa)**

| | | | | |
|---|---|---|---|---|
| 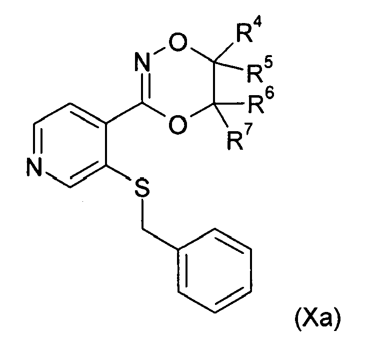 | | | | |

| Bsp. Nr. | R⁴ | R⁶ | R⁵ | R⁷ |
|---|---|---|---|---|
| Xa-1 | H | H | H | H |
| Xa-2 | CH₃ | H | H | H |
| Xa-3 | CH(CH₃)₂ | H | H | H |
| Xa-4 | CH₃ | H | CH₃ | H |
| Xa-5 | CH₂CH₂CH₃ | H | H | H |
| Xa-6 | CH₂CH₃ | H | H | H |
| Xa-7 | CH₃ | CH₃ | H | H |
| Xa-8 | CH₂CH₂CH₂ | | H | H |
| Xa-9 | CH₂CH₂CH₂CH₂ | | H | H |
| Xa-10 | CH₂-O-CH₂ | | H | H |
| Xa-11 | CH₂-S-CH₂ | | H | H |
| Xa-12 | CH₂-O-CH₂CH₂ | | H | H |
| Xa-13 | CH₂-S-CH₂CH₂ | | H | H |

Schmelzpunkt:
- Xa-1:: 110 - 112 °C

**Tabelle 4b: Verbindungen der Formel (Xb)**

| | | | | |
|---|---|---|---|---|
| 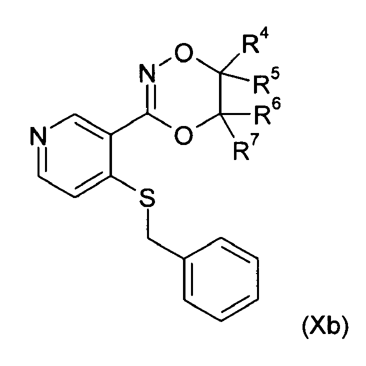 | | | | |

| Bsp. Nr. | R⁴ | R⁶ | R⁵ | R⁷ |
|---|---|---|---|---|
| Xb-1 | H | H | H | H |
| Xb-2 | CH₃ | H | H | H |
| Xb-3 | CH(CH₃)₂ | H | H | H |
| Xb-4 | CH₃ | H | CH₃ | H |
| Xb-5 | CH₂CH₂CH₃ | H | H | H |
| Xb-6 | CH₂CH₃ | H | H | H |
| Xb-7 | CH₃ | CH₃ | H | H |
| Xb-8 | CH₂CH₂CH₂ | | H | H |
| Xb-9 | CH₂CH₂CH₂CH₂ | | H | H |
| Xb-10 | CH₂-O-CH2 | | H | H |
| Xb-11 | CH₂-S-CH₂ | | H | H |
| Xb-12 | CH₂-O-CH_{Z}CH₂ | | H | H |
| Xb-13 | CH₂-S-CH₂CH₂ | | H | H |

Schmelzpunkt:
- Xb-1:: 150 - 151 °C

**Tabelle 4c: Verbindungen der Formel (Xc)**

| | | | | |
|---|---|---|---|---|
| 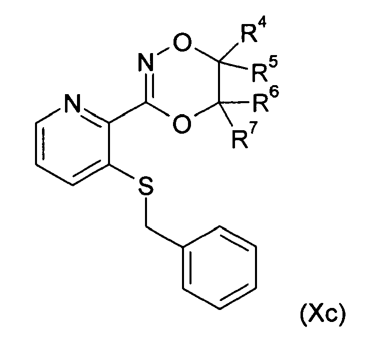 | | | | |

| Bsp. Nr. | 5R⁴ | R⁶ | R⁵ | R⁷ |
|---|---|---|---|---|
| Xc-1 | H | H | H | H |
| Xc-2 | CH₃ | H | H | H |
| Xc-3 | CH(CH₃)₂ | H | H | H |
| Xc-4 | CH₃ | H | CH3 | H |
| Xc-5 | CH₂CH₂CH₃ | H | H | H |
| Xc-6 | CH₂CH₃ | H | H | H |
| Xc-7 | CH₃ | CH₃ | H | H |
| Xc-8 | CH₂CH₂CH₂ | | H | H |
| Xc-9 | CH₂CH₂CH₂CH₂ | | H | H |
| Xc-10 | CH₂-O-CH₂ | | H | H |
| Xc-11 | CH₂-S-CH₂ | | H | H |
| Xc-12 | CH₂-O-CH₂CH₂ | | H | H |
| Xc-13 | CH₂-S-CH₂CH₂ | | H | H |

| | | | | |
|---|---|---|---|---|
| ¹H-NMR-Daten (400 MHz., Solvens: CD₃CN, interner Standard: Tetramethylsilan δ = 0.00 ppm; s = Singulett, d = Dublett, m = Multiplett) Xc-1: ö = 4.17 (m, 2H); 4.18 (s, 2H); 4.47 (m, 2H); 7.18 - 7.39 (m, 6H); 7.80 (d, 1H); 8.38 (m, 1H) ppm | | | | |

4. 3-(Benzylsulfanyl)-N-hydroxypyridin-4-carboximidsäure (XIa-1) 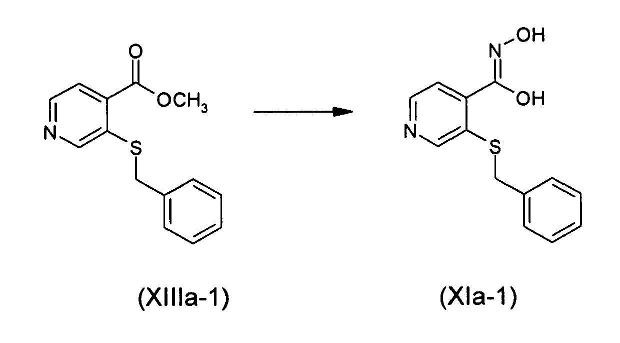 23.4 g (90 mmol) Methyl-3-(benzylsulfanyl)isonicotinat (XIIIa-1) werden zusammen mit 12.5 g (180 mmol) Hydroxylamin-Hydrochlorid in 200 ml Methanol aufgenommen. Unter Rühren werden bei <5 °C 76.2 g einer 30 %igen Natriummethylat-Lösung zügig zugetropft. Man lässt auf Raumtemperatur kommen und rührt 16 h nach. Der Reaktionsansatz wird auf 600 ml Eiswasser gegossen und mit konz. Salzsäure schwach sauer gestellt wobei dabei kräftig durchgerührt wird. Die gebildeten Kristalle werden abgesaugt und erst mit Wasser und dann mit kaltem Isopropanol nachgewaschen. Nach Verrühren mit Diisopropylether wird abgesaugt und getrocknet. Man erhält 17.8 g (64.3 mmol) 3-(Benzylsulfanyl)-N-hydroxypyridin-4-carboximidsäure vom Gehalt (HPLC) 94.1 %), Schmp.: 148 °C
Analog können hergestellt werden: 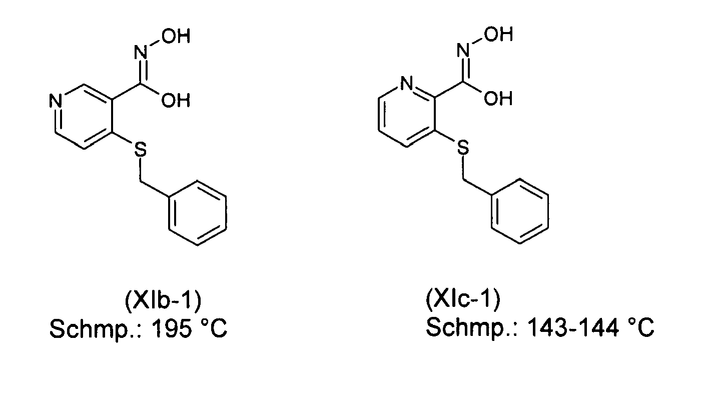 5. Methyl-3-(benzylsulfanyl)isonicotinat (XIIIa-1) 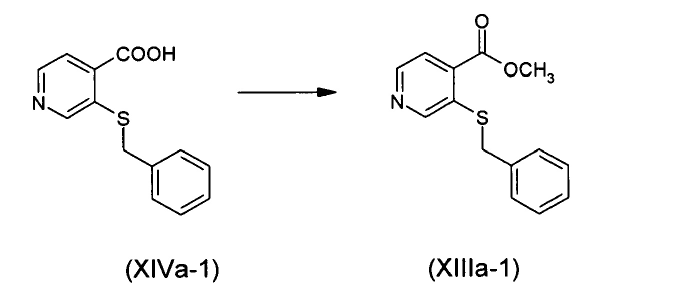 27.7 g (0.11 mol) 3-(Benzylsulfanyl)isonicotinsäure (XIVa-1) werden in 150 ml Thionylchlorid solange unter Rückfluss erhitzt bis die Gasentwicklung beendet und eine klare Lösung entstanden ist. Es werden 100 ml Methanol zugetropft wobei eine heftige HCl-Entwicklung einsetzt und der Ansatz sich stark erwärmt. Nach 15 min Rückfluss wird eingeengt, der Rückstand in Methylenchlorid aufgenommen und mit gesättigter Natrium-hydrogencarbonat-Lösung gewaschen. Nach dem Trocknen und Einengen wird der Rückstand in Diisopropylether verrührt und abgesaugt. Man erhält 22.6 g (86.5 mmol) Methyl-3-(benzylsulfanyl)isonicotinat vom Gehalt (HPLC) 99.2 %, Schmp.: 80 °C.
Analog können hergestellt werden: 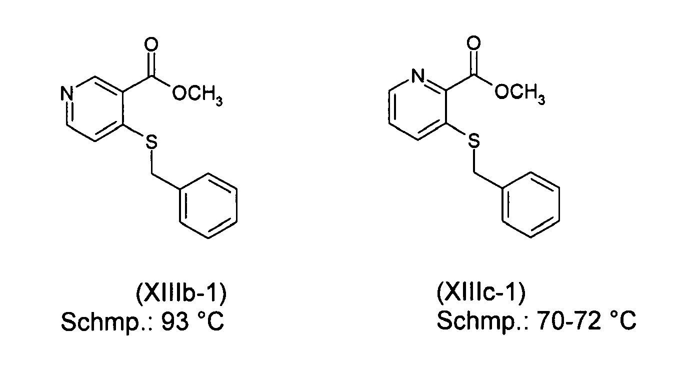 6. (Benzylsulfanyl)pyridincarbonsäuren (XIV-1) 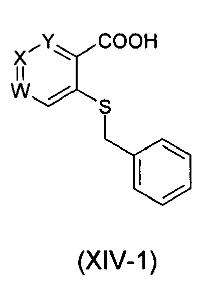 Die Synthese erfolgt nach Vorschriften oder modifizierten Vorschriften gemäß US 4,767,766.

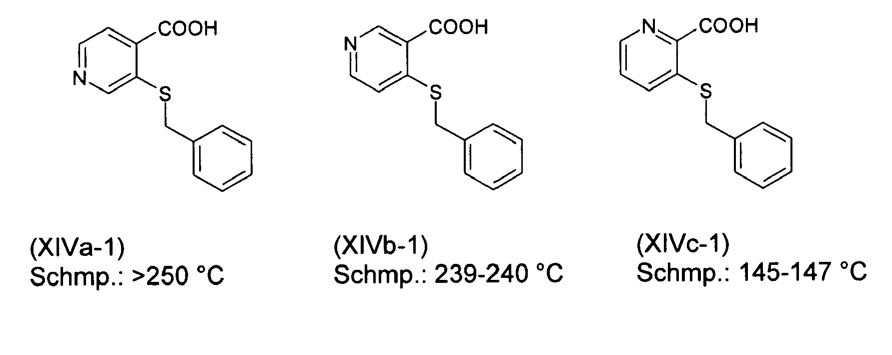

### B. Formulierungsbeispiele

a) Ein Stäubemittel wird erhalten, indem man 10 Gew.-Teile einer Verbindung der Formel (I) und 90 Gew.-Teile Talkum als Inertstoff mischt und in einer Schlagmühle zerkleinert.
b) Ein in Wasser leicht dispergierbares, benetzbares Pulver wird erhalten, indem man 25 Gewichtsteile einer Verbindung der Formel (I), 64 Gewichtsteile kaolinhaltigen Quarz als Inertstoff, 10 Gewichtsteile ligninsulfonsaures Kalium und 1 Gew.-Teil oleoylmethyltaurinsaures Natrium als Netz- und Dispergiermittel mischt und in einer Stiftmühle mahlt.
c) Ein in Wasser leicht dispergierbares Dispersionskonzentrat wird erhalten, indem man 20 Gewichtsteile einer Verbindung der Formel (I) mit 6 Gew.-Teilen Alkylphenolpolyglykolether (®Triton X 207), 3 Gew.-Teilen Isotridecanolpolyglykolether (8 EO) und 71 Gew.-Teilen paraffinischem Mineralöl (Siedebereich z. B. ca. 255 bis über 277 °C) mischt und in einer Reibkugelmühle auf eine Feinheit von unter 5 Mikron vermahlt.
d) Ein emulgierbares Konzentrat wird erhalten aus 15 Gew.-Teilen einer Verbindung der Formel (I), 75 Gew.-Teilen Cyclohexanon als Lösemittel und 10 Gew.-Teilen oxethyliertes Nonylphenol als Emulgator.
e) Ein in Wasser dispergierbares Granulat wird erhalten indem man

| | | |
|---|---|---|
| 75 | Gewichtsteile | einer Verbindung der Formel (I), |
| 10 | " | ligninsulfonsaures Calcium, |
| 5 | " | Natriumlaurylsulfat, |
| 3 | " | Polyvinylalkohol und |
| 7 | " | Kaolin |

mischt, auf einer Stiftmühle mahlt und das Pulver in einem Wirbelbett durch Aufsprühen von Wasser als Granulierflüssigkeit granuliert.
f) Ein in Wasser dispergierbares Granulat wird auch erhalten, indem man

| | | |
|---|---|---|
| 25 | Gewichtsteile | einer Verbindung der Formel (I), |
| 5 | " | 2,2'-dinaphthylmethan-6,6'-disulfonsaures Natrium, |
| 2 | " | oleoylmethyltaurinsaures Natrium, |
| 1 | Gewichtsteil | Polyvinylalkohol, |
| 17 | Gewichtsteile | Calciumcarbonat und |
| 50 | " | Wasser |

auf einer Kolloidmühle homogenisiert und vorzerkleinert, anschließend auf einer Perlmühle mahlt und die so erhaltene Suspension in einem Sprühturm mittels einer Einstoffdüse zerstäubt und trocknet.

### C. Biologische Beispiele

### 1. Herbizide Wirkung bzw. Kulturpflanzenverträglichkeit im Vorauflauf

Samen von mono- bzw. dikotylen Unkraut- bzw. Kulturpflanzen werden in Holzfasertöpfen in sandiger Lehmerde ausgelegt und mit Erde abgedeckt. Die in Form von benetzbaren Pulvern (WP) formulierten erfindungsgemäßen Verbindungen werden dann als wässrige Suspension bzw. Emulsion mit einer Wasseraufwandmenge von umgerechnet 600 I/ha unter Zusatz von 0,2% Netzmittel auf die Oberfläche der Abdeckerde appliziert.

Nach der Behandlung werden die Töpfe im Gewächshaus aufgestellt und unter guten Wachstumsbedingungen für die Testpflanzen gehalten. Nach ca. 3 Wochen wird die Wirkung der Präparate visuell im Vergleich zu unbehandelten Kontrollen bonitiert (herbizide Wirkung in Prozent (%): 100% Wirkung = Pflanzen sind abgestorben, 0 % Wirkung = wie Kontrollpflanzen).

Folgende Ergebnisse wurden mit den erfindungsgemäßen Verbindungen im Vorauflauf erreicht:

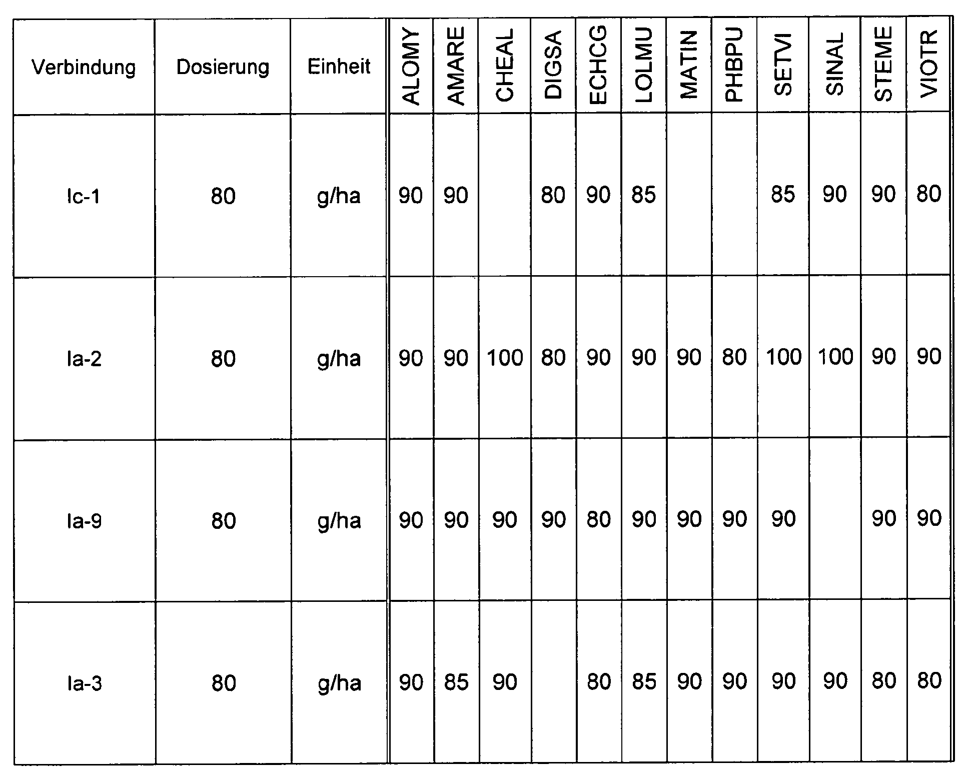

In der Tabelle weisen die einzelnen Kulturen die folgenden Abkürzungen auf:
- ALOMY:: Acker-Fuchsschwanz(gras) (*Alopecurus myosuroides*)
- AMARE:: Zurückgekrümmter Fuchsschwanz (*Amaranthus retroflexus*)
- CHEAL:: Weißer Gänsefuß (*Chenopodium album*)
- DIGSA:: (Blutrote) Fingerhirse (*Digitaria sanguinalis*)
- ECHCG:: Hühnerhirse (*Echinochloa crus-galli*)
- LOLMU:: Vielblütiger Lolch (*Lolium multiflorum*)
- MATIN:: Geruchlose Kamille (*Matricaria inodora*)
- PHBPU:: Purpur-Prachtwinde (*Pharbitis* / *Ipomoea purpurea*)
- SETVI:: Grüne Borstenhirse (*Setaria viridis*)
- SINAL:: Weißer Senf (*Sinapis alba*)

- STEME:: Vogelmiere (*Stellaria media*)
- VIOTR:: Wildes Stiefmütterchen (*Viola tricolor*)

### 2. Herbizide Wirkung im Nachauflauf

Samen von mono- bzw. dikotylen Unkraut- bzw. Kulturpflanzen werden in Holzfasertöpfen in sandigem Lehmboden ausgelegt, mit Erde abgedeckt und im Gewächshaus unter guten Wachstumsbedingungen angezogen. 2 bis 3 Wochen nach der Aussaat werden die Versuchspflanzen im Einblattstadium behandelt. Die in Form von benetzbaren Pulvern (WP) formulierten erfindungsgemäßen Verbindungen werden dann als wässrige Suspension bzw. Emulsion mit einer Wasseraufwandmenge von umgerechnet 600 I/ha unter Zusatz von 0,2% Netzmittel auf die grünen Pflanzenteile gesprüht. Nach ca. 3 Wochen Standzeit der Versuchspflanzen im Gewächshaus unter optimalen Wachstumsbedingungen wird die Wirkung der Präparate visuell im Vergleich zu unbehandelten Kontrollen bonitiert (herbizide Wirkung in Prozent (%): 100% Wirkung = Pflanzen sind abgestorben, 0 % Wirkung = wie Kontrollpflanzen).

Folgende Ergebnisse wurden mit den erfindungsgemäßen Verbindungen im Nachauflauf erreicht:

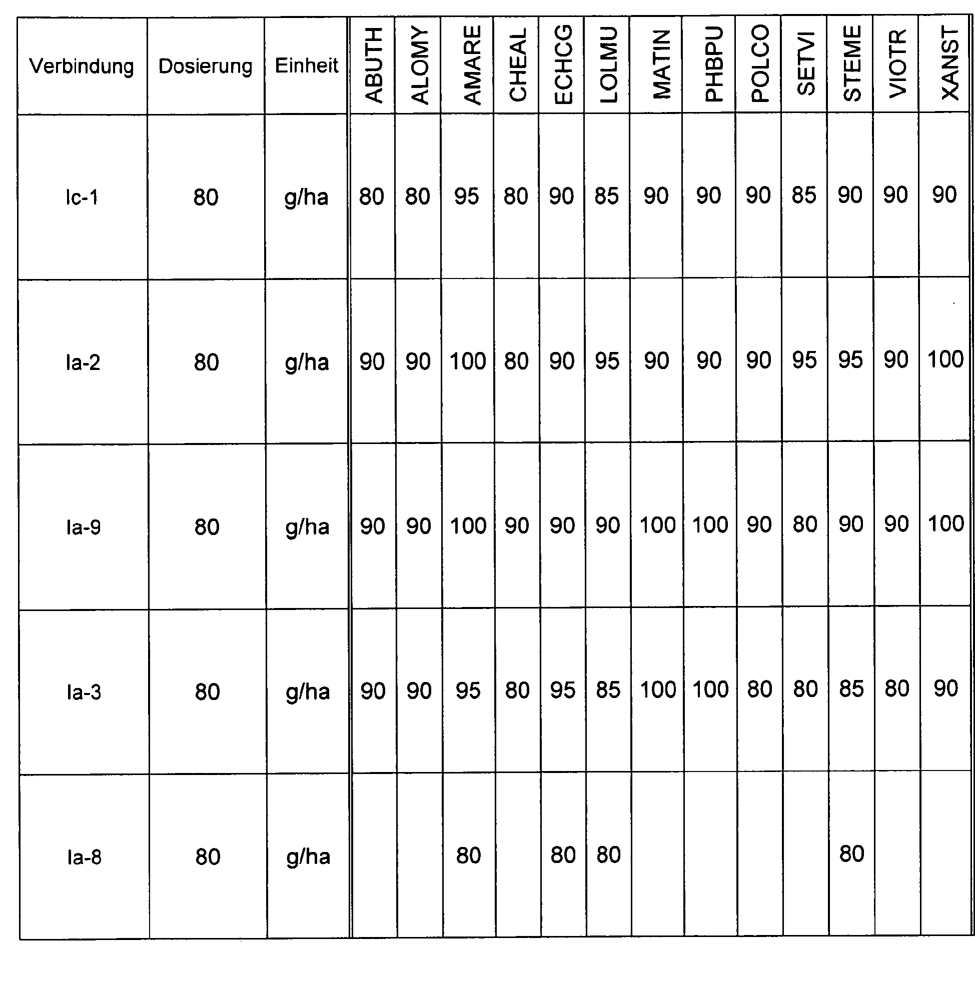

In der Tabelle weisen die einzelnen Kulturen die folgenden Abkürzungen auf:
- ABUTH:: Samtpappel (*Abutilon theophrasti*)
- ALOMY:: Acker-Fuchsschwanz(gras) (*Alopecurus myosuroides*)
- AMARE:: Zurückgekrümmter Fuchsschwanz (*Amaranthus retroflexus*)
- CHEAL:: Weißer Gänsefuß (*Chenopodium album*)
- ECHCG:: Hühnerhirse (*Echinochloa crus-galli*)
- LOLMU:: Vielblütiger Lolch (*Lolium multiflorum*)
- MATIN:: Geruchlose Kamille (*Matricaria inodora*)
- PHBPU:: Purpur-Prachtwinde (*Pharbitis* / *Ipomoea purpurea*)
- POLCO:: Gemeiner Windenknöterich (*Polygonum convolvulus*)
- SETVI:: Grüne Borstenhirse (*Setaria viridis*)
- STEME:: Vogelmiere (*Stellaria media*)
- VIOTR:: Wildes Stiefmütterchen (*Viola tricolor*)
- XANST:: Gewöhnliche Spitzklette (*Xanthium strumarium*)

## Patentansprüche

1. N-Azinyl-N'-pyridylsulfonyl-harnstoffe der allgemeinen Formel (I), 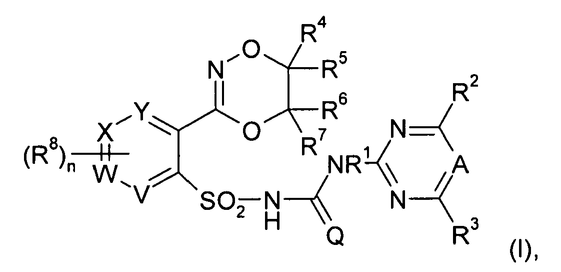 in welcher
W, X und Y so gewählt werden, dass ein Index dieser Indizes für ein Stickstoffatom steht und die übrigen Indizes für Kohlenstoffatome stehen, wobei die Kohlenstoffatome unsubstituiert oder unabhängig voneinander mit R⁸ substituiert sind;
V für ein Kohlenstoffatom steht, welches unsubstituiert oder mit R⁸ substituiert ist;
A ausgewählt ist aus der Gruppe, bestehend aus Stickstoff und CR⁹; wobei R⁹ ausgewählt ist aus der Gruppe, bestehend aus Wasserstoff, Alkyl, Halogen und Haloalkyl;
R¹ ausgewählt ist aus der Gruppe, bestehend aus Wasserstoff und einem gegebenenfalls substituierten Rest aus der Reihe Alkyl, Alkoxy, Alkoxyalkyl, Alkenyl, Alkinyl, Cycloalkyl, Cycloalkylalkyl, Aralkyl und Aryl;
R² ausgewählt ist aus der Gruppe, bestehend aus Wasserstoff, Halogen, gegebenenfalls durch Halogen substituiertes Alkyl, gegebenenfalls durch Halogen substituiertes Alkoxy, gegebenenfalls durch Halogen substituiertes Alkylthio, gegebenenfalls durch Halogen substituiertes Alkylamino oder gegebenenfalls durch Halogen substituiertes Dialkylamino;
R³ ausgewählt ist aus der Gruppe, bestehend aus Wasserstoff, Halogen, gegebenenfalls durch Halogen substituiertes Alkyl, gegebenenfalls durch Halogen substituiertes Alkoxy, gegebenenfalls durch Halogen substituiertes Alkylthio, gegebenenfalls durch Halogen substituiertes Alkylamino oder gegebenenfalls durch Halogen substituiertes Dialkylamino,
R⁴ bis R⁷, jeweils unabhängig voneinander, ausgewählt werden aus der Gruppe, bestehend aus Wasserstoff, Halogen, Cyano, Alkyl, Alkoxy, Alkylthio, Alkylsulfinyl, Alkylsulfonyl, Alkylamino, Dialkylamino, Alkylcarbonyl, Alkoxycarbonyl, Alkylaminocarbonyl oder Dialkylaminocarbonyl, wobei die Reste unsubstituiert sein können oder ein oder mehrere Reste, ausgewählt aus der Gruppe, bestehend aus Halogen, Cyano, Alkoxy und Alkylthio, tragen können, oder R⁴ und R⁶ bzw. R⁵ und R⁷ eine gegebenenfalls durch Sauerstoff oder Schwefel unterbrochene Alkyliden-Gruppe darstellen,
R⁸ ausgewählt ist aus der Gruppe, bestehend aus Halogen, Cyano, Thiocyanato, Nitro, Alkyl, Alkoxy, Alkylthio, Alkylsulfinyl, Alkylsulfonyl, Alkylamino, Dialkylamino, Alkylcarbonyl, Alkoxycarbonyl, Alkylaminocarbonyl, oder Dialkylaminocarbonyl, wobei die Reste unsubstituiert sein können oder ein oder mehrere Reste, ausgewählt aus der Gruppe, bestehend aus Halogen, Cyano, Alkoxy und Alkylthio, tragen können.
Q ausgewählt ist aus der Gruppe, bestehend aus Sauerstoff oder Schwefel, insbesondere Sauerstoff ist,
n gleich einer ganzzahligen Zahl von 0 bis 3 ist, und
sowie Salze von Verbindungen der Formel (I)
mit der Maßgabe, dass in den Fällen, in welchen n gleich 0, 1 oder 2 ist, die unsubstituierten Kohlenstoffatome V, W, X und/oder Y mit Wasserstoff abgesättigt sind.

2. N-Azinyl-N'-pyridylsulfonyl-harnstoffe nach Anspruch 1, **dadurch gekennzeichnet, dass** der Substituent A ausgewählt ist aus der Gruppe, bestehend aus Stickstoff und CH.

3. N-Azinyl-N'-pyridysulfonyl-harnstoffe nach Anspruch 1 oder 2, **dadurch gekennzeichnet, dass** der Substituent R¹ ausgewählt ist aus der Gruppe, bestehend aus Wasserstoff, gegebenenfalls durch Halogen substituiertes Alkyl, gegebenenfalls durch Halogen substituiertes Alkoxy, gegebenenfalls durch Halogen substituiertes Alkoxyalkyl, gegebenenfalls durch Halogen substituiertes Alkenyl und gegebenenfalls durch Halogen substituiertes Alkinyl.

4. N-Azinyl-N'-pyridylsulfonyl-harnstoffe nach einem der Ansprüche 1 bis 3, **dadurch gekennzeichnet, dass** der Substituent R² ausgewählt ist aus der Gruppe, bestehend aus Wasserstoff, Halogen, gegebenenfalls durch Halogen substituiertes Alkyl, gegebenenfalls durch Halogen substituiertes Alkoxy, gegebenenfalls durch Halogen substituiertes Alkylthio, gegebenenfalls durch Halogen substituiertes Alkylamino und gegebenenfalls durch Halogen substituiertes Dialkylamino.

5. N-Azinyl-N'-pyridysulfonyl-harnstoffe nach einem der Ansprüche 1 bis 4, **dadurch gekennzeichnet, dass** der Substituent R³ ausgewählt ist aus der Gruppe, bestehend aus Wasserstoff, Halogen, gegebenenfalls durch Halogen substituiertes Alkyl, gegebenenfalls durch Halogen substituiertes Alkoxy, gegebenenfalls durch Halogen substituiertes Alkylthio, gegebenenfalls durch Halogen substituiertes Alkylamino und gegebenenfalls durch Halogen substituiertes Dialkylamino.

6. N-Azinyl-N'-pyridysulfonyl-harnstoffe nach einem der Ansprüche 1 bis 5, **dadurch gekennzeichnet, dass** die Substituenten R⁴ bis R⁷, jeweils unabhängig voneinander, ausgewählt sind aus der Gruppe, bestehend aus Wasserstoff, Halogen, Cyano, Thiocyanato, gegebenenfalls durch Halogen substituiertes Alkyl, gegebenenfalls durch Halogen substituiertes Alkoxy, gegebenenfalls durch Halogen substituiertes Alkylthio, gegebenenfalls durch Halogen substituiertes Alkylsulfinyl, gegebenenfalls durch Halogen substituiertes Alkylsulfonyl, gegebenenfalls durch Halogen substituiertes Alkylamino, gegebenenfalls durch Halogen substituiertes Alkylcarbonyl, gegebenenfalls durch Halogen substituiertes Alkoxycarbonyl oder gegebenenfalls durch Halogen substituiertes Alkylaminocarbonyl.

7. N-Azinyl-N'-pyridysulfonyl-harnstoffe nach einem der Ansprüche 1 bis 6, **dadurch gekennzeichnet, dass** der Substituent R⁸ ausgewählt ist aus der Gruppe, bestehend aus Halogen, Cyano, Thiocyanato, gegebenenfalls durch Halogen substituiertes Alkyl, gegebenenfalls durch Halogen substituiertes Alkoxy, gegebenenfalls durch Halogen substituiertes Alkylthio, gegebenenfalls durch Halogen substituiertes Alkylsulfinyl, gegebenenfalls durch Halogen substituiertes Alkylsulfonyl, gegebenenfalls durch Halogen substituiertes Alkylamino, gegebenenfalls durch Halogen substituiertes Alkylcarbonyl, gegebenenfalls durch Halogen substituiertes Alkoxycarbonyl und gegebenenfalls durch Halogen substituiertes Alkylaminocarbonyl.

8. N-Azinyl-N'-pyridysulfonyl-harnstoffe nach einem der Ansprüche 1 bis 7, **dadurch gekennzeichnet, dass** n gleich 0 ist.

9. Verfahren zur Herstellung von N-Azinyl-N'-pyridyisulfonyl-harnstoffen gemäß einem der Ansprüche 1 bis 8, **gekennzeichnet durch** einen der folgenden Verfahrensschritte:
(a)
Umsetzung von (5,6-Dihydro-1,4,2-dioxazin-3-yl)pyridyl-sulfonamiden der allgemeinen Formel (II) 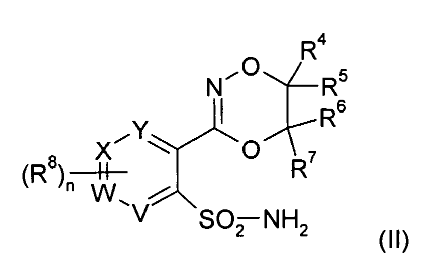 mit einem heterocyclischen (Thio)-Carbamat der allgemeinen Formel (III) 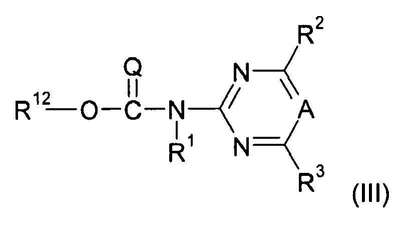 worin R¹² ein substituierter oder unsubstituierter (C₁-C₂₀)-Kohlenwasserstoffrest wie Aryl oder Alkyl, vorzugsweise gegebenenfalls substituiertes Phenyl oder gegebenenfalls substituiertes (C₁-C₄)-Alkyl bedeutet und worin V, W, X, Y, R¹ bis R⁸, Q, A und n die Bedeutungen gemäß einem der Ansprüche 1 bis 8 aufweisen; oder
(b)
Umsetzung von 5,6-Dihydro-1,4,2-dioxazin-3-yl-pyridin-sulfonsäureiso(thio)-cyanaten der allgemeinen Formel (IV) 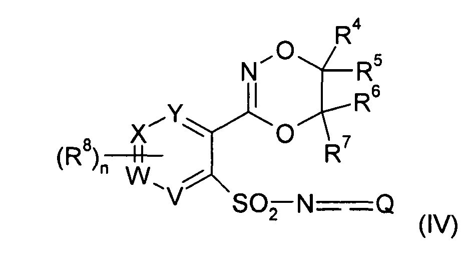 mit einem Aminoheterocyclus der allgemeinen Formel (V) 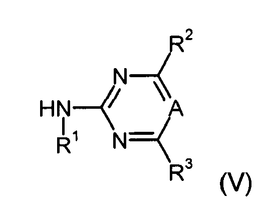 worin V, W, X, Y, R¹ bis R⁸, Q, A und n die Bedeutungen gemäß einem der Ansprüche 1 bis 8 aufweisen; oder
(c)
Umsetzung von Sulfonyl(thio)carbamaten der allgemeinen Formel (VI) 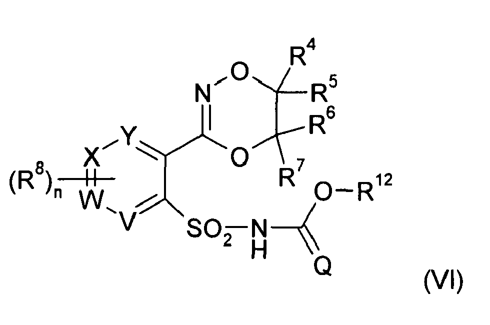 worin R¹² ein substituierter oder unsubstituierter (C₁-C₂₀)-Kohlenwasserstoffrest wie Aryl oder Alkyl, vorzugsweise gebenenfalls substituiertes Phenyl oder gegebenenfalls substituiertes (C₁-C₄)-Alkyl bedeutet, mit einem Aminoheterocyclus der allgemeinen Formel (V) 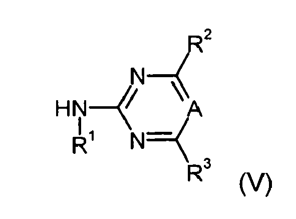 worin V, W, X, Y, R¹ bis R⁸, Q, A und n die Bedeutungen gemäß einem der Ansprüche 1 bis 8 aufweisen; oder
(d)
Umsetzung von (5,6-Dihydro-1,4,2-dioxazin-3-yl)pyridyl-sulfonamiden der allgemeinen Formel (II) 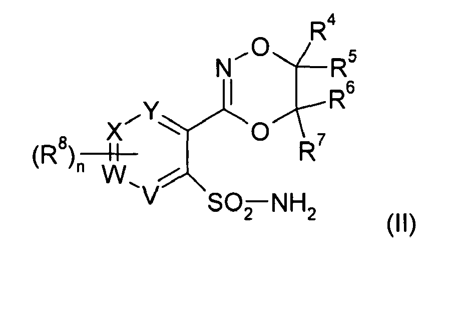 mit einem Iso(thio)cyanat der allgemeinen Formel (VII) 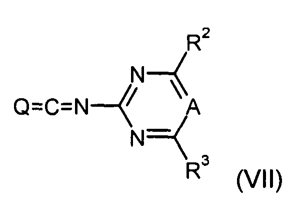 gegebenenfalls in Gegenwart eines Reaktionshilfsmittels umsetzt, worin R¹ Wasserstoff bedeutet und R² bis R⁸, V, W, X, Y, Q, A und n die Bedeutungen gemäß einem der Ansprüche 1 bis 8 aufweisen; oder
(e)
Umsetzung von einem Aminoheterocyclus der allgemeinen Formel (V) 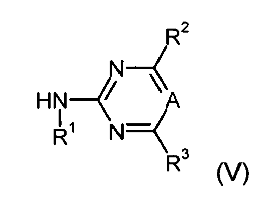 zunächst basenkatalysiert mit einem Kohlensäureester, insbesondere Diphenylcarbonat, und Umsetzung des gebildeten Intermediats der allgemeinen Formel (III) 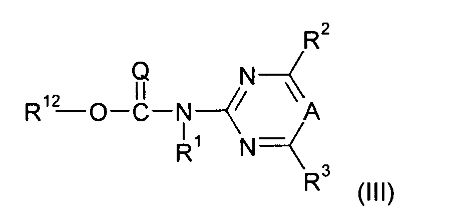 in einer Eintopfreaktion mit einem (5,6-Dihydro-1,4,2-dioxazin-3-yl)pyridylsulfonamid der allgemeinen Formel (II) 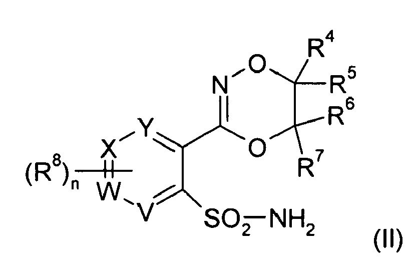 umsetzt, worin R¹ Wasserstoff bedeutet und R² bis R⁸, V, W, X, Y, Q, A und n die Bedeutungen gemäß einem der Ansprüche 1 bis 8 aufweisen, oder
(f)
Umsetzung von einem (5,6-Dihydro-1,4,2-dioxazin-3-yl)pyridyl-sulfonsäurehalogenid der allgemeinen Formel (VIII) 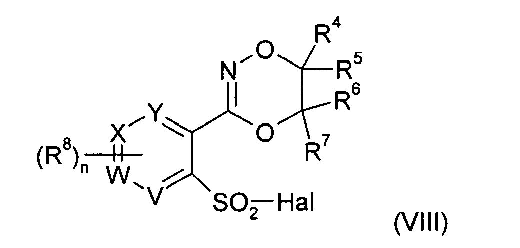 wobei Hal ein Halogenatom, vorzugsweise Chlor, ist, mit einem (Thio)-Cyanat, insbesondere einem Metall(thio)-cyanat, insbesondere einem Alkalimetall-(thio)cyanat, wie Natrium(thio)cyanat, zu einem Iso(thio)cyanat der Formel (IV) 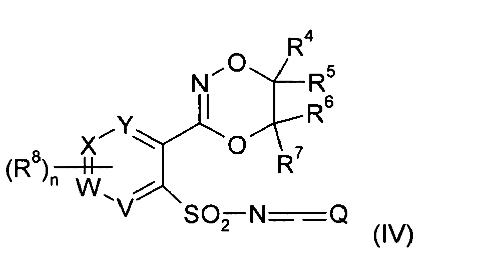 oder einem solvatisierten (stabilisierten) Derivat davon und anschließend mit einem Aminoheterocyclus der allgemeinen Formel (V) 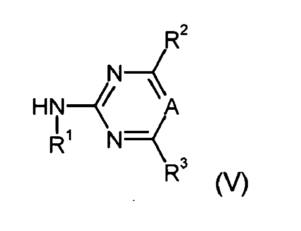 umsetzt, worin V, W, X, Y, R¹ bis R⁸, Q, A und n die Bedeutungen gemäß einem der Ansprüche 1 bis 8 aufweisen; oder
(g)
für Q = Sauerstoff **durch** Umsetzung von (5,6-Dihydro-1,4,2-dioxazin-3-yl)pyridyl-sulfonamiden der allgemeinen Formel (II) 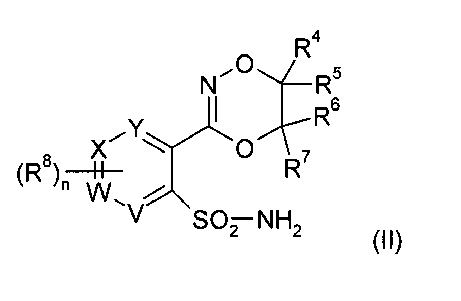 mit einem heterocyclischen Bis-carbamat der allgemeinen Formel (IX), 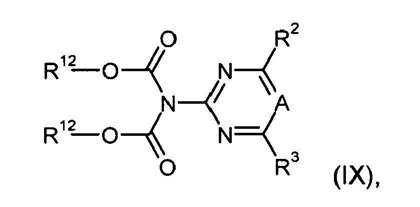 worin R¹² ein substituierter oder unsubstituierter (C₁-C₂₀)-Kohlenwasserstoffrest, wie Aryl oder Alkyl, vorzugsweise gegebenenfalls substituiertes Phenyl oder gegebenenfalls substituiertes (C₁-C₄)-Alkyl, bedeutet, in Gegenwart eines basischen Reaktionshilfsmittels, worin V, W, X, Y, R¹ bis R⁸, A und n die Bedeutungen gemäß einem der Ansprüche 1 bis 8 aufweisen; oder
(h)
Umsetzung von (5,6-Dihydro-1,4,2-dioxazin-3-yl)pyridyl-sulfonamiden der allgemeinen Formel (II) 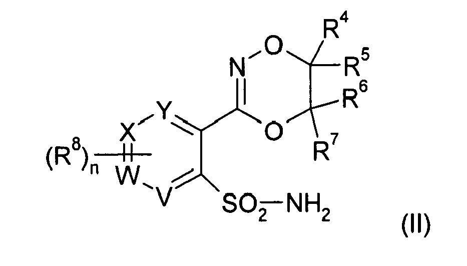 zunächst basenkatalysiert mit einem Kohlensäureester, insbesondere Diphenylcarbonat, und Umsetzung des gebildeten Intermediats der allgemeinen Formel (VI) 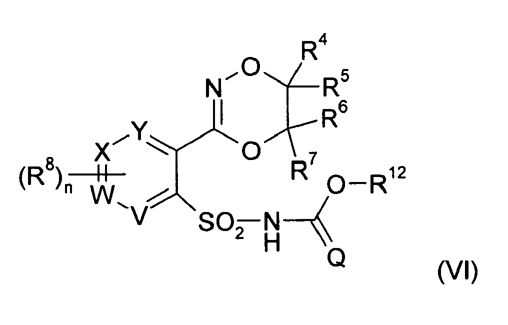 in einer Eintopfreaktion mit einem Aminoheterocyclus der allgemeinen Formel (V) 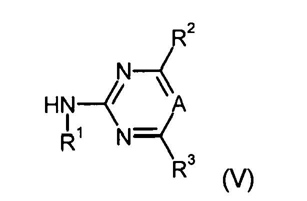 worin V, W, X, Y, R¹ bis R⁸, Q, A und n die Bedeutungen gemäß einem der Ansprüche 1 bis 8 aufweisen.

10. Verbindungen der allgemeinen Formel (II) 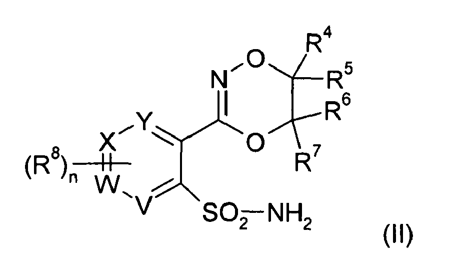 in welchen die Reste V, W, X, Y, R⁴, R⁵, R⁶, R⁷, R⁸ und n die Bedeutungen gemäß einem der Ansprüche 1 bis 8 aufweisen.

11. Verbindungen der allgemeinen Formel (IV) 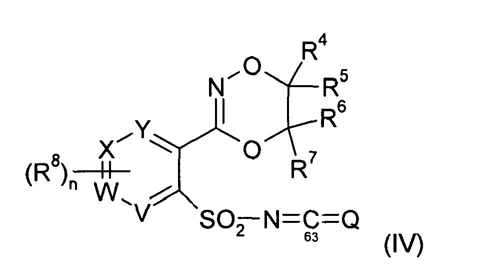 in welchen die Reste V, W, X, Y, R⁴, R⁵, R⁶, R⁷, R⁸, Hal und n die Bedeutungen gemäß einem der Ansprüche 1 bis 8 aufweisen.

12. Verbindungen der allgemeinen Formel (X) 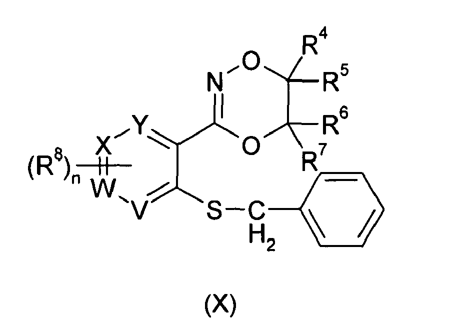 in welchen die Reste V, W, X, Y, R⁴, R⁵, R⁶, R⁷, R⁸ und n die Bedeutungen gemäß einem der Ansprüche 1 bis 8 aufweisen.

13. Verbindungen der allgemeinen Formel (VIII) 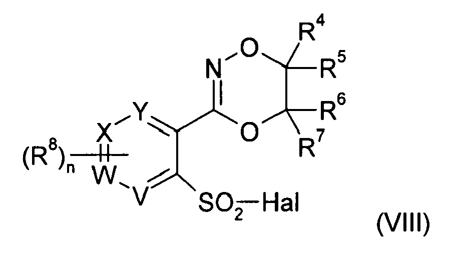 in welchen die Reste V, W, X, Y, R⁴, R⁵, R⁶, R⁷, R⁸ und n die Bedeutungen gemäß einem der Ansprüche 1 bis 8 aufweisen.

14. Verbindungen der allgemeinen Formel (VI) 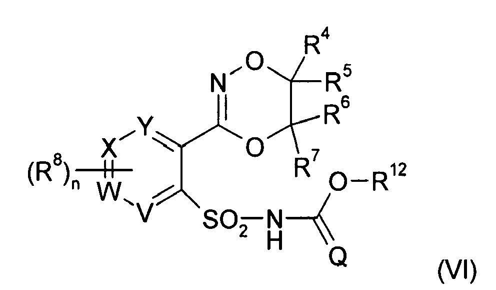 in welchen die Reste V, W, X, Y, R⁴, R⁵, R⁶, R⁷, R⁸ und n die Bedeutungen gemäß einem der Ansprüche 1 bis 8 aufweisen und worin R¹² ein substituierter oder unsubstituierter (C₁-C₂₀)-Kohlenwasserstoffrest wie Aryl oder Alkyl, vorzugsweise gebenenfalls substituiertes Phenyl oder gegebenenfalls substituiertes (C₁-C₄)-Alkyl bedeutet.

15. Zusammensetzungen, enthaltend mindestens eine Verbindung der allgemeinen Formel (I) gemäß einem der Ansprüche 1 bis 8.

16. Zusammensetzung nach Anspruch 15, **dadurch gekennzeichnet, dass** die Zusammensetzung mindestens einen weiteren Wirkstoff umfasst, welcher ausgewählt ist aus der Gruppe, bestehend aus mindestens einem weiteren Herbizid und mindestens einem Safener.

17. Verwendung der Verbindungen der allgemeinen Formel (I) gemäß einem der Ansprüche 1 bis 8 als Herbizide und Pflanzenwachstumsregulatoren.

18. Verwendung der Zusammensetzungen gemäß einem Anspruch 15 oder 16 als Herbizide und Pflanzenwachstumsregulatoren.

19. Verwendung nach Anspruch 16 oder 17 zur Pflanzenbekämpfung in speziellen Pflanzenkulturen oder als Pflanzenschutzregulator.
